# EUROPEAN PATENT SPECIFICATION

(11) **EP 0 719 267 B1**
(45) Date of publication and mention of the grant of the patent: **31.07.2002**
(21) Application number: 94913336.7
(22) Date of filing: 31.03.1994
(51) Int. Cl.: C07D 473/10, A61K 31/52, C07D 239/54, C07C 275/54

(54) **SECOND MESSENGER CELL SIGNALING INHIBITORS**
INHIBITOREN DES DURCH SEKUNDÄRE MESSENGER ÜBERTRAGENEN ZELLSIGNALE
INHIBITEURS DE LA SIGNALISATION CELLULAIRE VEHICULEE PAR DES MESSAGERS SECONDAIRES

(30) Priority: 31.03.1993 US 40820; 12.11.1993 US 152650; 08.12.1993 US 164081
(43) Date of publication of application: 03.07.1996
(73) Proprietor: CELL THERAPEUTICS, INC., Seattle, WA 98119 (US)
(72) Inventor: MICHNICK, John, Seattle, WA 98117 (US); UNDERINER, Gail, E., Brier, WA 98036 (US); KLEIN, J., Peter, Vashon Island, WA 98070 (US); RICE, Glenn, C., Seattle, WA 98177 (US); SUNKARA, Prasad, S., Mercer Island, WA 98040 (US); KUMAR, Dr Anil M., Seattle, Washington 98133 (US)
(74) Representative: Grünecker, August, Dipl.-Ing.
(86) International application number: US9403548
(87) International publication number: WO9422863

(56) References cited:
- EP-A- 0 113 102
- GB-A- 2 096 606
- JP-A- 58 096 087
- US-A- 4 668 786

## Description

### Field of the Invention

The invention provides a group of compounds that are effective agents to inhibit specific cellular signaling events often induced by inflammatory stimuli, to act as anti-inflammatory or immunosuppressive agents, to act as cytotoxic agents for treatment of cancers, or to be directly or indirectly antimicrobial to yeast or fungal infections. More specifically, the inventive compounds have at least one amino alcohol (or derivative thereof) functional group on a side chain attached to a core moiety. The inventive compounds are useful antagonists to control intracellular levels of specific sn-2 unsaturated phosphatidic acids and corresponding phosphatidic acid-derived diacylglycerols, intracellular cell signaling messengers which occur in response to pro-inflammatory and selectively proliferative stimuli.

### Background of the Invention

Pentoxifylline [1-(5-oxohexyl)-3,7-dimethylxanthine], abbreviated PTX, is a xanthine derivative widely used medically for increasing blood flow. U.S. Patents Nos. 3,422,107 and 3,737,433, both to Mohler *et al.* disclose PTX. Metabolites of PTX were summarized in Davis *et al.,* "Microbial Models of Mammalian Metabolism: Microbial Reduction and oxidation of Pentoxifylline," *Applied and Environmental Microbiology,* Vol. 48, No. 2, pages 327-381, August, 1984, and Bryce *et al.,* "Metabolism and Pharmacokinetics of ¹⁴C-Pentoxifylline in Healthy Voluteers," *Arzneim.-Forsch.*/*Drug Res.* Vol. 39, No. 4, pages 512-517, 1989. A metabolite of PTX is 1-(5-hydroxyhexyl)-3,7-dimethylxanthine, designated M1. M1 was also disclosed as increasing cerebral blood flow in U.S. Patents Nos. 4,515,795 and 4,576,947. Other metabolites include 1-(5-pentoyl)-3,7-dimethylxanthine carboxylic acid, designated M4, and 1-(4-butyl)-3,7-dimethylxanthine carboxylic acid, designated M5. In addition, U.S. Patents Nos. 4,833,146 and 5,039,666 disclose use of tertiary alcohol analogs of xanthine for enhancing cerebral blood flow.

PTX and its known metabolites thereof have been shown to have *in vivo* activity in specific biologic systems. U.S. Patent No. 4,636,507 describes an ability of PTX to enhance chemotaxis in polymorphonuclear leukocytes responding to chemotaxis stimulation. In addtion, PTX and related tertiary alcohol substituted xanthines inhibit activity of certain cytokines to affect chemotaxis as described in U.S. Patents Nos. 4,965,271 and 5,096,906. Furthermore, by co-administrating PTX and GM-CSF, patients undergoing allogeneic bone marrow transplant exhibited decreased levels of tumor necrosis factor (TNF). Bianco *et al*., "Pentoxifylline (PTX) and GM-CSF Decrease Tumor Necrosis Factor (TNF-α) Levels in patients undergoing allogeneic Bone Marrow Transplantation (BMT)," *Blood,* Vol. 76, No. 1, Suppl. 1 (522), page 133a, November 15, 1990. Reduction in assayable levels of TNF was accompanied by reduced BMT-related complications. However, in normal volunteers, TNF levels were higher among PTX recipients. Therefore, elevated levels of TNF are not the primary cause of such complications.

Therefore, effective therapeutic compounds that are safe and effective for human or animal administration and that can maintain cellular homeostasis in the face of a variety of inflammatory stimuli are needed. The invention is a result of research conducted in looking for such compounds.

GB-A-2 096 606 and US-A-4 668 786 disclose biologically active compounds which are structurally similar to the present compounds, differing because of the value of m. No mention is made in these documents of second messenger cell signalling inhibitory activity.

### Summary of the Invention

The invention provides compounds useful in a large variety of therapeutic indications for modulating disease by intracellular signaling through specific intracellular signaling pathways. In addition, the inventive compounds and pharmaceutical compositions are suitable for normal routes of therapeutic administration (*e.g.*, parenteral, oral, topical, etc.) for providing effective dosages of a therapeutic compound.

The invention provides for a class of compounds that are effective therapeutic agents to inhibit specific inflammatory and proliferative cellular signaling events. The compounds and pharmaceutical compositions thereof have the formula:

(X)j - (core moiety),

wherein j is an integer from one to three, the core moiety is a cyclic or heterocyclic core moiety having from one to three, five- to six-membered ring structures and X is a racemic mixture, R or S enantiomer, solvate, hydrate, or salt of: 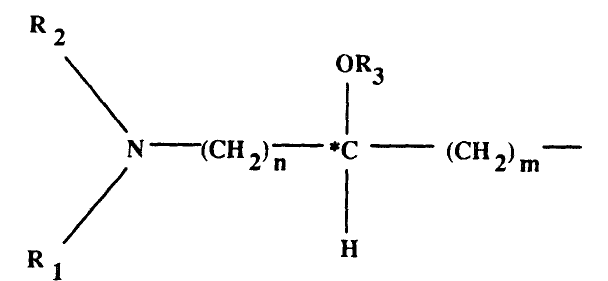 wherein *C is a chiral carbon atom; n is one; the carbon atom of (CH₂)ₙ may be substituted by a keto or hydroxy group; m is an integer from eight to fourteen; independently, R₁ and R₂ are hydrogen, a straight or branched chain alkane or alkene of up to twelve carbon atoms in length, or -(CH₂)_{w}R₅, w being an integer from two to fourteen and R₅ being a mono-, di- or tri-substituted or unsubstituted aryl group, substituents on R₅ being hydroxy, chloro, fluoro, bromo, or C₁₋₆ alkoxy; or jointly, R₁ and R₂ form a saturated or unsaturated heterocyclic group having from four to eight carbon atoms, N being a hetero atom; and R₃ is hydrogen or C₁₋₃

A core moiety is at least one five- to six-membered ring, preferably having from one to three, five- to six-membered ring structures in a predominantly planar configuration. Preferably, the substituent (X) is bonded to a ring nitrogen if one exists. For example, the core moiety may be selected from the group consisting of substituted or unsubstituted barbituric acid; benzamide; benzene; biphenyl; cyclohexane; cyclohexanedione; cyclopentanedione; lactam; glutarimide; homophthalimide; hydrophthalimide; imidazole; imidazole amide; indomethacin; isocarbostyril; lumazine; napthlalene; phenol; pteridine; pthalimide; piperidine; pyridine; pyrimidine; pyrrole amide; quinolizinedione; quinazolinone; quinolone; resorcinol; salicylic acid and derivatives thereof; stilbene; succinimide; theobromine; thymine; triazine; tricyclododecane; uric acid; uracil; vitamins A, E or K; or xanthine.

Preferred cyclic cores include substituted or unsubstituted glutarimide, methylthymine, methyluracil, thymine, theobromine, uracil and xanthine, most preferably halogen-substituted xanthine. Exemplary preferred cores include: 1,3-cyclohexanedione, 1,3-cyclopentanedione; 1,3-dihydroxynaphthalene; 1-methyllumazine; methylbarbituric acid; 3,3-dimethylglutarimide; orotic acid; tetra or hexahydrophthalimide; orthophenol; prostacyclin; 2-hydroxypyridine; methyldihydroxypyrazolopyrimidine, specifically, 1,3-dimethyldihydroxypyrazolo[4,3-d]pyrimidine; methylpyrrolopyrimidine; 1-methylpyrrolo [2,3-d] pyrimidine; 1,3-dihydroxynapthalene; 1-pyrrole amides; 2-pyrrole amides; 3-pyrrole amides; 1,2,3,4-tetrahydroisoquinolone; 1-methyl-2,4(1H,3H)-quinolizinedione (1-methylbenzoyleneurea); quinazolin-4(3H)-one; sulindac; dihydrothymine; alkyl-substituted (C1-6) thymine; 2,4-dioxohexahydro-1,3,5-tetrazine; methylthymine; alkyl-substituted (C1-6) uracil; uracil fused to naphthalene; 6-aminouracil; 1-methyl-5,6-dihydrouracil; 1-methyluracil; 5- and/or 6-position substituted uracils (such as, for example, 5-bromouracil); B-ionone as vitamin A; 2,6,6-methyl-1-cyclohexene-1-acetaldehyde as vitamin A; tetralone to vitamin K; 1,7-dimethylxanthine, 3,7-dimethylxanthine; 3-methylxanthine; 3-methyl-7-methylpivaloylxanthine; 8-substituted xanthines (having substituents such as N or S); and 7-methylhypoxanthine.

Preferably, X is bonded to a nitrogen of the core moiety, most preferably the core moiety is xanthine and X is bonded to an N₁ xanthine nitrogen and N₃ and N₇ xanthine nitrogens are independently substituted by a member selected from the group consisting of hydrogen, methyl, fluoro, chloro and amino.

The invention provides a pharmaceutical composition comprising an inventive compound and a pharmaceutically acceptable excipient. The pharmaceutical composition may be formulated for oral, parenteral or topical administration to a patient.

The invention includes a method for preparing a medicament for treating an individual having a variety of diseases. The disease is characterized by or can be treated by inhibiting an immune response or a cellular response to external or *in situ* primary stimuli, the cellular response being mediated through a specific phospholipid-based second messenger acting adjacent to a cell membrane inner leaflet. The second messenger pathway is activated in response to various noxious or proliferative stimuli characteristic of a variety of disease states. Biochemistry of this second messenger pathway is described herein. More specifically, the invention includes methods for preparing a medicament for treating or preventing clinical symptoms of various disease states or reducing toxicity of other treatments by inhibiting cellular signaling through a second messenger pathway involving signaling through phosphatidic acid and through glycan phosphatidylinostinol (Gly PI).

The inventive compounds are of particular significance for inhibiting IL-2-induced proliferative response. IL-2 signaling inhibition is potentially useful in the treatment of numerous disease states involving T-cell activation and hyperproliferation. Exemplary autoimmune diseases treated by inhibiting IL-2 signaling are lupus, scleroderma, rheumatoid arthritis, multiple sclerosis, glomerula nephritis as well as potential malignancies, including but not limited to, chronic myelogenous leukemia as well as others.

### Brief Description of the Drawings

Figures 1A and 1B are dose response curves for both cyclosporin A (CsA, Fig. 1A) and various inventive compounds (Fig. 1B) for murine thymocyte proliferation co-stimulated by Concanavalin A (ConA) and interleukin-2 alpha (IL-2).

Figures 2A and 2B illustrate immune modulating activity of compounds nos. 5 and 26 (see corresponding names and structures below) in an assay determining proliferative PMBC response to allogeneic stimulation using a two-way, mixed lymphocyte reaction.

Figure 2C and 2D report activity data for compounds nos. 27 and 13 and IC₅₀ data, respectively in a mixed lymphocyte reaction.

Figure 2E shows percent viability for five inventive compounds (corresponding chemical names below) in a mixed lymphocyte assay measuring immune suppression activity of the compounds tested.

Figure 2F shows percent viability results from a mixed lymphocyte reaction assay

Figure 3A reports results obtained for several inventive compounds exhibiting inhibitive effects on murine thymocyte proliferation.

Figure 3B shows comparative results from inhition assays for co-stimulated thymocyte proliferation.

Figure 4 reports IC₅₀ values for compounds nos. 27 and 28 in a murine splenocyte proliferation (anti-mu stimulated assay).

Figures 5A and 5B are frequency histograms of measurements for 20,000 cells in an assay illustrating IL-2 (alpha chain CD25) receptor expression.

Figure 6 shows results for compounds nos. 49 and 50 as compared with CsA in an inhibitive assay for production of IL-2 in murine thymocytes.

Figures 7 and 8 report dose response results from an *in vitro* assay emphasizing cellular or T cell immune response.

Figures 9A and 9B report inhibitive activity for the inventive compounds, as compared with CsA on direct IL-2-induced proliferation in a murine cytotoxic T-cell line, CT6.

Figure 10 illustrates activity of the inventive compounds in anti-yeast and anti-fungal assays.

Figure 11 reports activity data for inventive compounds in an assay designed to detect potential anitgen specific anergy-induction.

Figure 12 shows activity results for inventive compounds nos. 28, 30, 31, 33 and 29 in an assay useful in predicting therapeutic activity for preventing or treating restenosis, atherosclerosis and coronary artery disease.

Figure 13 shows cytotoxic results for inventive compounds in a human stromal cell/PDGF stimulation assay.

Figure 14 reports inhibitive activity results for inventive compounds nos. 27, 28, 32, 30, 31, 32 and 34 in an assay measuring inhibitive effects in a PDGF/IL-1 co-stimulation.

Figure 15 illustrates a dose response curve for selected inventive compounds in a PDGF-induced proliferation of Balb/3T3 cells.

Figure 16 reports suppressive results for selected inventive compounds in a murine thymocyte ConA/IL-2 co-stimulation assay.

Figure 17 compares IC₅₀ and ID₅₀ data for several inventive compounds.

Figure 18 reports cytotoxicity results for inventive compound no. 27 for transformed (Ras 3T3) cells and non-transformed (normal) cells.

Figure 19 shows data demonstrating inhibitory effects of inventive compound no. 58 on PDGF-induced proliferation of human aortic smooth muscle cells (aortic SMC).

Figures 20A and 20B show the effects of compound no. 58 on aFGF and bFGF- induced proliferation in human aortic smooth muscle cells (aortic SMC).

Figures 21A and 21B show inhibitory activity of compound no. 35 and CsA, respectively, on murine thymocytes, co-stimulated with ConA and IL-2.

Figures 21C and 21D illustrate inhibitory effects of compound no. 35 and CsA, respectively, on IL-2-induced proliferation of cytotoxic CT-6 cells.

Figure 22 reports activity results for compound no. 58 on vascular endothelial growth factor (VEGF)-induced proliferation in a human umbilical vein endothelial cell line (HUVEC).

Figure 23 is a series of frequency histograms obtained from flow cytometric analysis of HUVEC cells.

Figure 24 illustrates inhibitive results obtained for compounds nos. 50 and 57 on THP-1 cell adhesion to IL-1-activated HUVEC.

Figures 25A and 25B show the effects of compound no. 58 on aFGF and bFGF- induced proliferation in pulmonary smooth muscle cell.

Figures 26A, 26B and 26C are dose response curves for inventive compounds nos. 77, 7 and 78 in PDGF-induced proliferation of Balb/3T3 cells.

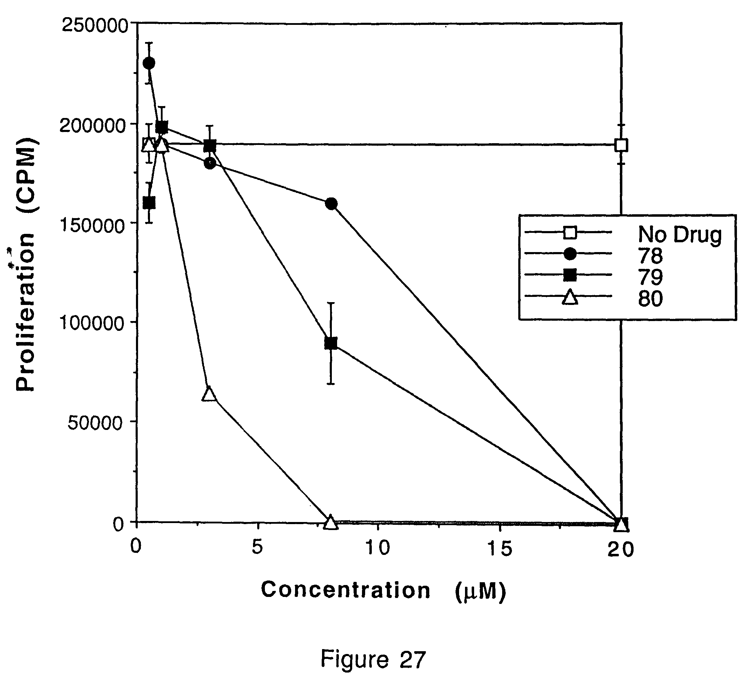

Figure 27 illustrates dose response curves for inventive compounds nos. 78, 79 and 80 for murine thymocyte proliferation co-stimulated by ConA and IL-2.

Figure 28 reports results for inventive compound no. 50 in the PDGF-induced proliferation of Balb/3T3 cells.

Figure 29 shows that TNF-α substantially increased expression of the 92 kD matrix metalloprotease (MMP) and moderately increased production of the 72 kD MMP and that the presence of compound no. 50 blocked the TNF-α-stimulated expression of each MMP. Figures 30 shows anti-proliferative activity and Figure 31 reports anti-clonogenicity activity of inventive compound no. 50 with HT-29 cells.

Figures 32 and 33 illustrate cytotoxicity and concentration dependence of inventive compound no. 50 against 3LL cells (Lewis lung carcinoma).

Figure 34 shows that inventive compound no. 50 lacks cytotoxic activity in normal human bone marrow stromal cells.

Figures 35 and 36 show the effects of inventive compound no. 50 on matrigel invasion and viability in 3LL cells.

Figure 37 illustrates VEGF induced proliferation of HUVEC as a predictive adhesion assay.

Figure 38 shows the effect of inventive compound no. 50 on THP-1 adherence to IL-1β-stimulated HUVEC.

Figure 39 illustrates the effect of inventive compound no. 50 on VCAM-1 surface expression of TNFα-stimulated HUVEC.

Figure 40 illustrates the effect of inventive compound no. 50 on ICAM-1 surface expression of TNFα-stimulated HUVEC.

Figures 41A, 41B and 41C illustrates the results from an *in vivo* study in mice with B16 melanoma cells

Figures 42A and 42B illustrate T and B cell response assays of mice treated with inventive compound no. 50 in a B16 melanoma cell assay.

Figure 43 reports results in an *in vivo* experiment showing that inventive compound no. 50 can arrest growth of Lewis Lung Carcinoma in mice.

Figure 44A and 44B illustrate platelet and neutrophil counts, respectively, of the sacrificed mice in a Lewis Lung Carcinoma assay.

Figures 45A, 45B and 45C illustrate a photographic comparison of lungs from the 3LL exposed mice with or without treatment using inventive compound no. 50.

Figure 46 reports results obtained in an assay investigating inventive compound no.'s 45 effect on IL-2-mediated proliferation

Figure 47 illustrates that inventive compound no. 45 inhibits CT-6.1 proliferation as measured by cell number.

Figure 48 illustrates that inventive compound no. 45 also inhibits mitogenic responses to IL-2, IL-4 and IL-7.

Figure 49 shows that inventive compound no. 45 also inhibited murine thymocyte proliferation in response to conconavalin A (conA) and IL-2.

Figures 50A and 50B illustrate that compound no. 45 inhibits a murine mixed tumor lymophocyte culture (MTLC) and a human mixed leukocyte reaction (MLR), respectively.

Figure 51 illustrates an effect of the delayed addition of inventive compound no. 45 on co-stimulated thymocyte proliferation.

Figure 52 illustrates that inventive compound no. 45 inhibits anti-CD3 stimulated splenocyte proliferation.

Figures 53A and 53B illustrate that compound no. 45 does not inhibit T cell receptor (CD3) mediated signaling.

Figures 54A and 54B illustrate that inventive compound no. 45 does not inhibit anti-CD3 mediated upregulation of the IL-2 receptor alpha subunit.

Figure 55 reports that inventive compound no. 45 does not inhibit IL-2 receptor beta (p70) subunit intemalization.

Figures 56A and 56B illustrate that the inventive compound no. 45 also induces antigen specific T-cell anergy.

Figure 57 illustrates that inventive compound no. 45 inhibits B cell proliferation. Figures 58A and 58B illustrate that inventive compound no. 45 does not inhibit CD28-mediated IL-2 release.

Figures 58C and 58D illustrate that compound no. 45 inhibits IFN-γ release by blocking IL-2 signaling.

Figures 59A, 59B and 59C report assay results investigating the effect of inventive compound no. 45 on cytokine release from anti-CD3-stimulated mouse splenocytes.

Figure 60A shows inhibition of proliferation in an MTLC assay with compound no. 45.

Figure 60B and 60C illustrate that compound no. 45 does not inhibit either IL-2 or TNFa release from the MTLC.

Figure 60D shows that inventive compound no. 45 inhibits IFN-γ release.

Figure 61 illustrates that inventive compound no. 45 does not have an effect on prostaglandin E2 release from IL-1α-stimulated human foreskin fibroblasts, HS68.

Figures 62A and 62B (TNFα or IL-1β, respectively) report inhibition of THP-1 adhesion to HUVEC.

Figures 63A and 63B illustrate that inventive compound no. 45 inhibits adhesion receptor expression on HUVEC.

Figure 64 reports that inventive compound no. 45 inhibits PDGF-BB-induced murine BALB/3T3 proliferation.

Figures 65A-65F illustrate inhibition of proliferation in either human aortic or pulmonary smooth muscle cells (SMC) by inventive compound no. 58.

Figures 66A and 66B are a dose response and cytoxicity curves, respectively, for inhibition of proliferation in Balb/3T3 cells by inventive compound no. 58.

Figures 67A and 67B illustrate that inventive compound no. 58 inhibits VEGF-induced proliferation in HUVEC and EGF-induced proliferation in Swiss/3T3 cells, respectively.

Figure 68 illustrates an effect of the delayed addition of inventive compound no. 58 to Balb/3T3 proliferation in response to PDGF-BB.

Figure 69 illustrates that inventive compound no. 58 inhibits PDGF- induced proliferation in Balb/3T3 cells to a greater extent than serum-induced proliferation.

Figure 70 illustrates that endothelial cell migration is inhibited by inventive compound no. 58.

Figure 71 illustrates that inventive compound no. 58 does not inhibit chemotaxis of human smooth muscle cells (SMC) to PDGF.

Figures 72A and 72B illustrate that inventive compound no. 58 inhibits THP-1 cell adhesion to either TNF or IL-1-stimulated HUVEC (respectively).

Figures 73A and 73B illustrate inhibition of VCAM or ICAM expression, respectively, in HUVEC activated by TNF by inventive compound no. 58.

Figure 74 illustrates that inventive compound no. 58 inhibits TNF release by inventive compound no. 58 using a human whole blood *ex vivo* assay.

### Detailed Description of the Invention

The invention provides a genus of compounds which can control cellular behavior by a particular phase of a secondary messenger pathway system (Bursten *et al*., "Interleukin-1 Rapidly Stimulates Lysophosphatidate Acyltransferase and Phosphatidate Phosphohydrolase Activities in Human Mesangial Cells," J. Biol. Chem., Vol. 266, No. 31, pages 20732-20743, November 5,1991). The second messengers are lipids or phospholipids and use the following abbreviations:
PE = phosphatidyl ethanolamine
LPE = lysophosphoethanolamine
PA = phosphatidic acid
LPA = lysophosphatidic acid
DAG = diacylglycerol
LPLD = lysophospholipase-D
LPAAT = lysophosphatidic acid acyl transferase
PAPH = phosphatidic acid phosphohydrolase
PLA2 = phospholipase A2
PLD = phospholipase D
PAA = phosphoarachidonic acid
PC = phosphatidyl choline
"remodeled" PA, cyclic pathway = PAA, LPA, PA and DAG intermediates substituted with 1-saturated, 2-linoleoyl or 1,2-dioleoyl, dioleoy/1,2-sn-dilinoleoyl at the indicated sn-1 and sn-2 positions.
"Classical PI Pathway" = PI, DAG, PA intermediates substituted with 1-stearoyl, 2-arachidonoyl fatty acyl side chains.
"PLD-generated PA" = PE, PC, LPA, PA and DAG intermediates substituted with, *e.g.*, 1,2-sn-dioleoyl-, 1-alkyl, 2-linoleoyl-, and 1-alkyl, 2-docosahexaenoyl-side chains.

Lysophosphatidic acid transferase (LPAAT) effects the synthesis of phosphatidic acid (PA) from lysophosphatidic acid (LPA) by incorporation of an acyl group from acyl CoA. Hydrolysis of the phosphate moiety by PA phosphohydrolase (PAPH) results in the formation of DAG. These aspects of the pathway appear to be activated immediately (within a minute) upon stimulation by a primary stimulus (*e.g.*, a cytokine such as IL-1, IL-2 or TNF) acting at a receptor on a cellular surface. An immediate detectable effect is an elevation of levels of PA and DAG. Administration of the compounds of the invention reverse this elevation.

The compounds and pharmaceutical compositions of the invention include inhibitors of subspecies of LPAAT and PAPH enzymes with substrate specificity for intermediates with 1,2-diunsaturated and 1-alkyl, 2-unsaturated subspecies. Each membrane phospholipid subclass (*e.g.*, PA, PI, PE, PC and PS) reaches a stable content of characteristic fatty acyl side chains due to cyclic remodeling of the plasma membrane as well as turnover for each subclass. PA is often stable, but present in relatively small quantities. PA in resting cells consists mostly of saturated acyl chains, usually consisting of myristate, stearate and palmitate. In resting cells, PC's acyl side chains consist mostly of acyl palmitate in the sn-1 position and oleate in the sn-2 position. PE and PI are predominantly composed of sn-1 stearate and sn-2 arachidonate.

Due to this characteristic content of acyl groups in the sn-1 and sn-2 positions, the origin of any PA species may be deduced from the chemical nature of its acyl groups in the sn-1 and sn-2 positions. For example, if PA is derived from PC through action of the enzyme PLD, the PA will contain the characteristic acyl side chains of PC substrate passed through the second messenger pathway. Further, the origin of any 1,2 sn-substrate species may be differentiated as to its origin. However, it is important to know whether or not each phospholipid species passes through a PA form previous to hydrolysis to DAG. The lyso-PA that is converted to PA and thence to DAG may be shown. The complexities of this second messenger pathway can be sorted by suitable analyses by fatty acyl side chain chemistry (*i.e.,* by thin layer chromatography, gas-liquid chromatography, or high pressure liquid chromatography) of intermediates in cells at various time points after stimulation of the second messenger pathway.

In certain meseachymal cells, such as neutrophils and rat or human mesangial cells, several signaling pathways may be activated in tandem, simultaneously or both. For example, in neutrophils, F-Met-Leu-Phe stimulates formation of PA through the action of PLD, followed in time by formation of DAG through the action of PAPH. Several minutes later, DAG is generated from PI through the classical phosphoinositide pathway. In many cells, DAG is derived from both PA that is being remodeled through a cycle whereby PA is sn-2 hydrolyzed by PLA2, followed by sn-2 transacylation by LPAAT, and a PLD-pathway from PA that is generated from either PE or PC or both substrates by PLD.

The present second messenger pathway involves substrates with unsaturated fatty acids in the sn-2 position other than arachidonate and those sub species of PAPH and LPAAT that are not involved in normal cellular housekeeping functions that are part of the classical PI pathway.

The PAPH and LPAAT enzymes involved in the present second messenger pathway are exquisitely stereo specific for different acyl side chains and isomeric forms of substrates. Therefore, the inventive compounds are preferably, substantially enantiomerically pure, and preferably are the R enantiomer at the chiral carbon atom bonded to the hydroxyl group.

An additional signaling pathway associatated with inflammatory transduction and cell membrane perturbation generates a separate PA species, enriched in myristate and derived from GlyPI, as described above. Under these signaling conditions, the inventive compounds prevent activation of or directly inhibit the PiG-PLD, hydrolyzing GlyPI to PA and glycan inositol. In some tumor cells and TNF-activated cells (*i.e.*, Type II receptors), the inventive compounds' efficacy may be dual inhibition of both LPAAT and GlyPI hydrolysis. Experimental results confirm this inhibitive effect. Stimulation of CT-6 cells with IL-2 results in rapid hydrolysis of GlyPI species 15-45 seconds after stimulation, followed by rapid resynthesis of GlyPI. The inventive compounds prevent this hydrolysis and stimulate GlyPI synthesis, resulting in a significant GlyPI increase throughout stimulation without evidence of hydrolysis or formation of GlyPI-derived PA. Stimulation of human umbilical vein endothelial cells with TNF results in LPAAT-derived and Gly-PI-derived PA species. The inventive compounds inhibit formation of both PA species. Accumulation of lyso-PA and Gly-PI results.

### Therapeutic Uses of the Inventive Compounds

The specific activation and/or inhibition of the second messenger pathway, as described above, activated primarily by various noxious stimuli, suggests that the inventive compounds are useful in treating a wide variety of clinical indications. Moreover, *in vitro* and *in vivo* data, presented herein, provides predictive data that a wide variety of clinical indications, having similar effects on the specific second messenger pathway, may be treated by the inventive compounds, which specifically inhibit the second messenger pathway activated by noxious stimuli and mediated through, for example, inflammatory cytokines. In fact, the mechanism of action for the inventive compounds explains why these compounds have multifarious clinical indications.

Activation of the second messenger pathway is a major mediator of response to noxious stimuli and results in intracellular signalling that leads to clinical manifestations of, for example, acute and chronic inflammation, autoimmune diseases and cancer cell growth. However, all inhibitors (*i.e.*, the inventive compounds) do not inhibit all enzymes of this second messenger pathway. Signals mediated by the present second messenger pathway include, for example, those cellular responses of LPS directly, T and B cell activation by antigen, cellular responses to first messengers, such as, IL-1- and TNF, growth stimulated by transformations including, but not limited to, activated oncogenes (*e.g., ras, abl, her2-neu* and the like), smooth muscle cell proliferation stimulated by platelet derived growth factor (PDGF), b-FGF, epidermal growth factor (EGF), vascular endothelial growth factor (VEGF) and IL-1, T and B cell growth stimulation by IL-2, IL-4 or IL-7; and more generally, T cell receptor signaling.

The inventive compounds: (1) block IL-1 signal transduction through the Type I receptor as shown, for example, by preventing IL-1 and IL-1 plus PDGF induction of proliferation of smooth muscle, endothelial and kidney mesangial cells (LPAAT effect); (2) suppress up-regulation of adhesion molecules as shown, for example, by blocking VCAM in endothelial cells; (3) inhibit TNF, LPS and IL-1 induced metalloproteases (an inflammation and cancer metasteses model); (4) block LPS, TNF or IL-1 induced secondary cytokine production (for prevention and treatment of septic shock symptoms); (5) suppress T and B cell activation by antigen, for example, IL-2 and IL-4; (6) inhibit mast cell activation by immunoglobulin E (IgE); (7) are cytotoxic for transformed cells and tumor cell lines, yet not for normal cells at equivalent doses; and (8) block signaling by IL-2, IL-4, IL-6 and IL-7 on T and B cells.

The foregoing cellular results are the basis for the following pharmacologic effects, including, but not limited to, protection and treatment of endotoxic shock and sepsis induced by gram positive or gram negative bacteria, inhibition and prevention of tumor cell growth and metastatic spread, synergistic immunosuppression, treatment of autoimmune diseases, suppression of allograft reactions, stimulation of hair growth (*e.g.* treatment of baldness or prevention of hair loss due to cytoreductive therapies), and treatment of hyperproliferative skin disorders such as psoriasis (*e.g.* cytoreductive therapies, IL-2 therapy, cyclosporin A, amphoteracin B, etc.). through reversal of an apoptotic process. The inventive compounds are most potent when used to prevent and treat septic shock, treat acute and chronic inflammatory disease, suppress an immune response, treat or prevent an autoimmune disease and stimulate hair growth (when applied topically).

The inventive compounds also are useful as an adjuvant to inhibit toxic side effects of drugs whose side effects are mediated through the present second messenger pathway. Metalloproteases mediate tissue damage such as glomerular diseases of the kidney, joint destruction in arthritis, and lung destruction in emphysema, and play a significant role in tumor metastases. Three examples of metalloproteases include a 92 kD type V gelatinase induced by TNF, IL-1 and PDGF plus b-FGF, a 72 kD type IV collagenase that is usually constitutively produced and stimulated by TNF or IL-1, and a stromelysin/PUMP-1 induced by TNF and IL-1. The inventive compounds can inhibit TNF or IL-1 induction of the 92 kD type V gelatinase inducable metalloprotease. Moreover, the inventive compounds can reduce PUMP-1 activity induced by 100 U/ml of IL-1. Accordingly, the inventive compounds prevent induction of certain metalloproteases induced by IL-1 or TNF and are not involved with constitutively produced proteases *(e.g.,* 72 kD type IV collagenase) involved in normal tissue remodeling.

The inventive compounds inhibit IL-1 signal transduction, and are therefore considered as IL-1 antagonists. A recent review article entitled "Mechanisms of Disease: The Role of Interleukin-1 in Disease" (Dinarello *et al.*, N. Engl. J. Med., Vol. 328, No. 2, pages 106-113, January, 14, 1993) described the role of IL-1 as "an important rapid and direct determinant of disease." "In septic shock, for example, IL-1 acts directly on the blood vessels to induce vasodilatation through the rapid production of platelet activating factor and nitric oxide, whereas in autoimmune disease it acts by stimulating other cells to produce cytokines or enzymes that then act on the target tissue." The article describes a group of diseases that are mediated by IL-1, including sepsis syndrome, rheumatoid arthritis, inflammatory bowel disease, acute and myelogenous leukemia, insulin-dependent diabetes mellitus, atherosclerosis and other diseases including transplant rejection, graft versus host disease (GVHD), psoriasis, asthma, osteoporosis, periodontal disease, autoimmune thyroiditis, alcoholic hepatitis, premature labor secondary to uterine infection and even sleep disorders. Since the inventive compounds are IL-1 antagonists, the inventive compounds are useful for treating all of the above-mentioned diseases.

For example, for sepsis syndrome, the mechanism of IL-1-induced shock appears to be the ability of IL-1 to increase the plasma concentrations of small mediator molecules such as platelet activating factor, prostaglandin and nitric oxide. These substances are potent vasodilators and induce shock in laboratory animals. Blocking the action of IL-1 prevents the synthesis and release of these mediators. In animals, a single intravenous injection of IL-1 decreases mean arterial pressure, lowers systemic vascular resistance, and induces leukopenia and thrombocytopenia. In humans, the intravenous administration of IL-1 also rapidly decreases blood pressure, and doses of 300 ng or more per kilogram of body weight may cause severe hypotension. The therapeutic advantage of blocking the action of IL-1 resides in preventing its deleterious biologic effects without interfering with the production of molecules that have a role in homeostasis. The present inventive compounds address the need identified by Dinarello *et al.* inhibiting IL-1 cellular signaling.

With regard to rheumatoid arthritis, Dinarello *et al.* state: "Interleukin-1 is present in synovial lining and synovial fluid of patients with rheumatoid arthritis, and explants of synovial tissue from such patients produce IL-1 *in vitro.* Intraarticular injections of interleukin-1 induce leukocyte infiltration, cartilage breakdown, and periarticular bone remodeling in animals. In isolated cartilage and bone cells *in vitro,* interleukin- 1 triggers the expression of genes for collagenases as well as phospholipases and cyclooxygenase, and blocking its action reduces bacterial-cell-wall-induced arthritis in rats." Therefore, the inventive compounds, as IL-1 antagonists, are useful to treat and prevent rheumatoid arthritis.

Inflammatory bowel disease, ulcerative colitis and Crohn's disease are characterized by infiltrative lesions of the bowel that contain activated neutrophils and macrophages. IL-1 can stimulate production of inflammatory eicosanoids such as prostaglandin E₂ (PGE₂) and leukotriene B₄ (LTB₄) and IL-8, an inflammatory cytokine with neutrophil-chemoattractant and neutrophil-stimulating properties. Tissue concentrations of PGE₂ and LTB₄ correlate with the severity of disease in patients with ulcerative colitis, and tissue concentrations of IL-1 and IL-8 are high in patients with inflammatory bowel disease. Therefore, an IL-1 antagonist, such as the inventive compounds, is effective to treat inflammatory bowel disease.

With regard to acute and chronic myelogenous leukemia, there is increasing evidence that IL-1 acts as a growth factor for such tumor cells. Therefore, the inventive compounds are effective to prevent disease deterioration for acute and chronic myelogenous leukemias.

IDDM is considered to be an autoimmune disease with destruction of beta cells in the islets of Langerhans mediated by immunocompetent cells. Islets of animals with spontaneously occurring IDDM (*e.g.,* BB rats or NOD mice) have inflammatory cells that contain IL-1. Therefore, the inventive compounds are useful for the prevention of and treatment of IDDM.

IL-1 also plays a role in the development of atherosclerosis. Endothelial cells are a target of IL-1. IL-1 stimulates proliferation of vascular smooth muscle cells. Foam cells isolated from fatty arterial plaques from hypercholesterolemic rabbits contain IL-1β and IL-1β messenger RNA. The uptake of peripheral blood monocytes results in initiation of IL-1 production by these cells. IL-1 also stimulates production of PDGF. Taken together, IL-1 plays a part in the development of atherosclerotic lesions. Therefore, an IL-1 antagonist, such as the inventive compounds is useful in preventing and treating atherosclerosis.

DAG and PA are up-regulated in oncogenically transformed cells. For example, activating *ras* mutations result in increased generation of DAG on stimulation with mitogens. In non-transformed renal mesangial cells, IL-1β stimulation increased PLA2 and LPAAT activation, resulting in generation of sn-2 unsaturated PA and subsequent hydrolysis to DAG by phosphatidate phosphohydrolase. The *ras* transformation in NIH/3T3 cells up-regulates serum-stimulated generation of DAG and PA. The specific species of DAG stimulated by serum is dioleoyl and for PA, dilinoleoyl and dioleoyl. This upregulation occurs over 4-12 hours and pretreatment of cells with an inventive compound blocks generation of these phospholipid second messengers. The inhibition occurs either through suppressing the generation of PA *de novo* from lysoPA, or through inhibition of one or both arms of the Lands cycle. Therefore *ras* transformation mediates an up-regulation of specific PA species through indirect stimulation of PLA2 and/or LPAAT activity. The inventive compounds inhibit the conversion of the upregulated lysoPA to PA and subsequently block cellular phenotypic changes induced by PA/DAG in the membrane.

The ability of the inventive compounds to inhibit generation of unsaturated phospholipids is mirrored by the ability of inventive compounds to inhibit proliferation and tumorogenicity of *ras*-transformed cells *in vitro* and *in vivo.* This inhibition is reversible and is not associated with significant cytotoxicity.

Excessive or unregulated TNF (tumor necrosis factor) production is implicated in mediating or exacerbating a number of diseases including rheumatoid arthritis, rheumatoid spondylitis, osteoarthritis, gouty arthritis and other arthritic conditions, sepsis, septic shock, endotoxic shock, gram negative sepsis, toxic shock syndrome, adult respiratory distress syndrome, cerebral malaria, chronic pulmonary inflammatory disease, silicosis, pulmonary sarcoidosis, bone resorption diseases, reperfusion injury, graft versus host reaction, allograft rejections, fever, myalgias due to infection such as influenza, cachexia secondary to infection, AIDS or malignancy, other viral infections (*e.g.,* CMV, influenza, adenovirus, herpes family), keloid formation, scar tissue formation, Crohn's disease, ulcerative colitis, or pyresis. The inventive compounds or pharmaceutically acceptable salts thereof can be used in the manufacture of a medicament for the prophylactic or therapeutic treatment of any disease state in a human or other mammal, which is exacerbated or signaled through the present second messenger cellular phospholipid-based signaling pathway and by excessive or unregulated production of "first messenger" inflammatory cytokines such as TNF or IL-1. With regard to TNF first messenger signaling, there are several disease states in which excessive or unregulated TNF production by monocytes/macrophages is implicated in exacerbating or causing the disease.
These include, for example, neurodegenerative diseases such as Alzheimers disease, endotoxemia or toxic shock syndrome (Tracey *et al.,* "Anti-cachectin/TNF Monoclonal Antibodies Prevent Septic Shock During Lethal Bacteraemia," Nature, Vol. 330, pages 662-664, December 17, 1987 and Hinshaw *et al.,* "Survival of Primates in LD₁₀₀ Septic Shock Following Therapy With Antibody to Tumor Necrosis Factor (TNFα)," Circ. Shock, Vol. 30, pages 279-292, 1990); cachexia (Dezube *et al.,* "Pentoxifylline and Wellbeing in Patients with Cancer," The Lancet, page 662, March 17, 1990), and adult respiratory distress syndrome (Miller *et al.,* "Tumour Necrosis Factor in Bronchopulmonary Secretions of Patients with Adult Respiratory Distress Syndrome," The Lancet, pages 712-713, September 23, 1989). The inventive compounds may be used topically in the treatment of prophylaxis of topical disease states mediated or exacerbated by excessive TNF or IL-1, such as viral infections (herpes or viral conjunctivitis), psoriasis, fungal or yeast infections (ringworm, athletes foot, vaginitis, dandruff, etc.) or other dermatologic hyperproliferative disorders. High TNF levels have been implicated in acute malaria attacks (Grau *et al.,* "Tumor Necrosis Factor and Disease Severity in Children with Falciparum Malaria," N. Engl. J. Med., Vol. 320, No. 24, pages 1586-1591, June 15, 1989), chronic pulmonary inflammatory diseases such as silicosis and asbestosis (Piguet *et al.,* "Requirement of Tumour Necrosis Factor for Development of Silica-induced Pulmonary Fibrosis," Nature, Vol. 344, pages 245-247, March 15, 1990, and Bissonnette *et al.,* "Pulmonary Inflammation and Fibrosis in a Murine Model of Asbestosis and Silicosis," Inflammation, Vol. 13, No. 3, pages 329-339, 1989), and reperfusion injury (Vedder *et al.,* "Inhibition of Leukocyte Adherence by Anti-CD18 Monoclonal Antibody Attenuates Reperfusion Injury in the Rabbit Ear, Proc. Natl. Acad. Sci. USA, Vol. 87, pages 2643-2646, April 1990).

The invention includes methods for preparing a medicament for treating or preventing clinical symptoms of various disease states or reducing toxicity of other treatments by inhibiting cellular signaling through the second messenger pathway. Disease state or treatment-induced toxicity are selected from the group consisting of proliferation of tumor cells in response to an activated oncogene; hematocytopenia caused by cytoreductive therapies; autoimmune diseases caused by a T cell response or a B cell response and antibody production; septic shock; resistance of mesenchymal cells to tumor necrosis factor (TNF); proliferation of smooth muscle cells endothelial cells, fibroblasts and other cell types in response to growth factors, such as PDGF, FGF, EGF and VEGF (*i.e*., atherosclerosis, restenosis, stroke, and coronary artery disease); human immunodeficiency virus infection (AIDS and AIDS related complex); proliferation of kidney mesangial cells in response to IL-1, MIP-1α, PDGF or FGF; inflammation; kidney glomerular or tubular toxicity in response to cyclosporin A or amphotericin B treatment; organ toxicity (*e.g*., gastrointestinal or pulmonary epithelial) in response to a cytoreductive therapy (*e.g*., cytotoxic drug or radiation); enhancing antitumor effects of non-alkylating antitumor agents; allergies in response to inflammatory stimuli (*e*.*g*., TNF, IL-1 and the like) characterized by production of cell surface metalloproteases or by degranulation of mast cells and basophils in response to IgE, bone diseases caused by overproduction of osteoclast-activating factor (OAF) by osteoclasts, CNS diseases caused by reduced signal transduction of the neurotransmitters epinephrine and acetylcholine, and combinations thereof. The inventive compounds are also useful as antimicrobial agents to directly treat fungal or yeast infections and to indirectly treat bacterial or viral infections through an immune stimulation and pro-hematopoietic effect.

In summary, the compounds and pharmaceutical compositions thereof: (1) inhibit proliferation of tumor cells; (2) suppress activation of T-cells by antigen or IL-2 stimulation; (3) suppress activation of monocyte/macrophage cells by endotoxin, TNF, IL-1 or GM-CSF stimulation; (4) suppress antibody production of B-cells in response to an antigen, IL-4 or CD40 ligand; (5) inhibit the proliferation of smooth muscle cells in response to growth factors capable of stimulating said proliferation; (6) lower systemic vascular resistance conferred by endothelial cells; (7) lower systemic vascular resistance induced by endothelial cells; (8) lower expression of adhesion molecules induced by enhancers thereof; (9) suppress the activation of T-cells and macrophages by HIV; (10) inhibit the proliferation of kidney mesangial cells in response to stimulation by IL-1 and/or MIP-1α and/or PDGF and/or FGF; (11) enhance the resistance of kidney glomerular or tubular cells to cyclosporin A or amphotericin B; (12) prevent the release of MIP-1α by IL-1, TNF, or endotoxin stimulated monocytes and macrophages; (13) prevent the release of platelet activating factor by IL-1, TNF, or endotoxin treated megakaryocytes, fibroblastic cells, and macrophages; (14) prevent the down-regulation of receptors for cytokines in TNF-treated hematopoietic progenitor cells; (15) suppress the production of metalloproteases in IL-1-stimulated or TNF-stimulated glomerular epithelial cells or synovial cells; (16) enhance the resistance of gastrointestinal or pulmonary epithelial cells to cytotoxic drugs or radiation; (17) enhance the antitumor effect of a non-alkylating antitumor agent; (18) to inhibit the production of osteoclast activating factor in response to IL-1; (19) inhibit degranulation in response to IgE; (20) enhance the release of adrenergic neural transmitters, dopamine, norepinephrine, or epinephrine, or the neurotransmitter, acetylcholine; (21) modulate the post-synaptic "slow current" effects of the adrenergic neurotransmitters dopamine, epinephrine, or norepinephrine, or the neurotransmitter acetylcholine; (22) suppress signaling by neurotransmitters including acetyl choline, leuenkephalin and seretonin; or (23) increase seizure theshold.

The inventive compounds are also useful to raise the seizure threshold, to stabilize synapses against neurotoxins such as strychnine, to potentiate the effect of anti-Parkinson drugs such as L-dopa, to potentiate the effects of soporific compounds, to relieve motion disorders resulting from administration of tranquilizers, and to diminish or prevent neuron overfiring associated with progressive neural death following cerebral vascular events such as stroke. In addition, the compounds of the invention are useful in the treatment of norepinephrine-deficient depression and depressions associated with the release of endogenous glucocorticoids, to prevent the toxicity to the central nervous system of dexamethasone or methylprednisolone, and to treat chronic pain without addiction to the drug. Further, the compounds of the invention are useful in the treatment of children with learning and attention deficits and generally improve memory in subjects with organic deficits, including Alzheimer's patients.

### Compounds of the Invention

The invention provides for a class of compounds that are effective agents to inhibit specific cellular signaling events. The inventive compounds and inventive pharmaceutical compositions thereof have the formula:

(X)j - (core moiety),

wherein j is an integer from one to three, the core moiety is a cyclic or heterocyclic core moiety having from one to three, five- to six-membered ring and X is a racemic mixture, R or S enantiomer, solvate, hydrate, or salt of: 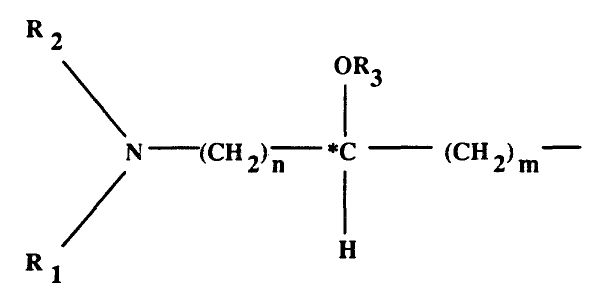 wherein *C is a chiral carbon atom; n is one; the carbon atom of (CH₂)ₙ may be substituted by a keto or hydroxy group; m is an integer from eight to fourteen; independently, R₁ and R₂ are hydrogen, a straight or branched chain alkane or alkene of up to twelve carbon atoms in length, or -(CH₂)_{w}R₅, w being an integer from two to fourteen and R₅ being a mono-, di- or tri-substituted or unsubstituted aryl group, substituents on R₅ being hydroxy, chloro, fluoro, bromo, or C₁₋₆ alkoxy; or jointly, R₁ and R₂ form a saturated or unsaturated heterocyclic group having from four to eight carbon atoms, N being a hetero atom; and R₃ is hydrogen or C₁₋₃;

A core moiety is at least one five- to six-membered ring, preferably having from one to three, five- to six-membered ring structures in a predominantly planar configuration. Preferably, the substituent (X) is bonded to a ring nitrogen if one exists. For example, the core moiety may be selected from the group consisting of substituted or unsubstituted barbituric acid; benzamide; benzene; biphenyl; cyclohexane; cyclohexanedione; cyclopentanedione; lactam; glutarimide; homophthalimide; hydrophthalimide; imidazole; imidazole amide; indomethacin; isocarbostyril; lumazine; napthlalene; phenol; pteridine; pthalimide; piperidine; pyridine; pyrimidine; pyrrole amide; quinolizinedione; quinazolinone; quinolone; resorcinol; salicylic acid and derivatives thereof; stilbene; succinimide; theobromine; thymine; triazine; tricyclododecane; uric acid; uracil; vitamins A, E or K; or xanthine.

Preferred cyclic cores include substituted or unsubstituted glutarimide, methylthymine, methyluracil, thymine, theobromine, uracil and xanthine, most preferably halogen-substituted xanthine. Exemplary preferred cores include: 1,3-cyclohexanedione, 1,3-cyclopentanedione; 1,3-dihydroxynaphthalene; 1-methyllumazine; methylbarbituric acid; 3,3-dimethylglutarimide; orotic acid; tetra or hexahydrophthalimide; orthophenol; prostacyclin; 2-hydroxypyridine; methyldihydroxypyrazolopyrimidine, specifically, 1,3-dimethyldihydroxypyrazolo[4,3-d]pyrimidine; methylpyrrolopyrimidine; 1-methylpyrrolo [2,3-d] pyrimidine; 1,3-dihydroxynapthalene; 1-pyrrole amides; 2-pyrrole amides; 3-pyrrole amides; 1,2,3,4-tetrahydroisoquinolone; 1-methyl-2,4(1H,3H)-quinolizinedione (1-methylbenzoyleneurea); quinazolin-4(3H)-one; sulindac; dihydrothymine; alkyl-substituted (C1-6) thymine; 2,4-dioxohexahydro-1.3.5tetrazine; methylthymine; alkyl-substituted (C1-6) uracil; uracil fused to naphthalene; 6-aminouracil; 1-methyl-5,6-dihydrouracil; 1-methyluracil; 5- and/or 6-position substituted uracils (such as, for example, 5-bromouracil); B-ionone as vitamin A; 2,6,6-methyl-1-cyclohexene-1-acetaldehyde as vitamin A; tetralone to vitamin K; 1,7-dimethylxanthine, 3,7-dimethylxanthine; 3-methylxanthine; 3-methyl-7-methylpivaloylxanthine; 8-substituted xanthines (having substituents such as N or S); and 7-methylhypoxanthine.

Preferably, X is bonded to a nitrogen of the core moiety, most preferably, the core moiety is xanthine and X is bonded to an N₁ xanthine nitrogen and N₃ and N₇ xanthine nitrogens are independently substituted by a member selected from the group consisting of hydrogen, methyl, fluoro, chloro and amino.

The invention provides a pharmaceutical composition comprising an inventive compound and a pharmaceutically acceptable excipient. The pharmaceutical composition may be formulated for oral, parenteral or topical administration to a patient.

The invention further provides a pharmaceutical composition comprising an inventive compound and a pharmaceutically acceptable excipient, the pharmaceutical composition being formulated for oral, parenteral or topical administration to a patient. A pharmaceutical composition may alternatively comprise one or a plurality of inventive compounds and a pharmaceutically acceptable carrier or excipient. Treatment of individuals with an inventive compound or pharmaceutical composition may include contacting with the inventive compound *in vitro* culture, in an extracorporeal treatment, or by administering (oral, parenteral or topical) the inventive compound or pharmaceutical composition to a subject whose cells are to be treated.

### Synthesis of the Inventive Compounds

The invention includes a method for preparing the inventive compounds. An exemplary method for preparing the inventive compounds is discussed below and in the following examples. In a synthesis according to the invention, a compound containing a desired core (intended as a "core moiety" in compounds of the invention) undergoes a reaction to produce an anion of the core-containing compound. Subsequently, the resulting anion is reacted with a substituted olefin to displace a targeted functional group on the olefin, resulting in an intermediate product. A predetermined amount of a core-containing compound is reacted with a suitable base, a solvent and a substituted olefin, the substituted olefin having at least one other functional group which may be substituted in a displacement reaction by the desired core-containing compound.

Preferred bases include, but are not limited to, sodium hydride, sodium amide, sodium alkoxide, lithium hydride, potassium hydride, lithium amide, sodium amide and potassium amide. An especially preferred base is sodium hydride. Preferred solvents may be dimethylsulfoxide, dimethylformamide, or an alcohol. Exemplary preferred alcohols include, but are not limited to, methanol, ethanol or isopropanol. Any substituted olefin comprising a chain structure of the inventive compounds may be used in the reaction according to the invention. Preferred olefins may be ω-substituted olefins. Preferred substituted olefins include, but are not limited to halo-substituted olefins.

The intermediate product, having a composite structure of the core-containing compound and substituted olefin, may subsequently be converted to a corresponding epoxide. In the method according to the invention, the intermediate product may be reacted with an organic peracid to obtain a desired epoxide. Preferred, exemplary organic peracids include 3-chloroperoxybenzoic acid, peracetic acid and trifluoroperacetic acid. An especially preferred peracid is 3-chloroperoxybenzoic acid.

Alternatively, the intermediate product may be converted first to a corresponding diol by reacting the intermediate product with a suitable oxidizing agent. Preferred oxidixing agents include, but are not limited to, osmium tetroxide. Preferred oxidizing agents, such as osmium tetroxide may require a catalytic amount of the oxidizing agent in the presence of a regenerating agent. Exemplary, regenerating agents may be 4-methylmorpholine-N-oxide and trimethylamine-N-oxide. An especially preferred regenerating agent is 4-methylmorpholine-N-oxide. In a subsequent halogenation reaction, the resulting diol is converted to a haloester using a halogenating agent in the presence of an organic acid. Exemplary halogenating agents include hydrogen bromide and hydrogen chloride. Preferred organic acids may be acetic acid and propionic acid. The resulting haloester is subsequently reacted with a basic ester-hydrolyzing reagent to obtain a desired epoxide product. Preferred ester-hydrolyzing agents include, but are not limited to metal alkoxides and metal hydroxides. Especially preferred metal alkoxides are sodium methoxide, ethoxide, isopropoxide and pentoxide. A preferred metal hydroxide is sodium hydroxide.

A final step in the inventive method is preparation of the desired inventive compound from a core-containing epoxide, synthesized in the foregoing procedure. The final step may be accomplished by either of two preferred methods. In a first method, the core-containing epoxide is heated in the presence of a substituted or unsubstituted amine having functional groups which are present in the final inventive compound. Preferred amine functional groups are disclosed above.

A second method comprises reacting the unsubstituted or substituted amine with the core-containing epoxide and a reaction activator in a solvent. Exemplary reaction activators include lithium perchlorate. Preferred solvents are disclosed above.

Exemplary, preferred compounds of the invention include both *R and S* enantiomers and racemic mixtures of the following compounds:

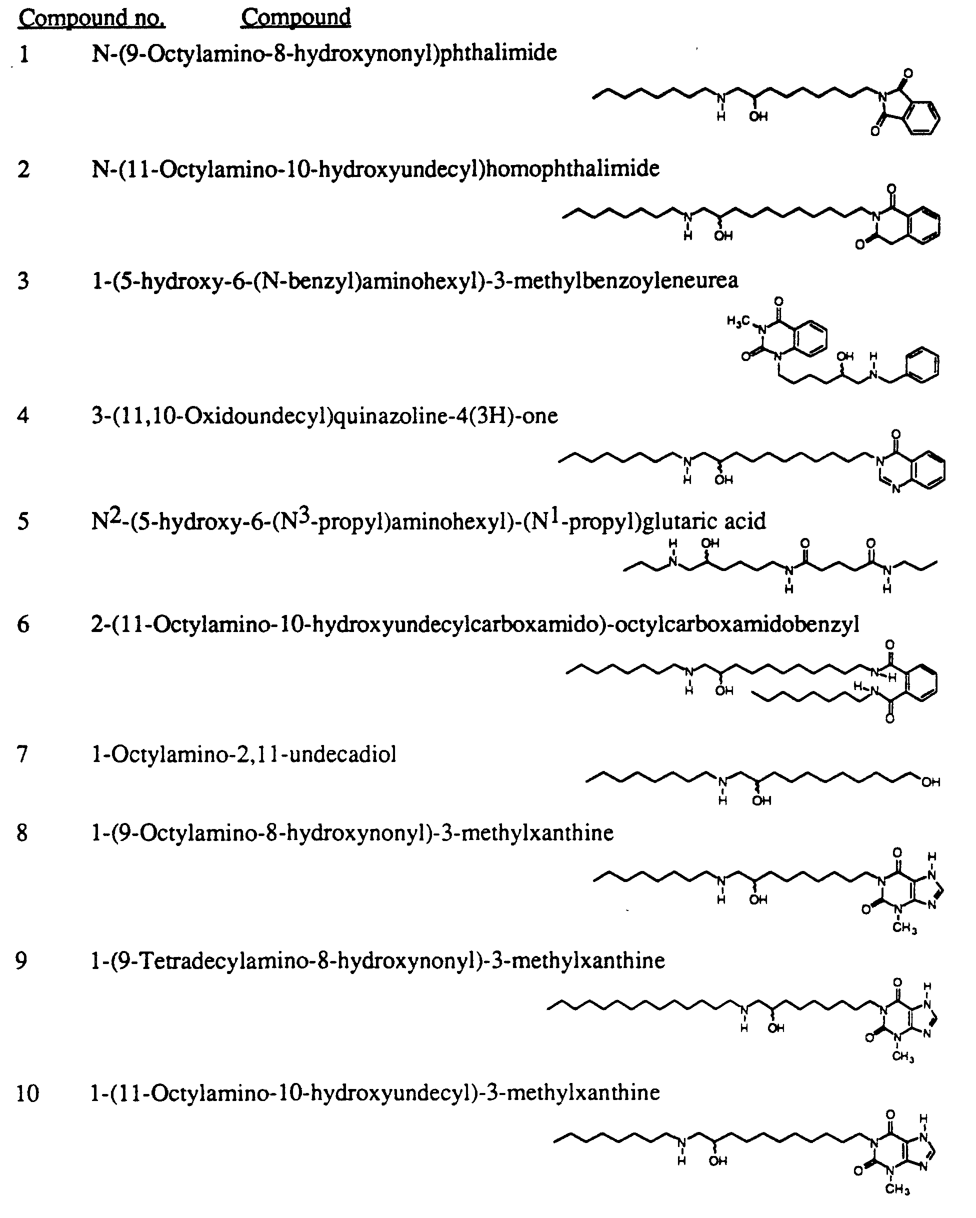

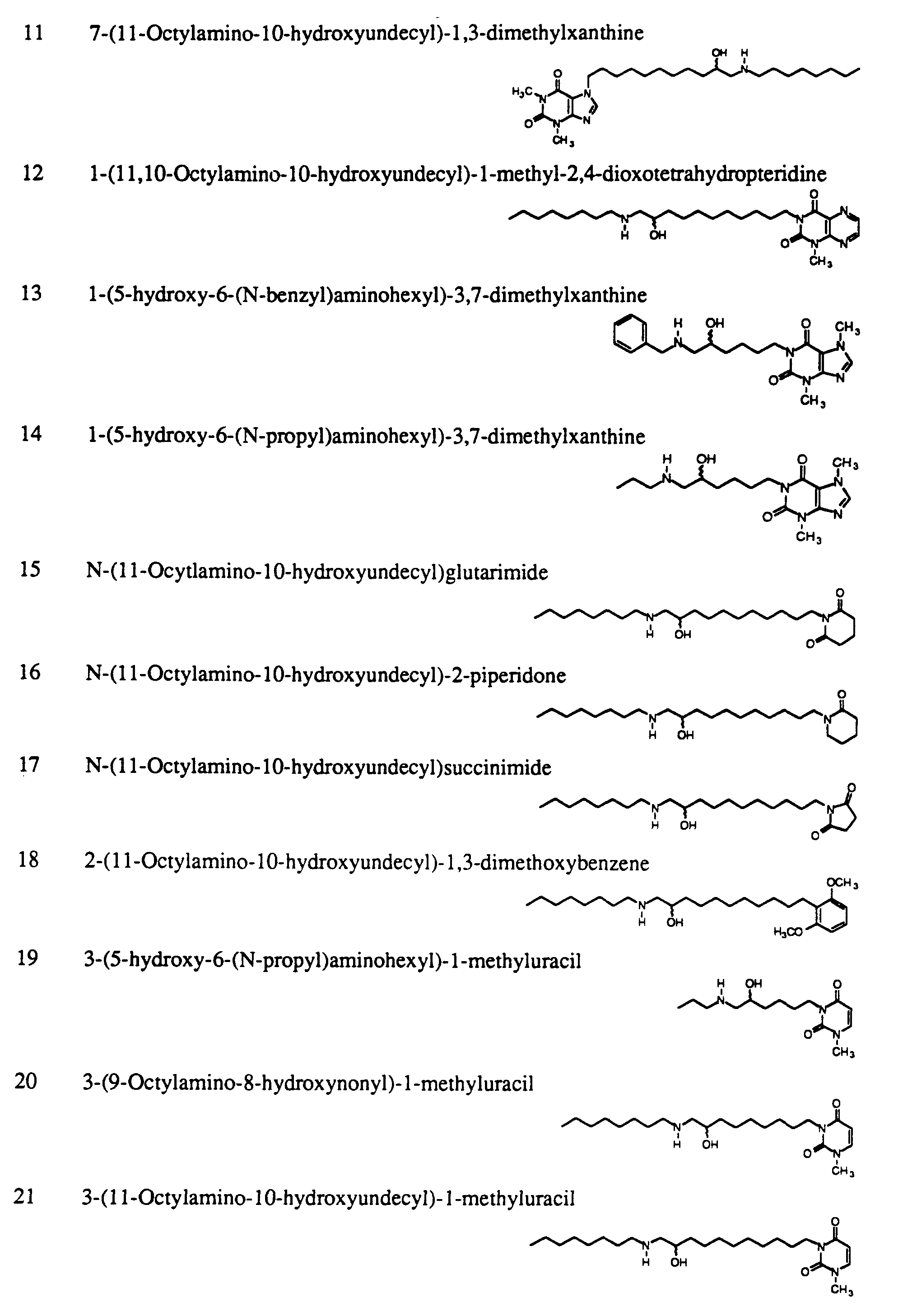

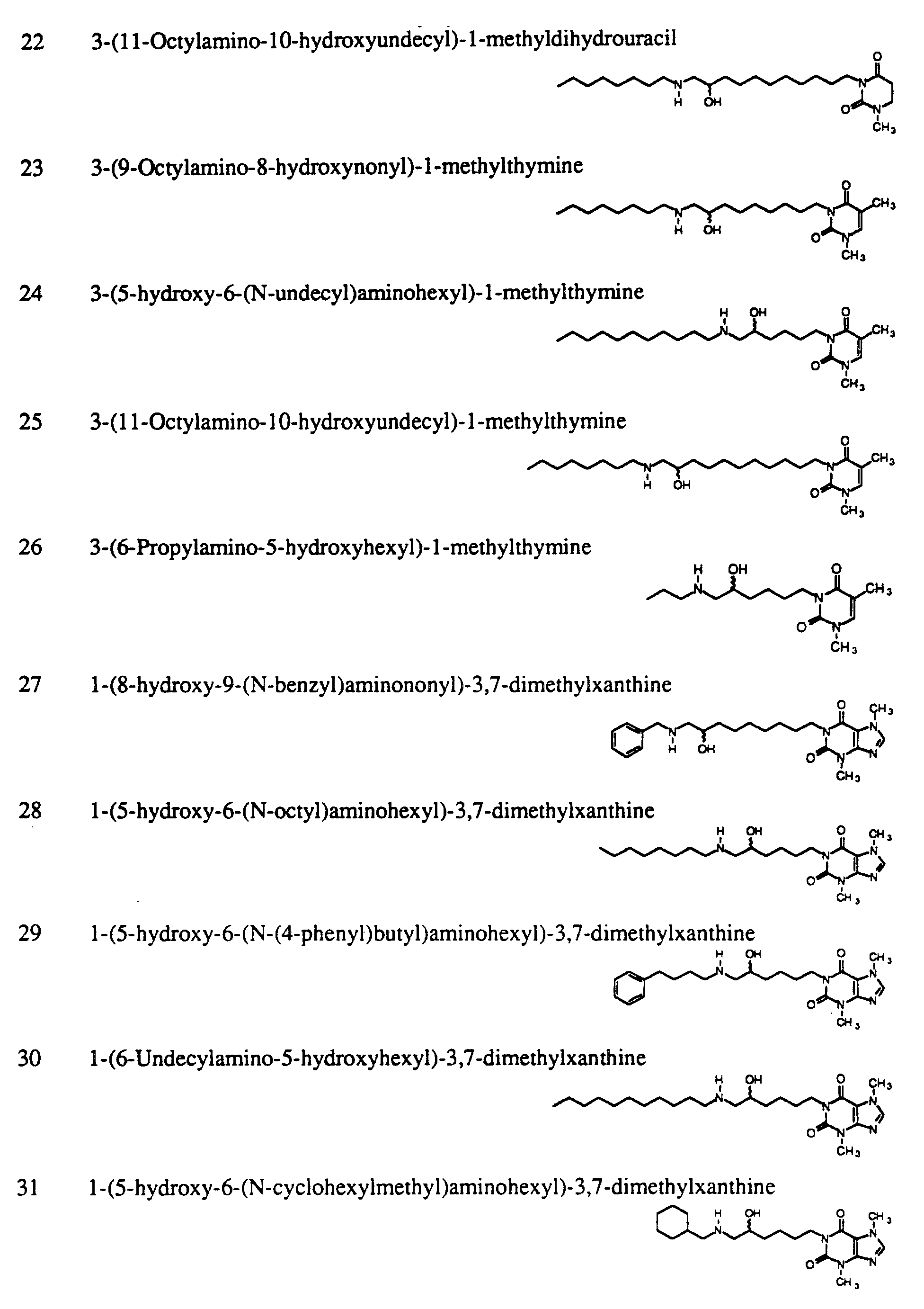

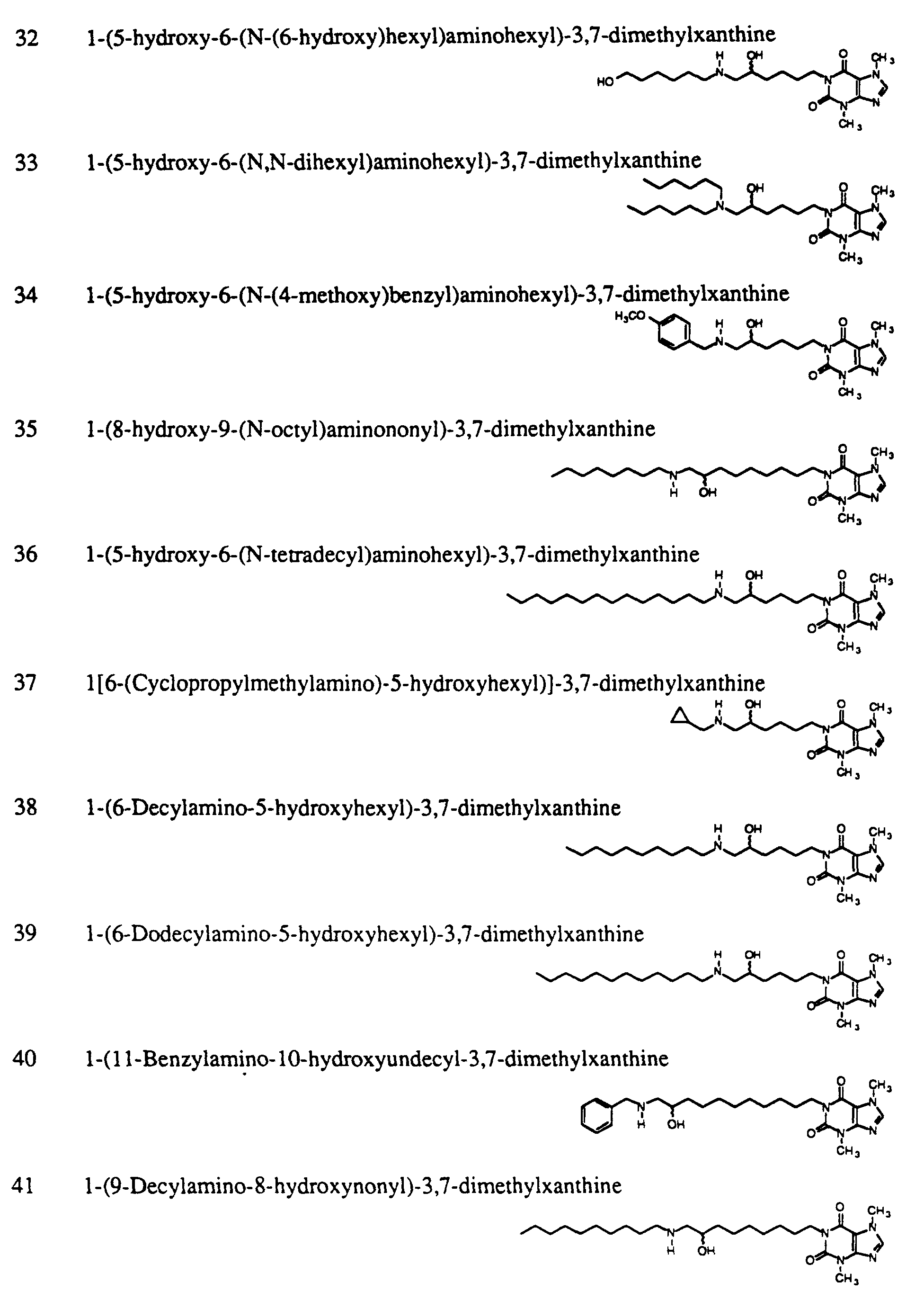

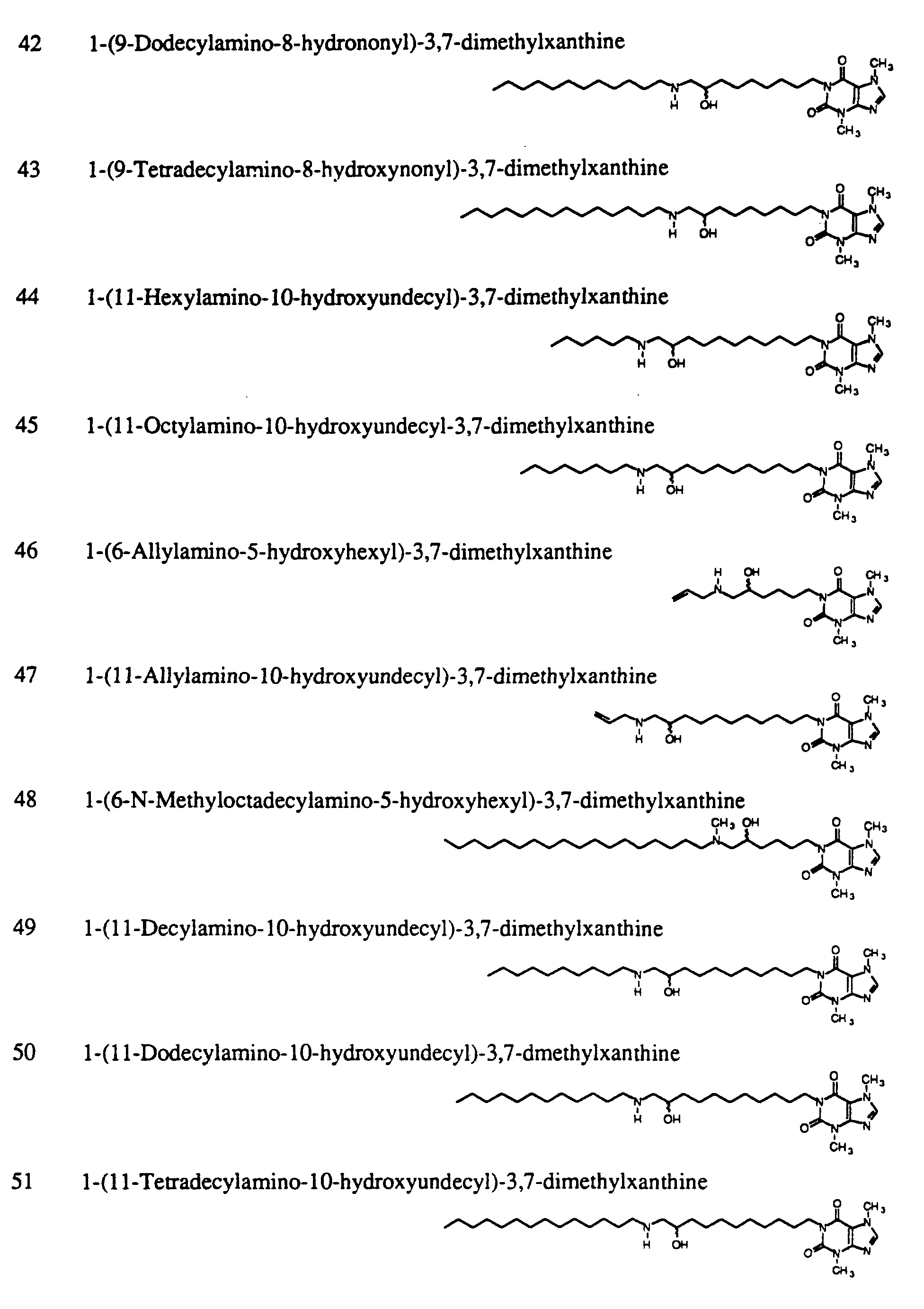

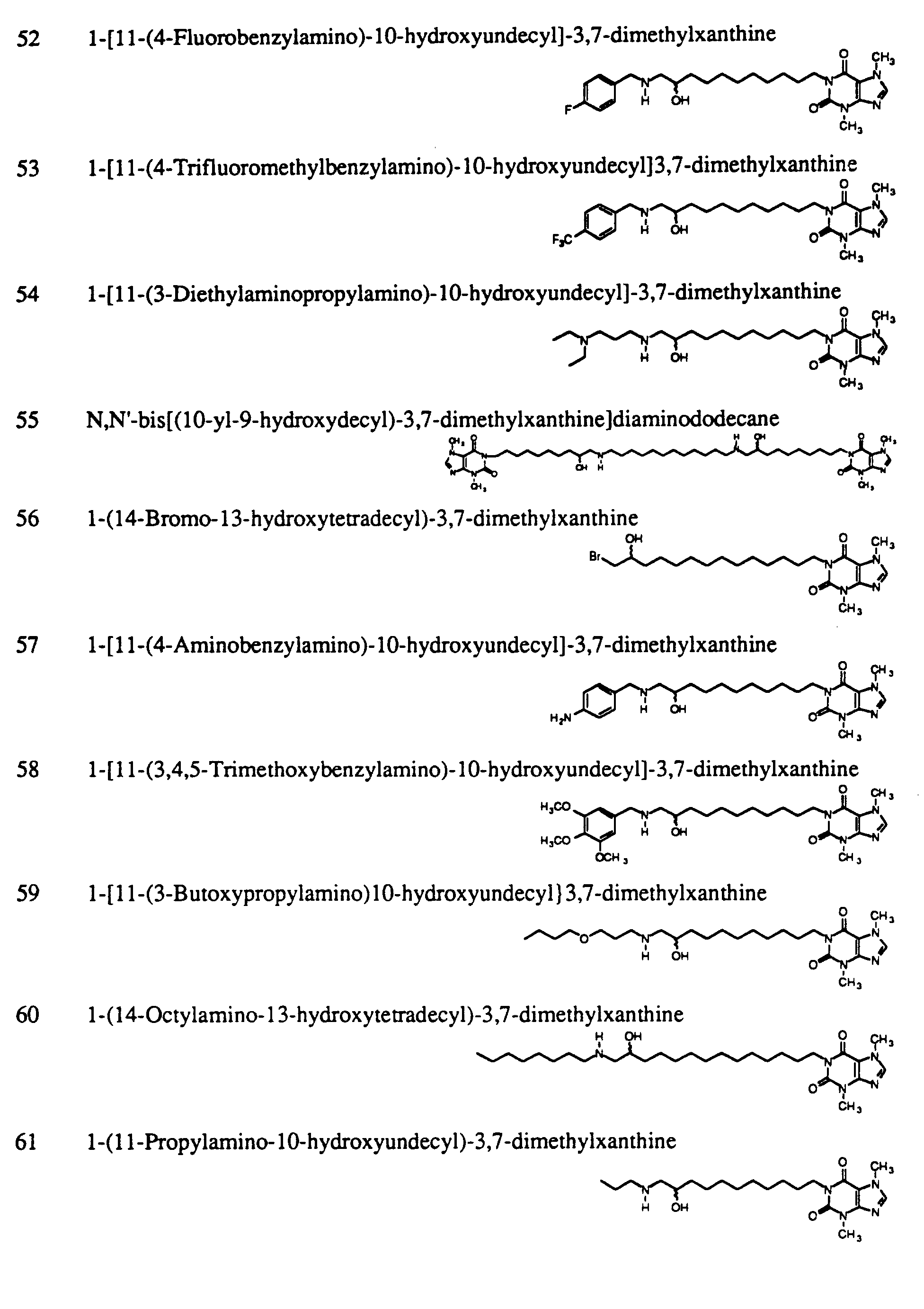

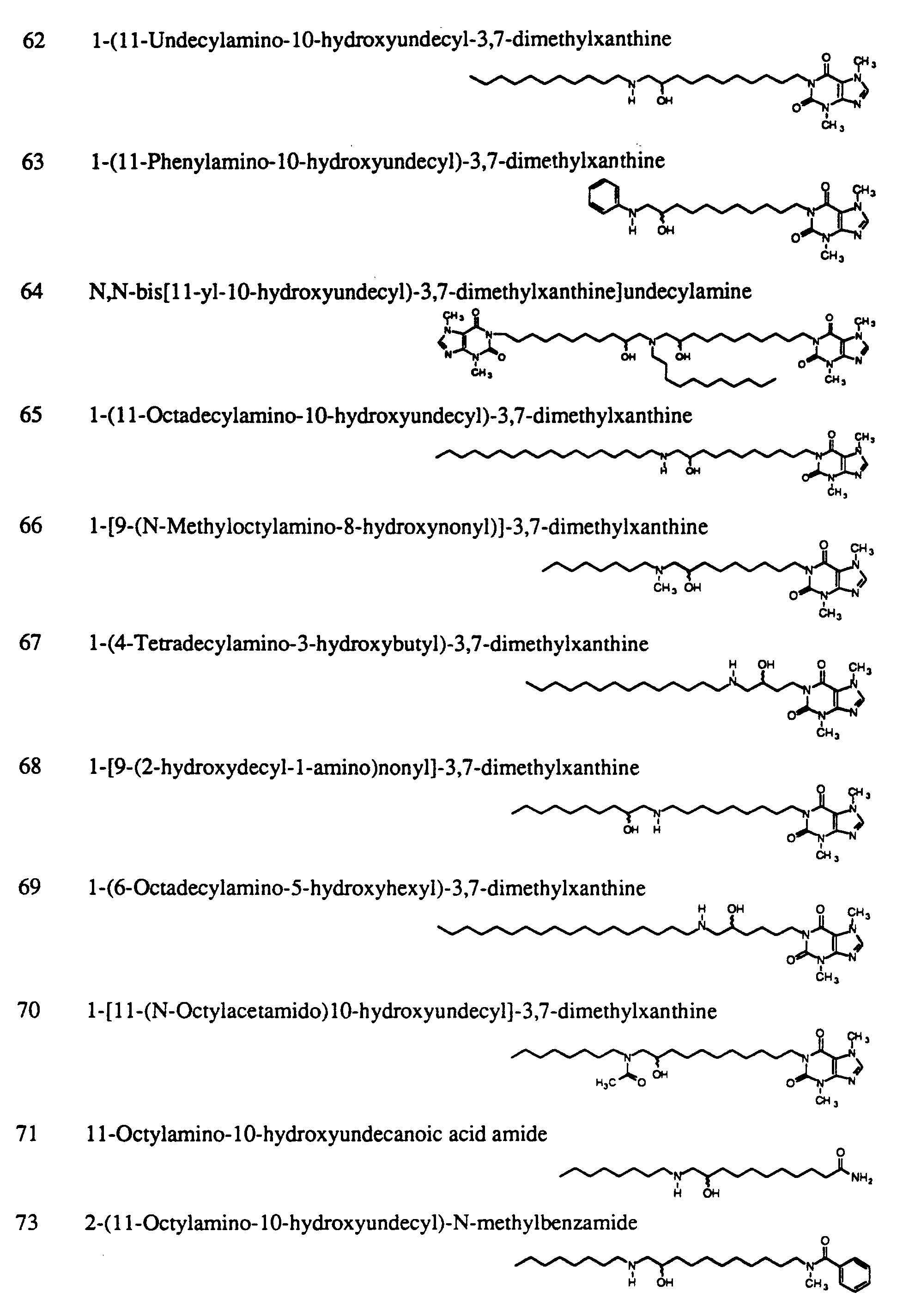

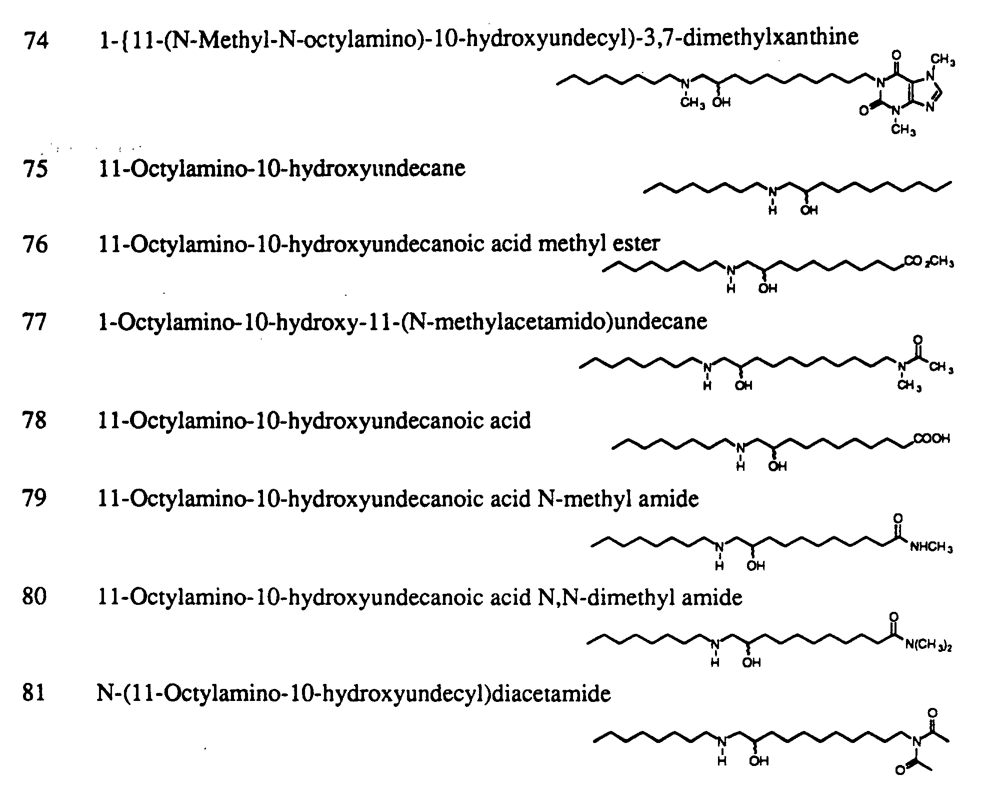

### Pharmaceutical Formulations

A suitable formulation will depend on the nature of the disorder to be treated, the nature of the medicament chosen, and the judgment of the attending physician. In general, the inventive compounds are formulated either for injection or oral administration, although other modes of administration such as transmucosal or transdermal routes may be employed. Suitable formulations for these compounds can be found, for example, in *Remington's Pharmaceutical Sciences* (latest edition), Mack Publishing Company, Easton, PA.

The inventive compounds and their pharmaceutically acceptable salts can be employed in a wide variety of pharmaceutical forms. The preparation of a pharmaceutically acceptable salt will be determined by the chemical nature of the compound itself, and can be prepared by conventional techniques readily available. Thus, if a solid carrier is used, the preparation can be tableted, placed in a hard gelatin capsule in powder or pellet form or in the form of a troche or lozenge. The amount of solid carrier will vary widely but preferably will be from about 25 mg to about 1 gram, wherein the amount of inventive compound per dose will vary from about 25 mg to about 1 gram for an adult. When a liquid carrier is used, the preparation will be in the form of a syrup, emulsion, soft gelatin capsule, sterile injectable liquid such as an ampule or nonaqueous liquid suspension. Where the inventive composition is in the form of a capsule, any routine encapsulation is suitable, for example, using the aforementioned carriers in a hard gelatin capsule shell. Where the composition is in the form of a soft gelatin shell capsule, any pharmaceutical carrier routinely used for preparing dispersions of suspensions may be considered, for example, aqueous gums, celluloses, silicates or oils and are incorporated in a soft gelatin capsule shell. A syrup formulation will generally consist of a suspension or solution of the compound or salt thereof in a liquid carrier (*e.g.*, ethanol, polyethylene glycol, coconut oil, glycerine or water) with a flavor or coloring agent.

The amount of inventive compound required for therapeutic effect on topical administration will, of course, vary with the compound chosen, the nature and severity of the disease and the discretion of the treatment provider. Parenteral includes intravenous, intramuscular, subcutaneous, intranasal, intrarectal, intravaginal or intraperitoneal administration. Appropriate dosage forms for such administration may be prepared by conventional techniques. A typical parenteral composition consists of a solution or suspension of the inventive compound or a salt thereof in a sterile or non-aqueous carrier, optionally containing a parenterally acceptable oil, for example polyethylene glycol, polyvinylpyrrolidone, lecithin, arachis oil, or sesame oil. The daily dosage for treatment of sepsis or another severe inflammatory condition via parenteral administration is suitable from about 0.001 mg/kg to about 40 mg/kg, preferably from about 0.01 mg/kg to about 20 mg/kg of an inventive compound or a pharmaceutically acceptable salt thereof calculated as the free base.

The inventive compounds may be administered orally. The daily dosage regimen for oral administration is suitably from about 0.1 mg/kg to about 1000 mg/kg per day. For administration the dosage is suitably from about 0.001 mg/kg to about 40 mg/kg of the inventive compound or a pharmaceutically acceptable salt thereof, calculated as the free base. The active ingredient may be administered from 1 to 6 times a day, sufficient to exhibit activity.

The inventive compounds may be administered by inhalation (*e.g.*, intranasal or oral). Appropriate dosage forms include an aerosol or a metered dose inhaler, as prepared by conventional techniques. The daily dosage is suitably from about 0.001 mg/kg to about 40 mg/kg of the inventive compound or a pharmaceutically acceptable salt thereof, calculated as the free base. Typical compounds for inhalation are in the form of a solution, suspension or emulsion that may be administered as a dry powder or in the form of an aerosol using a conventional propellant.

The invention is illustrated by the following examples.

### Example 1

This example illustrates the synthesis of several compounds that are used as intermediates for the synthesis of other compounds.

1-(8,9-Oxidononyl)-3,7-dimethylxanthine was synthesized as follows: A mixture of theobromine (17.64 g, 98 mmol) and sodium hydride (2.35 g, 98 mmol) in dimethylsulfoxide (250 ml) was stirred for 15 minutes. 9-Bromo-1-nonene (20.0 g, 98 mmol) was added and stirring continued for 3 days. The reaction mixture was poured into water (300 ml) and extracted with dichloromethane (4 x 200 ml). The combined organic layers were washed with saturated aqueous sodium chloride solution (2 x 150 ml), dried over sodium sulfate, and the solvents evaporated under vacuum. The residue was crystallized (dichloromethane-ether) to give 1-(8-nonenyl)-3,7-dimethylxanthine (24.34 g, 99% yield) as white crystals.

A solution of 1-(8-nonenyl)-3,7-dimethylxanthine (810 mg, 2.7 mmol), 4-methylmorpholine-N-oxide (340 mg, 2.9 mmol) and 2.5 % osmium tetroxide in *t*-butanol (3 drops) in acetone (20 ml) and water (20 ml) was stirred for 24 hours. Saturated aqueous sodium dithionite solution (5 ml) was added. After stirring for 15 minutes, the reaction was extracted with 25% ethanol-dichloromethane (4 x 50 ml). The combined organic phases were dried over sodium sulfate and the solvents evaporated under vacuum. The solid residue was recrystallized (ethanol-chloroform) to give 1-(8,9-Dihydroxynonyl)-3,7-dimethylxanthine (490 mg, 54% yield).

A mixture of 1-(8,9-dihydroxynonyl)-3,7-dimethylxanthine (428 mg, 1.3 mmol) and 30% hydrogen bromide in acetic acid (0.8 ml, 3.9 mmol) was stirred for 90 minutes. The solution was poured into a mixture of water (10 ml), sodium bicarbonate (1.35 g), and dichloromethane (10 ml). After 10 minutes of vigorous stirring the layers were separated and the aqueous portion was extracted with dichloromethane (3 x 15 ml). The combined organic phases were dried over sodium sulfate and the solvent was evaporated under vacuum to give 1-(8-acetoxy-9-bromononyl)-3,7-dimethylxanthine (550 mg, 96% yield) as a yellow oil. Without further purification, the oil was dissolved in methanol (5 ml) and then a 1 M solution of sodium methoxide in methanol (1.4 ml) was added. After 30 minutes the reaction mixture was poured into water (30 ml) and extracted with dichloromethane (3 x 40 ml). The combined organic phases were dried over sodium sulfate and the solvents evaporated under vacuum. The solid residue was recrystallized (dichloromethane-petroleum ether) to give 1-(8,9-oxidononyl)-3,7-dimethylxanthine (380 mg, 91% yield).

1-(5,6-Oxidohexyl)-3,7-dimethylxanthine was synthesized as follows: A mixture of 1-bromohexene (10.7 g, 66 mmol), sodium hydride (1.58 g, 66 mmol), and theobromine (11.9 g, 66 mmol) in dimethylsulfoxide (100 ml) was stirred for 43 hours. The solution was treated with water (200 ml) and then extracted with dichloromethane (3 x 80 ml). The combined extracts were washed with water (3 x 100 ml), dried over magnesium sulfate, and then the solvent was evaporated under vacuum to give 1-(5-hexenyl)-3,7-dimethylxanthine (17 g, 98% yield) as a white powder.

To 1-(5-hexenyl)-3,7-dimethylxanthine (1.07 g, 4.1 mmol) and 4-methylmorpholine-*N*-oxide (1.44 g, 12.3 mmol) in water (20 ml) and acetone (10 ml) was added 2.5% solution of osmium tetraoxide in *t*-butanol (6 drops). After stirring for 48 hours, the mixture was treated with 20% aqueous sodium dithionite solution (20 ml). After 2 minutes, the mixture was extracted with 25% ethanol-dichloromethane (3 x 30 ml). The combined extracts were dried over magnesium sulfate and the solvents were evaporated under vacuum to give 1-(5,6-dihydroxyhexyl)-3,7-dimethylxanthine (750 mg, 62% yield) as a white powder.

To 1-(5,6-dihydroxyhexyl)-3,7-dimethylxanthine (1.0 g, 3.38 mmol) was added 30% hydrogen bromide-acetic acid (3.4 mL) over 30 seconds and then stirred until all of the solid had dissolved (2.5 hours). The solution was poured carefully over a mixture of sodium bicarbonate (12 g) and ice water (50 ml). After carbon dioxide evolution had subsided, the mixture was extracted with dichloromethane (3 x 25 ml). The combined extracts were dried over magnesium sulfate and the solvent was evaporated under vacuum to give 1-(5-acetoxy-6-bromohexyl)-3,7-dimethylxanthine (1.3 g, 96% yield) as a viscous oil which was dissolved in methanol (5 mL). A 1M solution of sodium methoxide in methanol (3.9 ml) was added over 30 seconds. After stirring for 20 minutes, the solution was treated with water (20 ml) and then extracted with dichloromethane (3 x 15 ml). The combined extracts were dried over magnesium sulfate and the solvents were evaporated under vacuum to give 1-(5,6-oxidohexyl)-3,7-dimethylxanthine (900 mg, 100% yield) as white crystals.

3-(5,6-Oxidohexyl)-1-methyluracil was synthesized as follows: A mixture of sodium hydride (86 mg, 3.6 mmol) and 1-methyluracil (500 mg, 4 mmol) in dimethyl sulfoxide (25 ml) was stirred for 15 minutes, and then 6-bromo-1-hexene (647 mg, 4 mmol) was added. After stirring for 20 hours, the reaction mixture was poured into water (50 ml) and extracted with 20% ethanol-dichloromethane (3 x 50 ml). The combined organic layers were washed with saturated aqueous sodium chloride solution (20 ml) and dried over sodium sulfate. The solvent was evaporated under vacuum to give a residue which was purified by column chromatography (silica, ethyl acetate) to give 3-(5-hexenyl)-1-methyluracil (598 mg, 72% yield).

A solution of 3-(5-hexenyl)-1-methyluracil (598 mg, 2.9 mmol), 4-methylmorpholine-N oxide (408 mg, 3.5 mmol), and a 2.5% solution of osmium tetroxide in *t*-butanol (3 drops) in acetone (15 ml) and water (5 ml) was stirred for 3 days. Saturated aqueous sodium hydrosulfite solution (10 ml) was added and the mixture was stirred for 15 minutes. Water (50 ml) was added and the mixture was extracted with 20% ethanol-dichloromethane (4 x 40 ml). The combined organic layers were dried over sodium sulfate and the solvents were evaporated under vacuum to give 3-(5,6-dihydroxyhexyl)-1-methyluracil (461 mg, 66% yield) as a colorless oil.

3-(5,6-Dihydroxyhexyl)-1-methyluracil (350 mg, 1.4 mmol) was stirred with 30% hydrogen bromide in acetic acid (0.87 ml, 4.3 mmol) for 45 minutes. The solution was added to a mixture of sodium bicarbonate (1.6 g), water (10 ml) and dichloromethane (20 ml). After 15 minutes of vigorous stirring, the layers were separated and the aqueous layer was extracted with dichloromethane (3 x 40 ml). The combined organic layers were dried over sodium sulfate and the solvent was evaporated under vacuum to give 3-(5-acetoxy-6-bromohexyl)-1-methyluracil (500 mg, 100% yield). The bromoacetate thus obtained was used in the next step without further purification. 3-(5-Acetoxy-6-bromohexyl)-1-methyluracil (360 mg, 1.0 mmol) was dissolved in methanol (5 ml) and treated with a solution of 1M sodium methoxide in methanol (1 ml). After stirring for 15 minutes, the solution was poured into water (10 ml) and extracted with dichloromethane (3 x 30 ml). The combined organic layers were dried over sodium sulfate and the solvents were evaporated to give 3-(5,6-oxidohexyl)-1-methyluracil (150 mg, 67% yield) as a colorless oil.

3-(5,6-Oxidohexyl)-1-methylthymine was synthesized as follows: A mixture of sodium hydride (343 mg, 14 mmol) and 1-methylthymine (2.00 g, 14 mmol) in dimethylsulfoxide (30 ml) was stirred for 15 minutes, and then 6-bromo-1-hexene (2.30 g, 14 mmol) was added. After stirring for 69 hours, the reaction mixture was poured into water (100 ml) and extracted with dichloromethane (4 x 50 ml). The combined organic layers were washed with saturated aqueous sodium chloride solution (40 ml), dried over sodium sulfate, and then the solvent was evaporated under vacuum to give a residue which was recrystallized (dichloromethane-ethyl ether) to give 3-(5-hexenyl)-1-methylthymine (2.80g, 88% yield).

A solution of 3-(5-hexenyl)-1-methylthymine (2.00 g, 9 mmol), 4-methylmorpholine-N-oxide (1.17 mg, 10 mmol), and osmium tetroxide (0.15 ml of a 2.5% solution in *t*-butanol) in acetone (15 ml) and water (10 ml) was stirred for 20 hours. Saturated aqueous sodium hydrosulfite solution (10 ml) was added and after 15 minutes of stirring, the mixture was extracted with 20% ethanol-dichloromethane (4 x 40 ml). The combined organic layers were dried over sodium sulfate and the solvents were evaporated under vacuum to give a solid residue. The solid was recrystallized (ethanol) to give 3-(5,6-dihydroxyhexyl)-1-methylthymine (2.00 g, 89% yield).

3-(5,6-Dihydroxyhexyl)-1-methylthymine (1.65 g, 6.4 mmol) was stirred with 30% hydrogen bromide in acetic acid (3.8 ml, 19.3 mmol) for 1.5 hours. The mixture was then added to a mixture of sodium bicarbonate (6.7 g), water (40 ml), and dichloromethane (50 ml). After 15 minutes of vigorous stirring, the layers were separated and the aqueous layer was extracted with dichloromethane (2 x 50 ml). The combined organic layers were dried over sodium sulfate and the solvent was evaporated under vacuum to give 3-(5-acetoxy-6-bromohexyl)-1-methylthymine (2.30 g, 100% yield). The bromoacetate was used in the next step without further purification. 3-(5-Acetoxy-6-bromohexyl)-1-methylthymine (2.30 g, 6.4 mmol) was dissolved in methanol (10 ml) and a solution of 1 M sodium methoxide in methanol (7 ml) was added. After stirring for 15 minutes, the solution was poured into water (60 ml) and extracted with 20% ethanol-dichloromethane (2 x 70 ml). The combined organic layers were dried over sodium sulfate and the solvents were evaporated under vacuum to give 3-(5,6-oxidohexyl)-1-methylthymine (1.30 g, 85% yield) as a white solid.

3-(5,6-Oxidohexyl)-1-methylbenzoyleneurea was synthesized as follows: A solution of sodium hydride (0.76 g, 30 mmol) and benzoyleneurea (4.86 g, 30 mmol) in dimethylsulfoxide (100 ml) was stirred for 10 minutes and then methyl iodide (1.87 ml, 30 mmol) was added. After stirring for 14 hours, water (100 ml) was added and the solution was extracted with dichloromethane (3 x 100 ml). The mixture was filtered and the dichloromethane phase was dried over sodium sulfate. After evaporation of the solvent under vacuum, the residue was recrystallized (dichloromethane) to give 1-methylbenzoyleneurea (1.3 g, 25% yield) as a white solid.

A solution of sodium hydride (0.17 g, 6.8 mmol) and 1-methylbenzoyleneurea (1.07 g, 6.1 mmol) in dimethyl sulfoxide (50 ml) was stirred for 10 minutes and then 1-bromohexene (0.82 ml, 6.8 mmol) was added. After 14 hours, water (50 ml) was added and the solution was extracted with dichloromethane (3 x 30 ml). The combined organic phases were washed with water (3 x 50 ml), dried over sodium sulfate, and the solvent was evaporated under vacuum to give 3-(5-hexenyl)-1-methylbenzoyleneurea (1.51 g, 96 %) as a white solid.

A solution of 3-(5-hexenyl)-1-methylbenzoyleneurea (1.5 g, 5.8 mmol), 4-methylmorphline-*N*-oxide (0.87 g, 7.4 mmol), and potassium osmate(IV) dihydrate (0.021 g, 0.1 mmol) in acetone (12.5 ml) and water (4 ml) was stirred. After 18 hours, a 20% aqueous solution hydrosulfite (20 ml) was added and stirred for 30 minutes. The solution was extracted with dichloromethane (3 x 75 ml). The combined organic phases were dried over sodium sulfate and the solvent was evaporated under vacuum. The residue was purified by flash chromatography (silica, 5% methanol-dichloromethane) to give 3-(5,6-dihydroxyhexyl)-1-methylbenzoyleneurea (1.59 g, 94%) as a white solid.

A mixture of 3-(5,6-dihydroxyhexyl)-1-methylbenzoyleneurea (0.92 g, 3.1 mmol) in 30% hydrogen bromide in acetic acid (0.63 ml, 9.3 mmol) was stirred for 90 minutes. The reaction mixture was poured into a mixture of sodium bicarbonate (0.78 g, 9.3 mmol), water (20 ml), and dichloromethane (20 ml). The phases were separated and the aqueous phase was extracted with dichloromethane (2 x 20 ml). The combined organic phases were washed with brine (20 ml), dried over sodium sulfate, and the solvent was evaporated under vacuum to give 3-(5-acetoxy-6-bromohexyl)-1-methylbenzoyleneurea (1.2 g, 96%).

To a 1 M solution of sodium methoxide in methanol (3.1 ml) was added 1-(5-acetoxy-6-bromohexyl)-3-methylbenzoyleneurea (1.17 g, 2.9 mmol) in methanol (25 ml) over 5 minutes. After stirring for 1 hour, water (50 ml) was added. The solution was extracted with dichloromethane (3 x 25 ml). The combined organic phases were dried over sodium sulfate and the solvents were evaporated under vacuum to give 3-(5,6-oxidohexyl)-1-methylbenzoyleneurea (0.77 g, 97%) as a white solid.

1-(5,6-Oxidohexyl)glutarimide was synthesized as follows: A mixture of glutarimide (2.00 g, 7.7 mmol) and sodium hydride (425 mg, 17.7 mmol) in dimethyl sulfoxide (40 ml) was stirred for 20 minutes and then 6-bromo-1-hexene (2.90 g, 17.7 mmol) was added. After 20 hours of stirring, the reaction was poured into water (100 ml) and extracted with dichloromethane (4 x 50 ml). The combined organic layers were washed with water (50 ml) and then with saturated aqueous sodium chloride solution (50 ml). After drying over sodium sulfate the solvent was evaporated under vacuum to give 1-(5-hexenyl)glutarimide (2.92 g, 85% yield).

To a solution of 1-(5-hexenyl)glutarimide (630 mg, 3.2 mmol) in dichloromethane (10 ml) was added sodium bicarbonate (2.20 g, 26 mmol) in water (10 ml) by 50% *m*-chloroperoxybenzoic acid (2.5 g, 7.2 mmol). After stirring for 17 hours, sodium metabisulfite (1.7 g, 9.0 mmol) was added and stirred for 30 minutes. The mixture was extracted with dichloromethane (3 x 10 ml) and then the combined organic layers were washed with saturated aqueous sodium bicarbonate solution (10 ml). After drying over sodium sulfate and evaporation of the solvent under vacuum, the residue was purified by column chromatography (silica, 10% ethanol-dichloromethane) to give 1-(5,6-oxidohexyl)glutarimide (180 mg, 27% yield).

### Example 2

This example illustrates a method for synthesis of 1-(8-hydroxy-9-(N-benzyl)aminononyl)-3,7-dimethylxanthine (compound no. 27). A mixture of 1-(8,9-oxidohexyl)-3,7-dimethylxanthine (500 mg, 1.6 mmol) from Example 1 and benzylamine (2.0 g, 19 mmol) was heated at 150 °C for 4 hours. After cooling to ambient temperature, ether (30 ml) was added. The precipitate was washed with cold ether to give (compound no. 27) (278 mg, 41% yield).

### Example 3

This example illustrates the synthesis of 1-(5-hydroxy-6-(N-octyl)aminohexyl)-3,7-dimethylxanthine (compound no. 28). A mixture of 1-(5,6-oxidohexyl)-3,7-dimethylxanthine (400 mg, 1.4 mmol) synthesized in example 1, and 1-octylamine (391 mg, 3 mmol) was heated at 135 °C for 4 hours. After cooling to ambient temperature, ether (15 ml) was added. The precipitate was washed several times with hexane to give compound no. 28 (537 mg, 94% yield).

### Example 4

This example illustrates the synthesis of 1-(5-hydroxy-6-(N-(4-phenyl)butyl)amino)hexyl)-3,7-dimethylxanthine (compound no. 29). A mixture of 1-(5,6-oxidohexyl)-3,7-dimethylxanthine (300 mg, 1.1 mmol) from example 1 and 4-phenyl-1-butylamine (322 mg, 2.2 mmol) was heated at 130 °C for 70 minutes. After cooling to ambient temperature, the residue was dissolved in dichloromethane (2 ml) and added to ether (20 ml). The precipitate was washed several times with hexane to give compound no. 29 (280 mg, 60% yield).

### Example 5

This example illustrates the synthesis of 1-(5-hydroxy-6-(N-undecyl)aminohexyl)-3,7-dimethylxanthine (compound no. 30). A mixture of 1-(5,6-oxidohexyl)-3,7-dimethylxanthine (300 mg, 1.1 mmol) from example 1 and 1-undecylamine (754 mg, 4.4 mmol) was heated at 100 °C for 4 hours and then at 130 °C for 1 hour. After cooling to ambient temperature, ether (10 ml) was added. The waxy precipitate was washed several times with hexane to give compound no. 30 (403 mg, 82% yield).

### Example 6

This example illustrates the synthesis of 1-(5-hydroxy-6-(N-cyclohexylmethyl)aminohexyl)-3,7-dimethylxanthine (compound no. 31). A mixture of 1-(5,6-oxidohexyl)-3,7-dimethylxanthine (300 mg, 1.1 mmol) from example 1 and cyclohexanemethylamine (249 mg, 2.2 mmol) was heated at 100 °C for 5 hours and then at 120 °C for 1 hour. After cooling to ambient temperature, ether (7 ml) and hexane (10 ml) were added. The precipitate was washed several times with hexane to give compound no. 31 (294 mg, 68% yield).

### Example 7

This example illustrates the synthesis of 1-(5-hydroxy-6-(N-(6-hydroxy)hexyl)aminohexyl)-3,7-dimethylxanthine (compound no. 32). A mixture of 1-(5,6-oxidohexyl)-3,7-dimethylxanthine (300 mg, 1.1 mmol) from example 1 and 6-amino-1-hexanol (754 mg, 2.6 mmol) was heated at 120 °C for 2 hours. After cooling to ambient temperature, ether (20 ml) was added. The precipitate was washed several times with hexane to give compound no. 32 (321 mg, 74% yield).

### Example 8

This example illustrates the synthesis of 1-(5-hydroxy-6-(N,N-dihexyl)aminohexyl)-3,7-dimethylxanthine (compound no. 33). A mixture of 1-(5,6-oxidohexyl)3,7-dimethylxanthine (300 mg, 1.1 mmol) from example 1 and dihexylamine (556 mg, 3.0 mmol) was heated at 135 °C for 5 hours and then at 170 °C for 2 hours. After cooling to ambient temperature, petroleum ether (20 ml) was added. After cooling in a freezer, the precipitate was washed several times with petroleum ether to give compound no. 33 (263 mg, 52% yield).

### Example 9

This example illustrates the synthesis of 1-(5-hydroxy-6-(N-(4-methoxy)benzyl)aminohexyl)-3,7-dimethylxanthine (compound no. 34). A mixture of 1-(5,6-oxidohexyl)-3,7-dimethylxanthine (300 mg, 1.1 mmol) from example 1 and 4-methoxybenzylamine (0.7 g, 5 mmol) was heated at 100 °C for 4 hours. After cooling to ambient temperature, ether (10 ml) was added. The precipitate was washed several times with petroleum ether to give compound no. 34 (355 mg, 78% yield).

### Example 10

This example illustrates the synthesis of 3-(5-hydroxy-6-(N-propyl)aminohexyl)-1-methyuracil (compound no. 19). A mixture of 3-(5,6-oxidohexyl)-1-methyluracil (100 mg, 0.4 mmol) from example 1 and *n*-propylamine (10 ml) was heated in a sealed pressure bottle at 80-90 °C for 69 hours. Evaporation of the unreacted *n*-propylamine gave a yellow oil which was crystallized (ether-dichloromethane) to give compound no. 19 (80 mg, 71%) as a white solid.

### Example 11

This example illustrates the synthesis of 3-(5-hydroxy-6-(N-benzyl)aminohexyl)-1-methylbenzoyleneurea (compound no. 3). A mixture of 3-(5,6-oxidohexyl)-1-methylbenzoyleneurea (0.1 g, 0.4 mmol) from example 1 and benzylamine (0.13 g, 1.2 mmol) was stirred under argon at 115 °C. After 3 hours, the unreacted benzylamine was evaporated under vacuum. The residue crystallized on standing to give (compound no. 3) (0.14 g, 93% yield) as a white solid.

### Example 12

This example illustrates the synthesis of 1-(5-hydroxy-6-(N-propyl)aminohexyl)-3,7-dimethylxanthine (compound no. 14). A solution of 1-(5,6-oxohexyl)-3,7-dimethylxanthine (238 mg, 0.86 mmol) from example 1 in *n*-propylamine (5 ml) was heated at 100 °C in a sealed pressure bottle for 23 hours. After cooling to 4 °C, the bottle was unsealed and unreacted *n*-propylamine was evaporated under vacuum to give (compound no. 14) (190 mg, 64% yield) as a viscous oil.

### Example 13

This example illustrates the synthesis of 1-(5-hydroxy-6-(N-benzyl)aminohexyl)-3,7-dimethylxanthine (compound no. 13). A mixture of (5,6-oxidohexyl)-3,7-dimethylxanthine (500 mg, 1.8 mmol) from example 1 and benzylamine (1.7 g, 15.8 mmol) was heated at 150 °C for 4 hours. After cooling to ambient temperature, ether was added (20 ml). The precipitate was washed with cold ether to give compound no. 13 (470 mg, 70% yield).

### Example 14

This example illustrates the synthesis of N²-(5-hydroxy-6-(N³-propyl)aminohexyl)-(N¹-propyl)flutaric acid (compound no. 5). A solution of 1-(5,6-oxidohexyl)glutarimide (60 mg, 0.3 mmol) from example 1 in *n*-propylamine (5 ml) was heated in a sealed pressure bottle at 80-90 °C for 30 hours. Unreacted *n*-propylamine was evaporated under vacuum to give a residue which was triturated with ether to give compound no. 5 (100 mg, 100% yield).

### Example 15

This example illustrates the synthesis of 3-(5-hydroxy-6-(N-undecyl)aminohexyl)-1-methylthymine (compound no. 24). A mixture of 3-(5,6-oxidohexyl)-1-methylthymine from example 1 (250 mg, 1.1 mmol) and 1-undecylamine (0.7 ml) were heated at 110 °C for 4 hours. After cooling to ambient temperature, ether (5 ml) and petroleum ether (10 ml) were added. After cooling to -10 °C for 2 hours, the precipitate was washed several times with petroleum ether to give compound no. 24 (361 mg, 80% yield).

### Example 16

This example illustrates the synthesis of 3-(6-propylamino-5-hydroxyhexyl)-1-methylthymine (compound no. 26). A solution of 3-(5,6-oxidohexyl)-1-methylthymine (200 mg, 0.8 mmol) from example 1 in *n*-propylamine (10 ml) was heated in a sealed pressure bottle at 100-105 °C for 24 hours. After evaporation of unreacted *n*-propylamine, the residue was crystallized (ether) to give compound no. 26 (162 mg, 68% yield).

### Example 17

This example illustrates the synthesis of 1-(8-hydroxy-9-(N-octyl)aminononyl)-3,7-dimethyl-xanthine (compound no. 35). A mixture of 1-(8,9-oxidohexyl)-3,7-dimethylxanthine (300 mg, 0.9 mmol) from example 1 and octylamine (1 ml) were heated at 110 °C for 3 hours. After cooling to room temperature, ether (10 ml) was added. The precipitate was washed several times with petroleum ether to give compound no. 35 (342 mg, 85% yield).

### Example 18

This example illustrates the synthesis of 1-(5-hydroxy-6-(N-tetradecyl)aminohexyl)-3,7-dimethylxanthine (compound no. 36). A mixture of 1-(5,6-oxohexyl)-3,7-dimethylxanthine (300 mg, 1.1 mmol) from example 1 and 1-tetradecylamine (604 mg, 2.8 mmol) was heated at 110 °C for 3 hours. After cooling to ambient temperature, ether (6 ml) was added. The precipitate was washed several times with petroleum ether to give compound no. 36 (356 mg, 66% yield).

### Example 19

This example illustrates a method of synthesis for 1-(9-Tetradecylamino-8-hydroxynonyl)-3,7-dimethylxanthine (compound no. 73). A mixture of 1-(8,9-oxidononyl)-3,7-dimethylxanthine (synthesized in example 1 above, 1.00 g, 3.1 mmol) and anhydrous lithium perchlorate (329 mg, 3.1 mmol) was stirred in anhydrous acetonitrile (30 ml). After addition of 1-tetradecylamine (Aldrich, 722 mg, 3.4 mmol), the mixture was stirred at 60 °C for 4 hours. After cooling, water (50 ml) was added and the mixture was extracted with dichloromethane (3 x 50 ml). The combined organic layers were washed with water (30 ml) and saturated aqueous salt solution (30 ml) and subsequently dried over sodium sulfate. The solvent was removed under vacuum to give a white residue. Chromatography (neutral activity II alumina, dichloromethan/5%methanol) of the white residue resulted in 860 mg of compound no. 73 (52% yield).

### Example 20

This example illustrates a method of synthesis for compound no. 63. Sodium hydride (95%) (1.26 g, 50 mmol) was added to a solution of theobromine (7.2g, 40 mmol) in dimethylsulfoxide (300 ml). After 20 minutes of stirring, undecenylmesylate (7.95 g, 30 mmol) was added and stirred for 12 hours at room temperature. The reaction, warmed to 70-80 °C, was stirred for 4 hours. The reaction mixture was poured into a separatory funnel containing 1 L of water and extracted with dichloromethane (5 x 200 ml). The organic extracts were combined, washed with water (100 ml) and brine (100 ml), dried over anhydrous magnesium sulfate and concentrated under reduced pressure. A crude product obtained was further purified by flash chromatography over silica gel using an eluant of 20% hexane/dichloromethane to obtain 4.6 g of 1-10-undecenyl)-3,7-dimethylxanthine (yield, 46.3%).

A solution of 1-(10-undecenyl)-3,7-dimethylxanthine, prepared above (4.3 g, 13 mmol), 4-methylmorpholine-N-oxide (1.942 g, 16.6 mmol) and potassium osmate dihydrate (9.5 mg; 0.026 mmol) in acetone (45 ml) and water (10 ml) was stirred for 6 hours. A solution of 20% aqueous sodium sulphite (12 ml) was added and stirred for 30 minutes. The reaction mixture was extracted with 25% ethanol/dichloromethane (4 x 100 ml). The combined organic extracts were dried over anhydrous magnesium sulfate, concentrated under reduced pressure and purified by flash chromatography over silica gel using a methanol/5% dichloromethane eluent to obtain 3.6 g of 1-(10,11-dihydroxyundecanyl)-3,7-dimethylxanthine (yield, 76%).

1-(10,11-dihydroxyundecanyl)-3,7-dimethylxanthine, prepared above (3.6 g, 10 mmol), was stirred with hydrogen bromide (6.2 ml, 8.4 g of a 30% solution in acetic acid, 31.1 mmol) for 90 minutes. The mixture was then added to a flask containing 100 ml aqueous sodium bicarbonate solution and 75 ml dichloromethane. After 10 minutes of vigorous stirring, the layers were separated and an aqueous portion washed with dichloromethane (3 x 75 ml). The organic portions were combined, dried over magnesium sulfate, and evaporated to give 1-(10-acetoxy-11-bromoundecanyl)-3,7-dimethylxanthine (3.6 g). Without further purification, the bromoacetate was taken up in methanol (25 ml) and treated with a solution of sodium methoxide (prepared from 0.28 g, 12.2 mmol sodium, and 25 ml methanol). After 30 minutes, most of the solvent was removed under reduced pressure and the residue was extracted with dichloromethane (3 x 75 ml). The organic portions were combined, dried over magnesium sulfate and concentrated under reduced pressure to give an off-white solid, purified by column chromatography over silica gel using dichloromethane/3% methanol eluant to obtain 2.0 g of 1-(10,11-oxidoundecanyl)-3,7-dimethylxanthine (yield, 57.5%).

A mixture of 1-(10,11-oxidoundecyl)-3,7-dimethylxanthine, prepared above (500 mg, 1.4 mmol), and lithium perchlorate (from Aldrich, 149 mg, 1.4 mmol) was stirred in anhydrous acetonitrile (from Aldrich, 20 ml) until homogeneous. Aniline ( from Aldrich, 670 mg, 7.2 mmol) was added, and the mixture stirred at ambient temperature for 16 hours, then at a reflux temperature for 3 hours. The residue was directly deposited on a silica column. Chromatography using a dichloromethane/10% methanol gradient produced 0.45 g of compound no. 63 (73% yield).

### Example 21

This example illustrates data of proliferative activity of various inventive compounds for inducing CMV promoter activity. The CMV promoter assay measures gene transcription and translation activity wherein any active compounds will have cytotoxic activity for inhibiting cellular protein synthesis in transformed (adenovirus) cells. Each compound was tested and the data is listed in Table I below. Compound no. 30 was the most cytotoxic compound tested.

**TABLE I**

| Compound | IC₅₀ (µM) |
|---|---|
| 13 | >500 |
| 28 | 50 |
| 29 | >100 |
| 30 | 15 |
| 31 | >100 |
| 32 | >100 |
| 33 | 100 |
| 5 | >500 |
| 19 | >500 |
| 26 | >500 |

### Example 22

This example shows the effects of five inventive compounds on inhibition of mast cell degranulation by a serotonin release assay. This assay provides an *in vitro* model for an allergy and asthma therapeutic product. Table II below shows the results of five inventive compounds (see above for chemical names and structures).

**TABLE II**

| Compound | % Inhibition | Concentration (µM) |
|---|---|---|
| 13 | 28% | 100 |
| 27 | 29% | 50 |
| 30 | too toxic | 50 |
| 35 | too toxic | 50 |
| 19 | inactive | 50 |

### Example 23

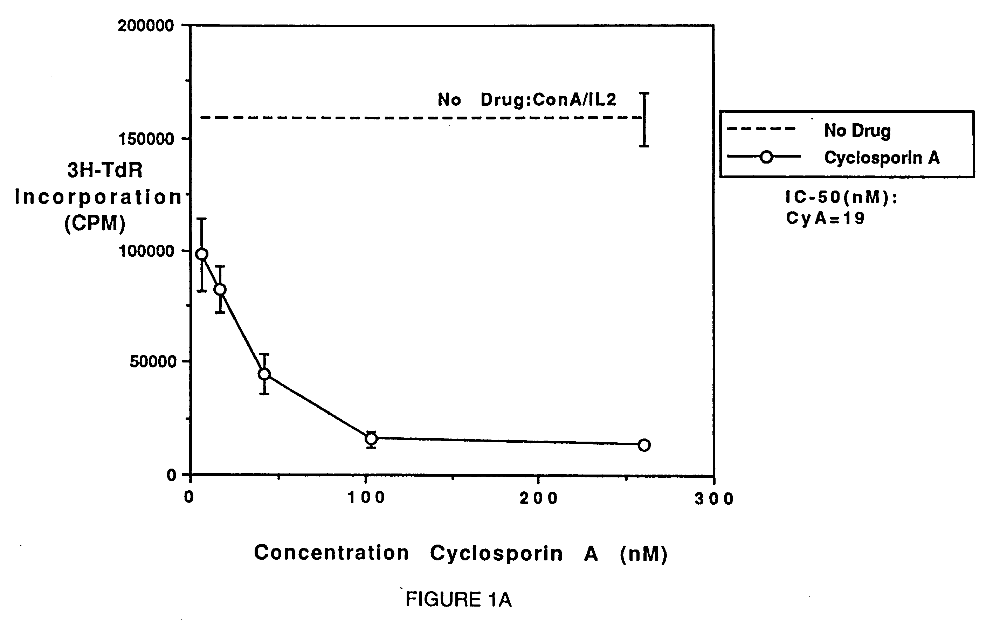

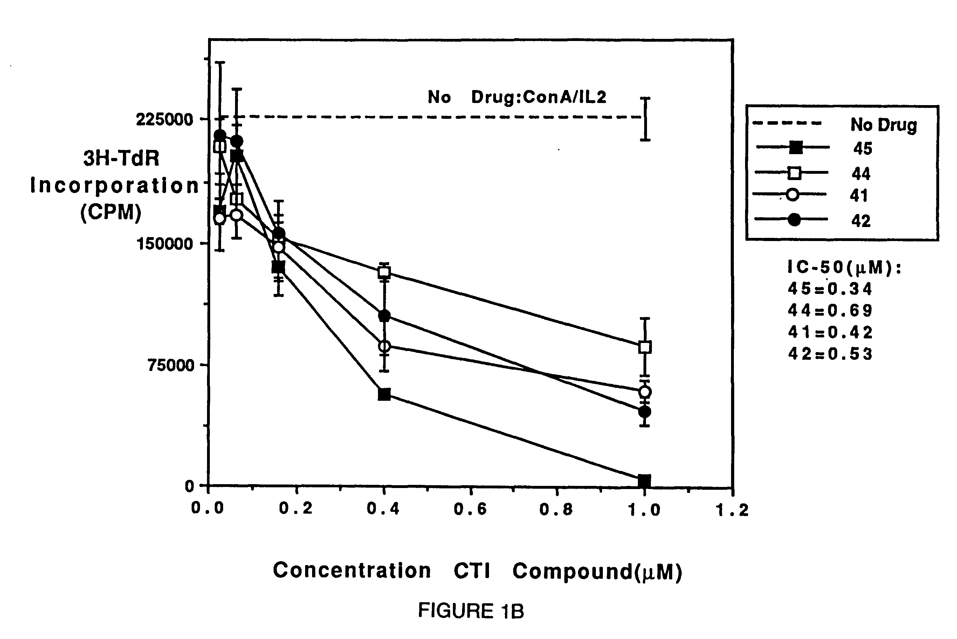

This example illustrates dose response curves used to generate 50% inhibition concentrations (IC₅₀) of for both cyclosporin A (CsA, fig. 1A) and various inventive compounds (fig. 1B) for murine thymocyte proliferation, co-stimulated by Concanavalin A (ConA) and interleukin-2 (IL-2). ConA, used to activate CD3, induces T-cell proliferation and differentiation. Thymuses, obtained from normal, female Balb/C mice, were dissociated and plated into 96-well plates at a density of 2 x 10⁵ cells/well. ConA (0.25 mg/ml) and IL-2 (15 U/ml) were added to the wells. The cells were incubated for 4 days at 37°C. On day 4, the cells were pulsed with tritiated thymidine and incubated for an additional 4 hours. The amount of tritiated thymidine dye incorporated by the harvested cells was determined in a liquid scintillation counter. Drug doses (shown in figs. 1A and 1B) were added two hours prior to ConA and IL-2 activation. Background counts were less than 200 cpm. Both CsA and the inventive compounds tested inhibit thymocyte proliferation and activation.

### Example 24

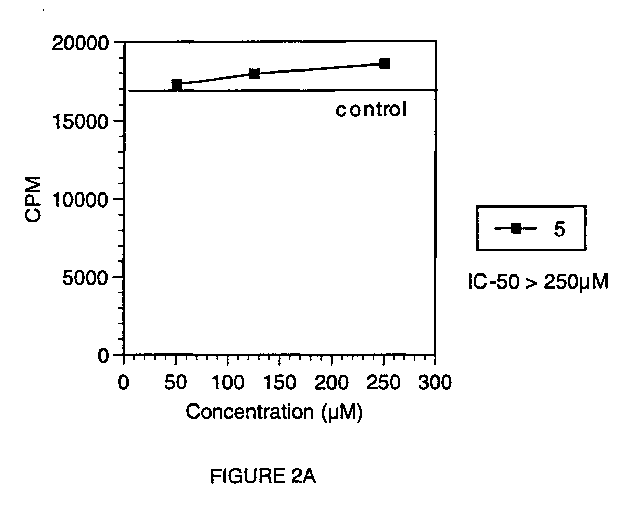

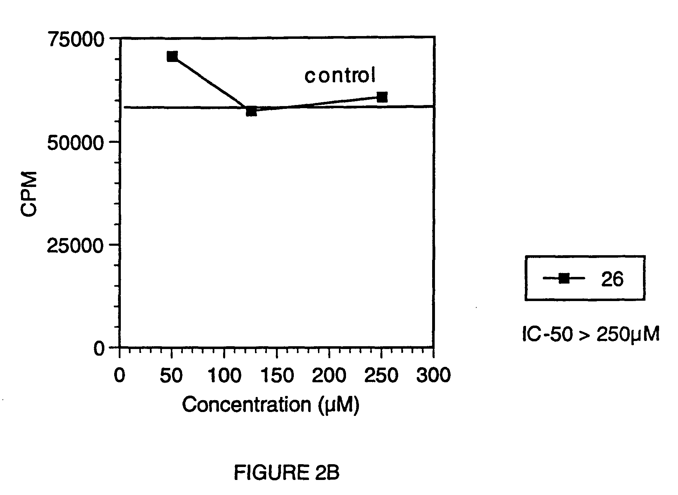

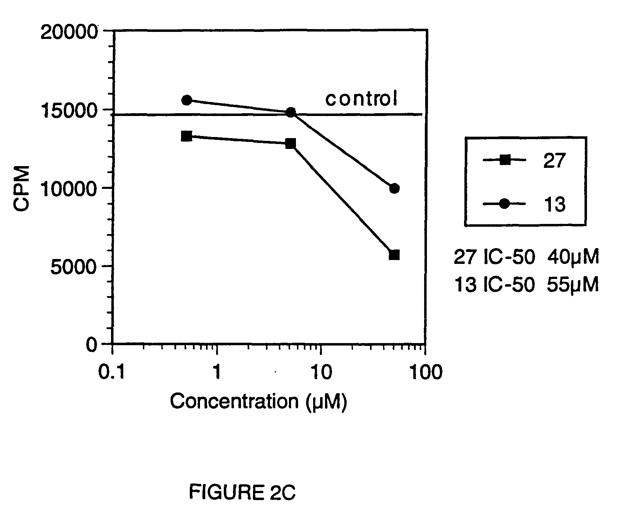

This example illustrates therapeutic potential for various autoimmune and inflammatory diseases of the inventive compounds by comparing potency (inhibitive activity) with cytotoxicity data obtained using the following indicative assay procedures. In a mixed lymphocyte reaction assay of compounds nos. 5, 13, 26 and 27, a two-way mixed lymphocyte reaction shows a proliferative PMBC response to allogeneic stimulation. Compounds nos. 5 and 26 exhibit the least assay activity in this specific, immune-modulating activity assay, having IC₅₀ values exceeding 250 µM, as shown in figs. 2A and 2B. Both compounds nos. 27 and 13 exhibit dose-response activity in this assay, having IC₅₀'s of 40 and 55 µM, respectively, illustrated in fig. 2C.

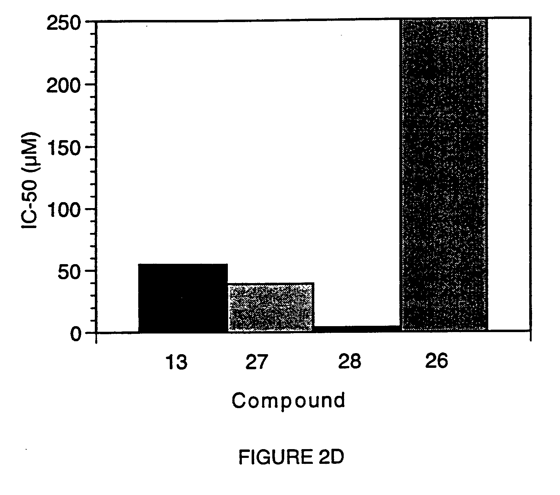

Figure 2D shows a bar graph of IC₅₀ values for five inventive compounds (see above for corresponding chemical names) in a mixed lymphocyte assay measuring immune suppression activity of the compounds. Compound no. 27 did not exhibit significant suppressive activity. Compound no. 28 proved the most potent compound of those assayed.

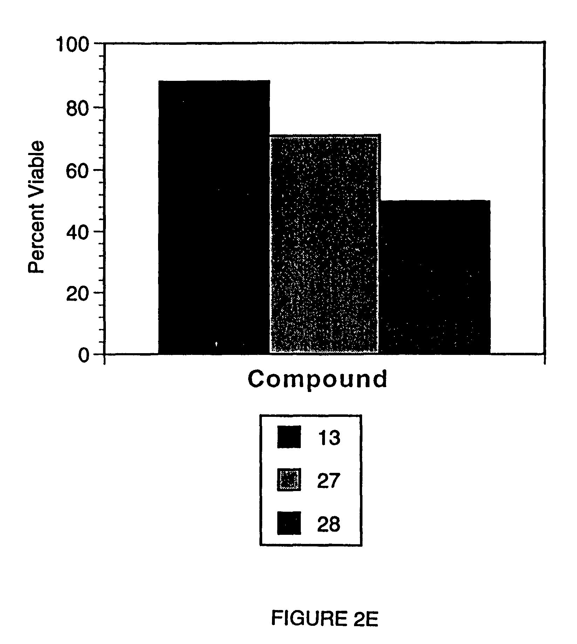

Cell percent viability in mixed lymphocyte reaction assay culture was determined after six days of cell culture. Figure 2E shows percent viability bar graph results. Control cells unexposed to drugs are generally 78 to 85% viable under these culture conditions. In this assay, all inventive compounds were present at 100 µM concentrations, generally well above corresponding IC₅₀ values in this assay (see fig. 2D). The most potent compound, compound no. 28, was also the most cytotoxic at 100 µM, but this concentration is well above its IC₅₀ value, suggesting a significant therapeutic window. Compounds nos. 13 and 27 exhibited little or no cytotoxicity at concentrations well above their respective IC₅₀ values.

Ten additional representative inventive compounds were assayed, showing impressive biological activity results, in a procedure according to that used in Example 21. IC₅₀ values for tested inventive compounds were obtained in the thymocyte ConA/IL-2, co-stimulation assay, as described above. Fifty percent (50%) lethal dose concentrations (LD₅₀) for these ten compounds were obtained using the following cytotoxicity assay.

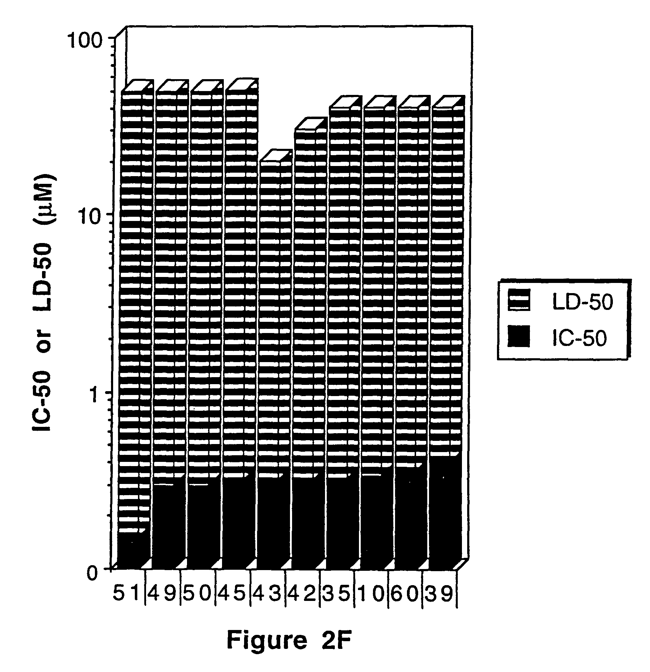

Human bone marrow-derived stromal cells (early passage) were plated at 10⁴ cells/well and allowed to reach confluency following 3 days of refeeding. The stromal cells were treated with inventive compounds for 2 hours prior to washing, and using a viability dye, BCECF, analyzing fluorescence. The highest concentration used was 50 µM (hence, an LD₅₀ value greater than 50 µM would indicate no effect at 50 µM). Figure 2F illustrates results obtained from both the thymocyte co-stimulation assay (IC₅₀ values) and the cytotoxicity assay (ID₅₀) for inventive compounds nos.: 10, 35, 39, 42, 43, 45, 49, 50, 51 and 60. As shown, most of the compounds are non-cytotoxic to stromal cells, yet are very potent proliferation inhibitors in the thymocyte IL-2, co-stimulation assay.

### Example 25

This example illustrates the effects of several inventive compounds exhibiting inhibitive effects on murine thymocyte proliferation. The data presented and discussed suggests that the inventive compounds function by mechanisms previously unknown. In a first part of this example, compounds nos. 30, 33, 28 and 27 (see above for chemical names and structures) show effective inhibition of murine thymocyte proliferation stimulated by Con A and interleukin 1 alpha (IL-1α). Compounds nos. 30, 33, 28 and 27 were added to cell cultures two hours prior to activation with Con A and IL-1α in a thymocyte co-stimulation procedure akin to that discussed in example 21. All compounds tested in the assay exhibited dose-response inhibitive properties and dose-response curves for each compoud were obtained. IC₅₀ values determined for each inventive compound tested are as follow: compound no. 30 has an IC₅₀ of 0.94 µM, compound no. 33 has an IC₅₀ of 8.6 µM, compound no. 28 has an IC₅₀ of 4.6 µM, and compound no. 27 has an IC₅₀ of less than 12.5 µM. Background counts in the assay were less than 200 cpm.

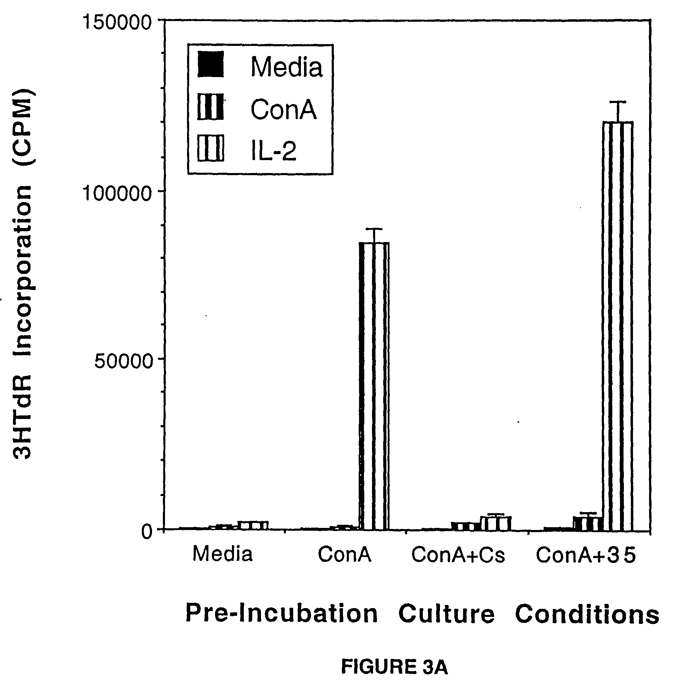

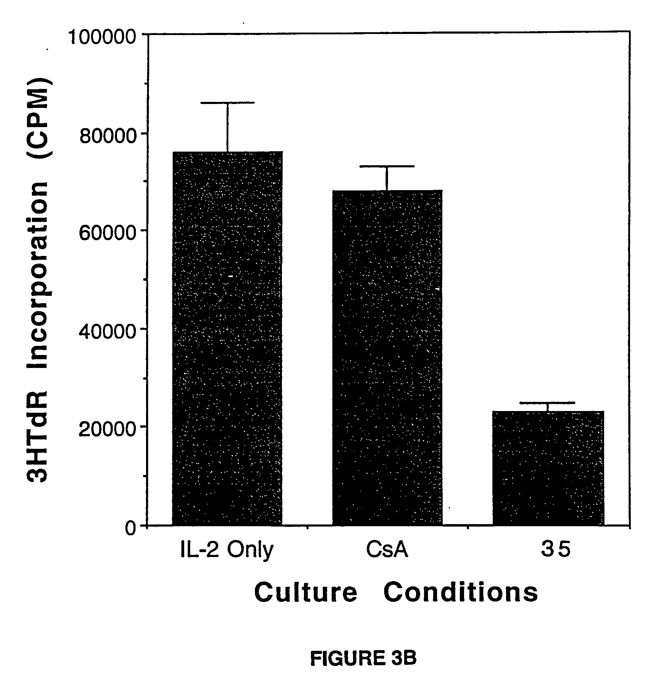

In supplemental investigations, results obtained illustrate that the inventive compounds utilize different immunosupressive mechanisms than known mechanisms of two widely-studied immunosuppressants, CsA and or FK506. In a proliferation assay, mouse thymocytes were pre-incubated overnight with Con A (a "priming step"), washed, and restimulated with IL-2 in the absence of inventive compounds. On day 4, the cells were pulsed with tritiated thymidine and allowed to incubate for an additional 4 hours. The cells were harvested and the amount of tritiated thymidine incorporated by the harvested cells was determined in a liquid scintillation counter. Figure 3A reports results obtained. In control cells, pre-incubation with Con A "primes" thymocytes by stimulating the CD3 receptor in a manner similar to antigen recognition. Research has shown that CD3 antibody can be substituted for Con A. When IL-2 was subsequently added, the thymoctye cells proliferated. CsA added during Con A incubation, "priming," inhibited thymocyte proliferation in response to IL-2 stimulation. However, when thymocytes were pre-incubated with ConA and inventive compound no. 35, "priming" occurred, shown by subsequently-observed, normal thymocyte proliferation in response to IL-2 stimulation, as shown in fig. 3. The inventive compounds do not inhibit proliferation by interfering with this "priming step," necessary for subsequent proliferation in response to IL-2 stimulation.

Additionally, the cells were pre-incubated with Con A overnight, washed and stimulated with IL-2 (with or without addition of CsA or inventive compound). On day 4, the cells were pulsed with tritiated thymidine and allowed to incubate for an additional 4 hours. The cells were harvested and the amount of incorporated tritiated thymidine was determined in a liquid scintillation counter. Cells pre-treated with Con A proliferated in response to IL-2 addition. Figure 3B shows results obtained in these assays. CsA (50µM) exhibited very little inhibition of thymocyte proliferation (indicated by the amount of incorporated dye recorded). In sharp contrast however, inventive compound no. 35 (at far less concentration, 1µM) dramatically inhibited thymocyte proliferation.

These experiments conclusively indicate that CsA and FK506 (inhibiting CD3 in like manner) have a different action mechanism, as compared with the inventive compounds. CsA inhibits ConA "priming." The inventive compounds do not. The inventive compounds inhibit IL-2 stimulation, CsA does not. The results shown indicate that the inventive compounds would be useful for reducing or preventing side effects from conditions requiring cellular stimulants.

### Example 26

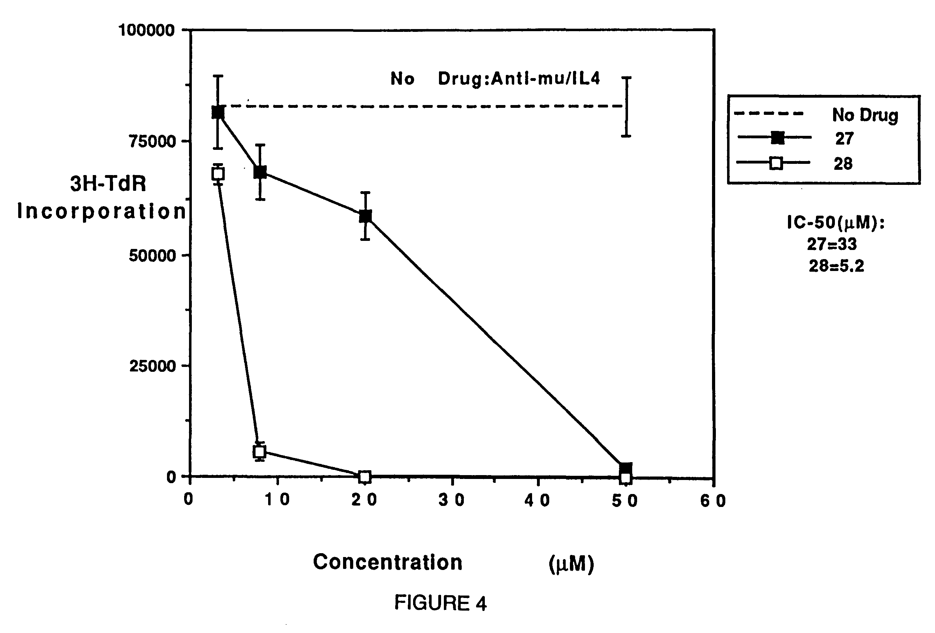

This example illustrates inhibitive effects of compounds nos. 27 and 28 on murine splenocyte proliferation stimulated by anti-mu (10 mg/ml) and interleukin-4 (IL-4, 12.5 ng/ml). This *in vitro* assay, described above, is indicative of immune-suppressive/autoimmune treatment assay emphasizing humoral or B cell immune response. The inventive compound was added to the cells at the doses indicated two hours prior to activation with anti-mu and IL-4. Both compounds nos. 27 and 28 inhibited splenocyte proliferation in a dose-response manner. IC₅₀ values for compounds nos. 27 and 28 were 3.3 µM and 5.2 µM, respectively, as shown in Figure 4. Background counts were less than 200 cpm.

### Example 27

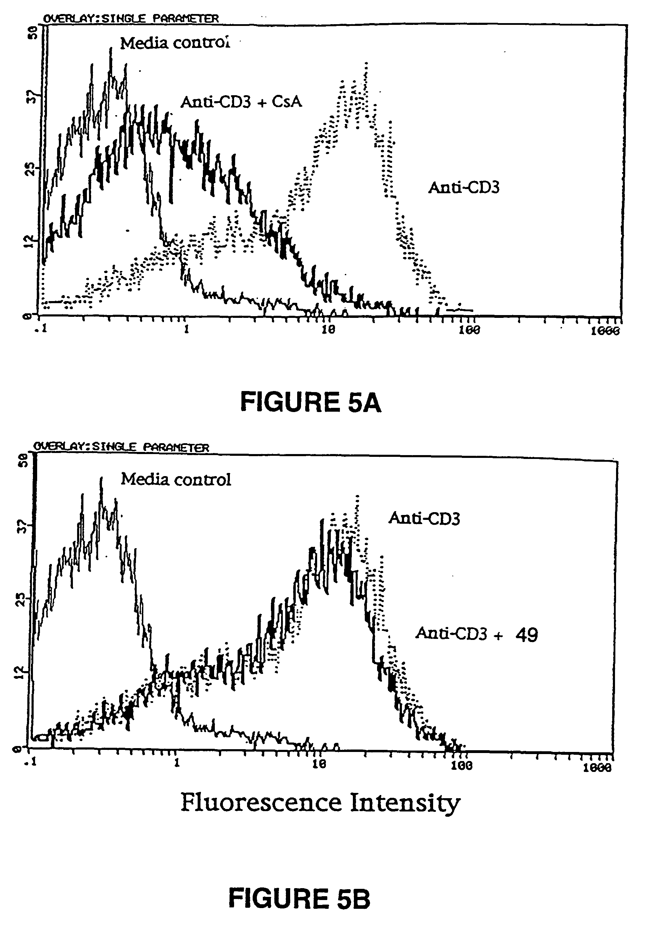

This investigation illustrates IL-2 (alpha chain CD25) receptor expression on mouse splenoyctes. The IL-2 receptor is not expressed on resting T-cells, but is rapidly-induced by certain stimulants, *e.g.* antigen recognition or treatment with ConA or antibodies to CD3 (anti-CD3). IL-2-dependent proliferation requires IL-2 receptor expression. Splenocytes were stimulated with anti-CD3 antibody (10 µg/ml) with or without the addition of CsA (20 µM) or compound no. 49 (1 µM). Following overnight incubation, the splenocytes were stained with a fluoresceinated anti-IL-2 receptor antibody and fluorescence measured by flow cytometry. Figures 5A and 5B are frequency histograms of measurements for 20,000 cells per sample. The media control has a low level of fluorescence, while stimulation with anti-CD3 stimulates large relative increases in IL-2 receptor expression (peak labeled anti-CD3 in figs. 5A and 5B). Co-incubation with CsA inhibits CD3-stimulated, IL-2 receptor expression, while incubation with inventive compound no. 49, at a concentration that blocks 90% of splenocyte IL-2-stimulated proliferation, has no effect on receptor expression. These data confirm that CsA and compound no. 49 affect immune cells by different mechanisms.

### Example 28

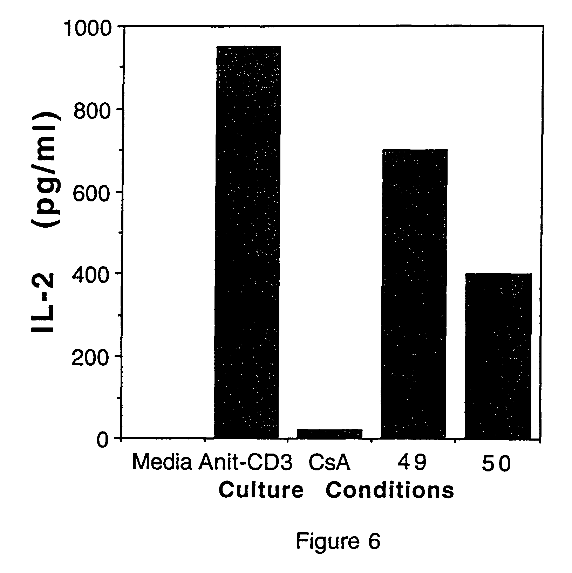

This example shows that compounds nos. 49 and 50 do not inhibit production of IL-2 in murine thymocytes, in contrast to the effects of CsA. Thymocytes were stimulated with ConA and IL-1 for 4 days with or without adding inventive compounds to the culture media. The supernatants were removed and assayed by ELISA for IL-2 levels by a commercially available kit. The results, shown in figure 6, illustrate that CsA incubation at 20nM inhibits IL-2 production and secretion. Also shown, these exemplary inventive compounds tested do not inhibit IL-2. These data illustrate that CsA and the inventive compounds tested interfere with immune cell function via different mechanisms.

### Example 29

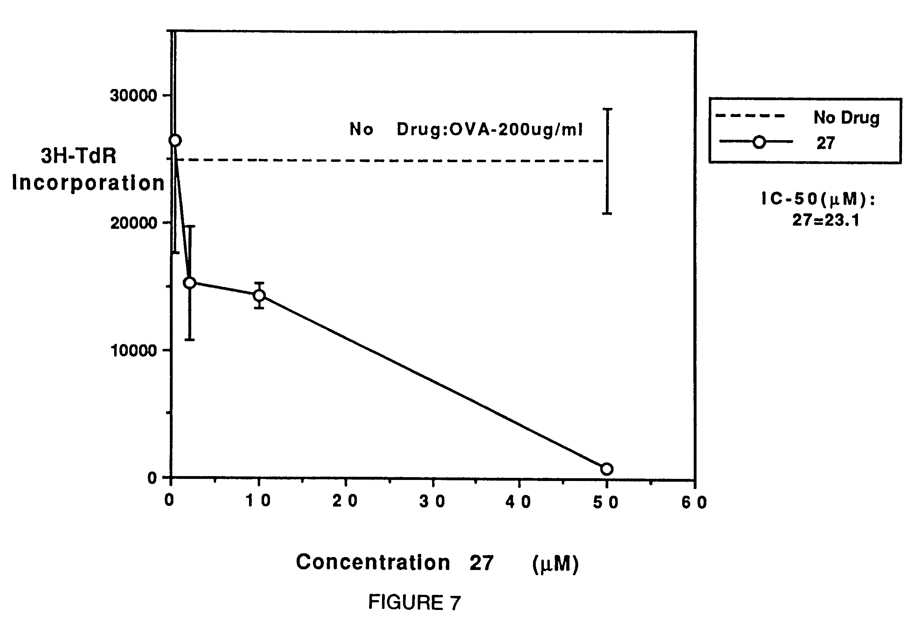

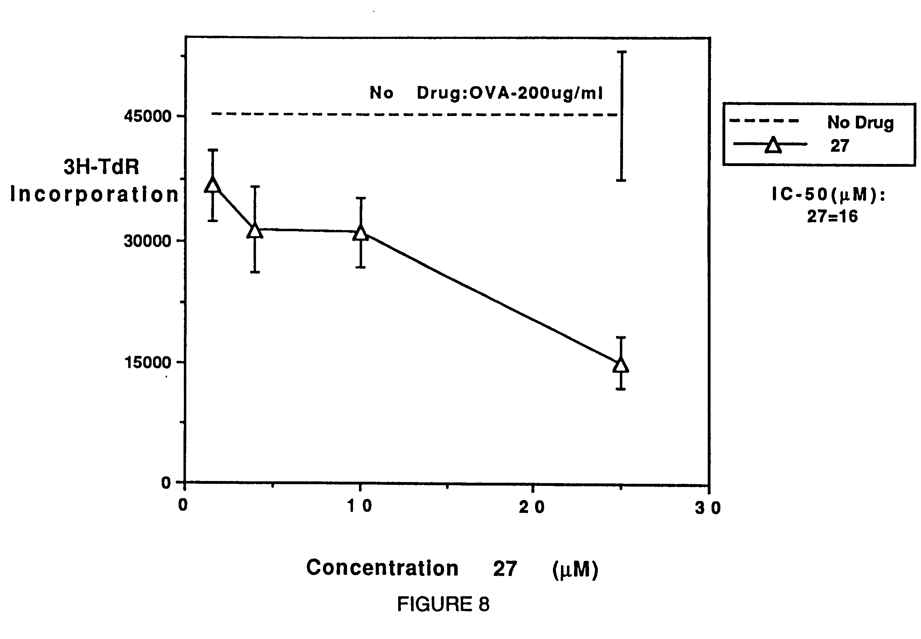

This example illustrates activity of representative inventive compounds nos. 27, 49, 50, 45, 43 or 41 (above) in assays detecting activity in response to cell stimulus. One assay, murine lymph node cell proliferation (stimulated by anitgen) was used to determine inhibitive activity of compound no. 27 on proliferation of the lymph node cells. This *in vitro* assay is an immune suppressive/autoimmune treatment predictive assay emphasizing cellular or T cell immune response. The assay used murine T cells that proliferate *in vitro* in response to a soluble protein antigen, used to prime the T cells *in vivo.* Compound was added to the cells at doses indicated in figures 7 and 8 (showing assay results) two hours prior to activation with alloantigen. Compound no. 27 inhibited T cell proliferation in a dose-response manner. IC₅₀ values for compound no. 27 were 23.1 µM for a first experiment (results shown in fig. 7) and
19 µM for a second experiment (results shown in fig. 8).

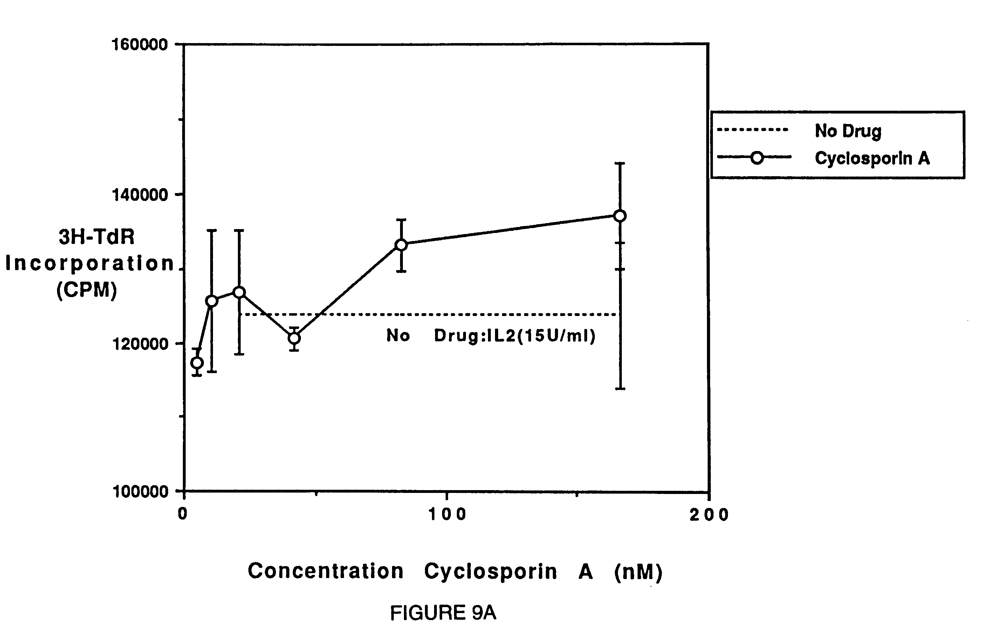

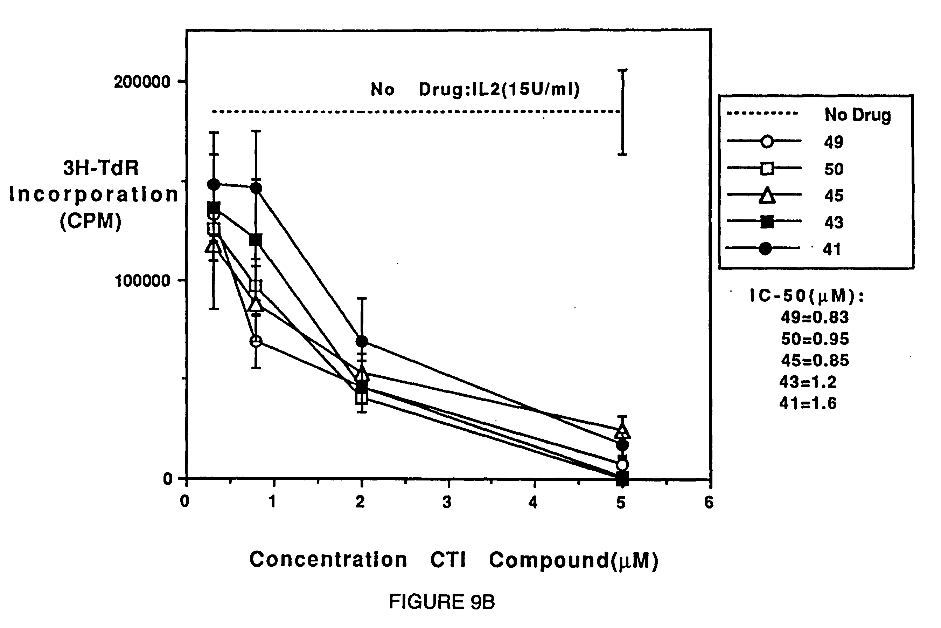

Another assay in this example was used to determine inhibitive activity of the inventive compounds on direct IL-2-induced proliferation in a murine cytotoxic T-cell line, CT6. The CT6 cell line is an IL-2-dependent cell line. IL-2 was removed from the medium for 24 hours prior to stimulation. One hour prior to IL-2 stimulation, either CsA or compounds nos. 49, 50, 45, 43 or 41 were added at various concentrations. The cells were stimulated with IL-2 and amount of tritiated thymidine dye incorporated was measured 48 hours later. Background counts were less than 5000 cpm. Assay results are graphically represented in figures 9A and 9B. The two graphs show that CsA and the inventive compounds have divergent effects on IL-2-induced proliferation. CsA did not inhibit proliferation, even at very high concentrations. However, in distinct contrast, the inventive compounds inhibited direct IL-2-induced CT6 proliferation.

### Example 30

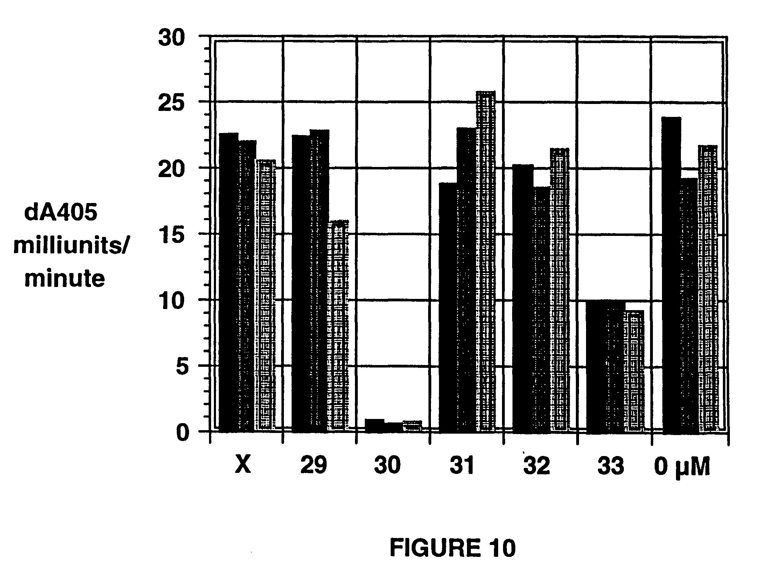

This example illustrates the effects of a reference compound "X", and inventive compounds nos. 29, 30, 31, 32 and 33 (see above for names and structures) on yeast growth (*Saccharomyces cervisiae*)--using 100 µM concentrations--as compared with yeast activity in the absence of any compound additive (control). This assay measured anti-yeast and anti-fungal activity of the the inventive compounds tested. As shown in figure 10, compounds no. 30 and 33 showed yeast-growth inhibitive activity, predicting that the inventive compounds tested are topical and systemic anti-microbial compounds.

### Example 31

This example illustrates potential anitgen specific anergy-induction of inventive compounds. Anergy is a prolonged state of T cell "unresponsiveness" due to T cell anitgen recognition (without co-stimulation) or induced proliferation blockage. This later T cell anergy may occur when a T cell's proliferation ability in response to IL-2 is blocked by some agent. Anergy is generally considered to be a type of tolerance to antigen activation. Thus, *in vitro* anergy is a means for predicting *in vivo* tolerance-enhancing agents. Tolerence is important in preventing organ rejection in tranplant procedures, as well as other autoimmune diseases such as scleroderma, rheumatoid arthritis, lupus, and diabetes-related autoimmunity.

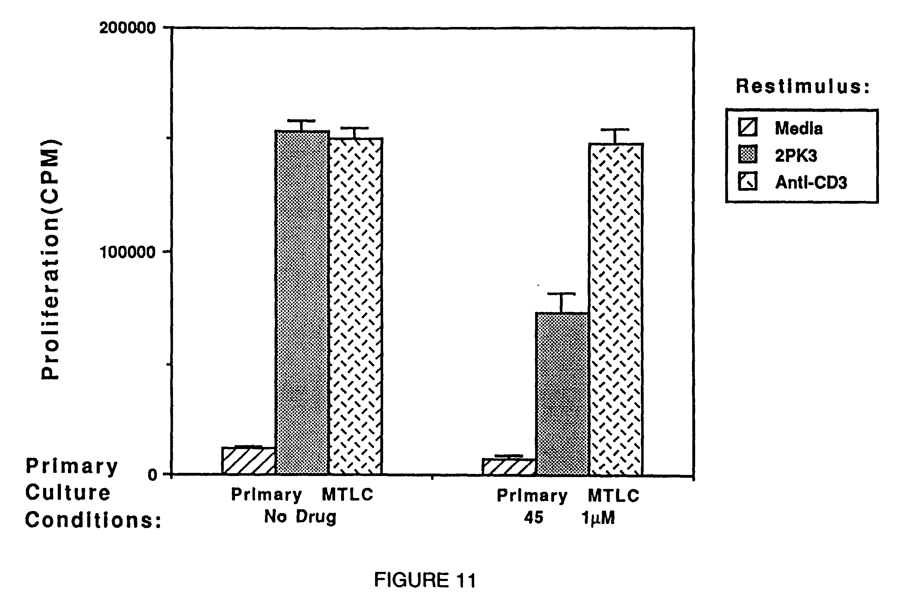

A B cell tumor, 2PK3 (H-2d) was used as a stimulating cell for C57BL/6 splenocytes (H-2b), a responding cell. Mixed cultures of B-cell tumor and splenocytes were incubated for 5 days with and without 1 µM of compound no. 45. After 5 days, the cells were washed and resuspended with either media, the original antigen (2PK3) or anti-CD3. Tritiated thymidine was added to the resulting suspensions and thymidine incorporation measured 24 hours later. Results are shown in figure 11. As illustrated, culture treated with inventive compound no. 45 and untreated cultures responded equivilantly to anti-CD3. However, cultures incubated with inventive compound no. 45 for 5 days exhibited a decreased response to primary antigen, 2PK3. Thus, C57BL/6 splenocytes were inhibited from responding to an original antigen by the inventive compound used in the pre-incubation step. However, a normal culture response to anti-CD3 stimulation predicts the inventive compounds exhibited anitgen-specific anergy induction properties.

### Example 32

This example illustrates inhibitive effects of the inventive compounds on human stromal and Balb/3T3 cell proliferation in response to PDGF stimulation.

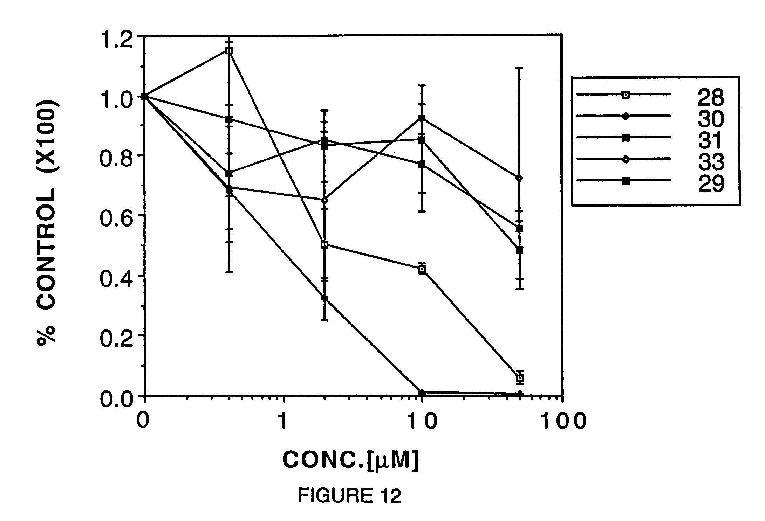

This assay is useful for predicting therapeutic activity for preventing or treating restenosis, atherosclerosis and coronary artery disease. Human stromal cells were starved in serum-free media for one day and then stimulated with 50 ng/ml PDGF-BB. Inventive compounds, at various concentrations, were added one hour prior to PDGF stimulation. PDGF and tritiated thymidine were added and the cells allowed to incubate for one day, following addition of the PDGF and thymidine. 24 Hours later, the cells were harvested and counted by liquid scintillation counting. Results for compounds nos. 28, 30, 31, 33 and 29 are shown in figure 12. Background counts (*i.e.*, starved cells) were approximately 1% of control levels. The results illustrate that the drugs were active in this predictive *in vitro* model with IC₅₀ values (in µM) of 0.9, 3.2, 40, >50 and >50 for compounds nos. 30, 28, 29, 32 and 31, respectively.

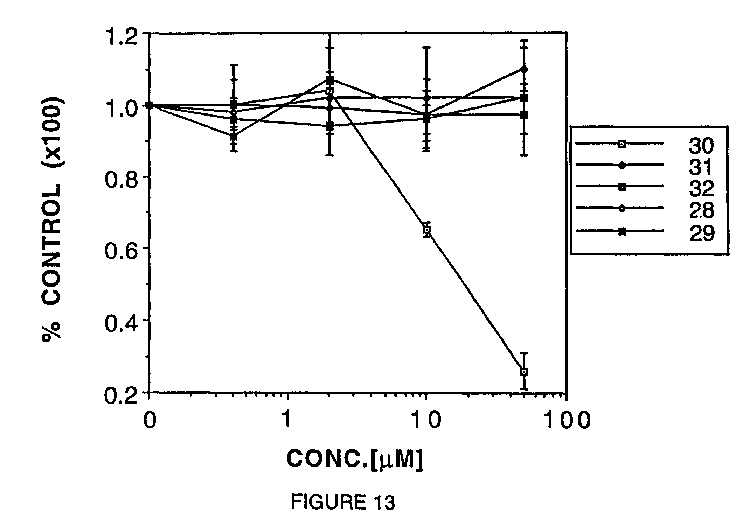

In conjunction with the human stromal cell/PDGF stimulation assay, cytotoxicity of the inventive compounds tested to stromal cells was also determined. Figure 13 shows results for this cytotoxicity assay. Only compond no. 30, exhibiting the most pronounced inhibitive activity of the compounds tested, exhibited cytotoxic effects at concentrations above 0.9 µM, its IC₅₀ value.

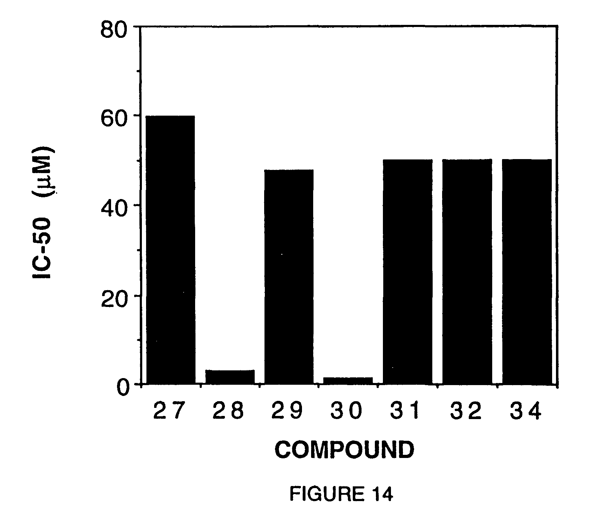

In an assay measuring inhibitive effects in a PDGF/IL-1 co-stimulation, proliferation assay, a group of inventive compounds showed inhibitive properties. The PDGF/IL-1 assay is useful in measuring *in vitro* activity, indicative of therapeutic potential for treating or preventing restenosis and reperfusion. Figure 14 reports IC₅₀ bar graph results for a group of inventive compounds nos. 27, 28, 32, 30, 31, 32 and 34. In this predictive, *in vitro* model, compounds nos. 28 and 30 exhibited potent inhibitive activity, predicting therapeutic applications for restenosis and reperfusion.

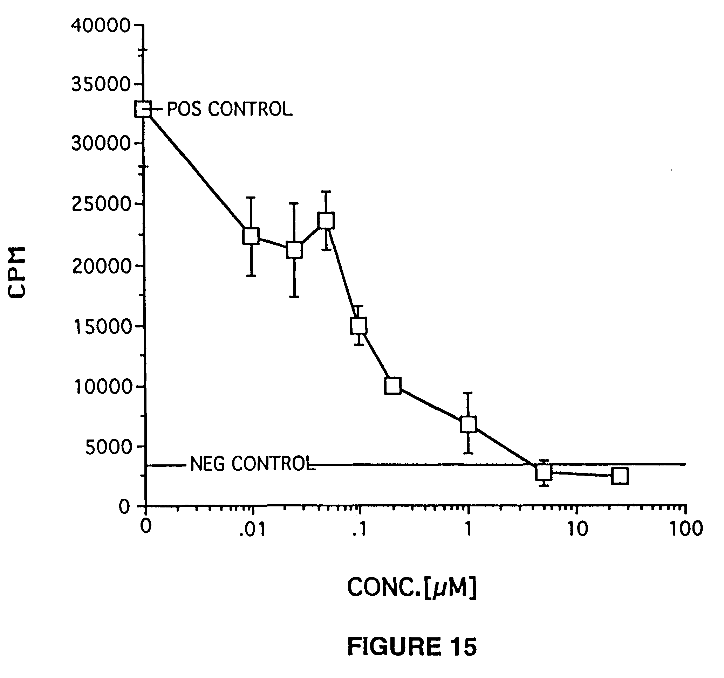

The inventive compounds possess inhibitory effects on PDGF-induced proliferation of Balb/3T3 cells. Balb/3T3 cells respond vigorously to PDGF stimulation, and are useful *in vitro* models for further study of PDGF-induced proliferation. Disregulated PDGF-proliferative response has been linked to a variety of diseases, including, *e.g.*, restenosis, atherosclerosis, fibrosis, and tumor cell angiogenesis. Cells were plated in low serum-containing medium for 24 hours prior to stimulation with various concentrations of inventive compound no. 45. PDGF-BB was added at constant 10 µM concentrations. Tritiated thymidine was added and cells harvested for scintillation counting 24 hours later. Figure 15 illustrates a dose response curve from this assay, including an IC₅₀ value of approximately 0.08 µM, exemplifying inhibitory activity of the tested compound.

### Example 33

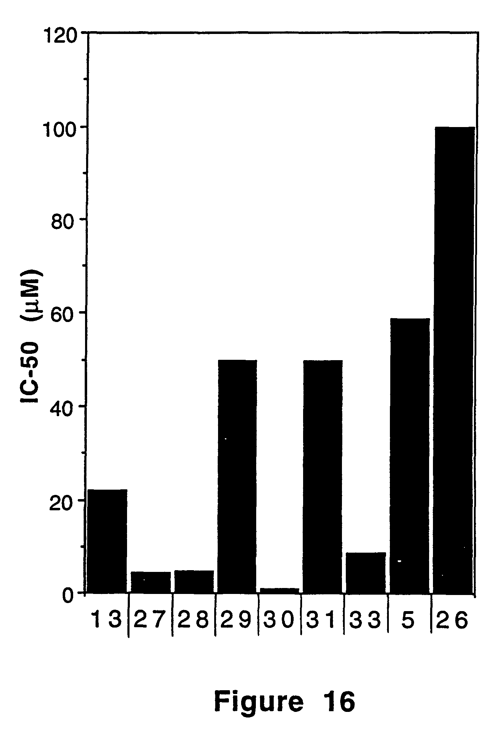

In assays desribed above and similar to those used in Example 32 and other examples, the ability of various inventive compounds to suppress murine thymocyte and Balb/3T3 cell proliferation in response to selected stimuli was investigated. In a murine thymocyte ConA/IL-2 co-stimulation assay according to Example 23, IC₅₀ values were obtained for inventive comopound nos. 13, 27, 28, 29, 30, 31 , 33,5 and 26. Figure 16 reports comparative results for the different compounds tested in this *in vitro* model. Compounds nos. 28, 30, and 27 exhibited the most potent immune-suppressive effects in these *in vitro* models.

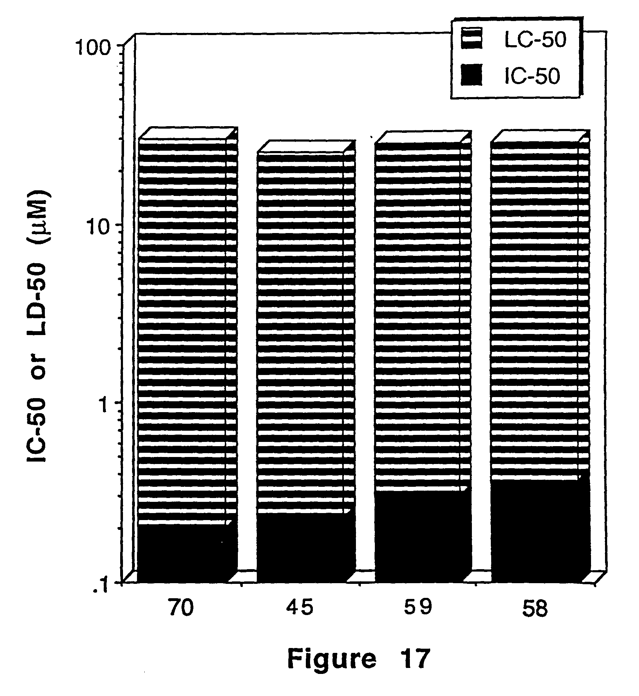

In another investigation similar to the Balb/3T3 proliferation assay of Example 32, IC₅₀ and ID₅₀ values for inventive compounds nos. 70, 45, 59 and 58 were determined. LD₅₀ values were determined using a cytotoxicity assay, as in Example 32. In these assays, Balb/3T3 cells, stimulated with PDGF and treated with one of the above inventive compounds in a manner identical to the tritiated thymidine procedure above, were incubated instead with a viable dye BCECF, a fluorescent dye. Fluorescence was measured using a fluorescent plate reader. The highest concentration used was 50 µM, therefore an LD₅₀ value greatrer than 50 µM indicates no effect at 50 µM. Figure 17 illustrates assay results by comparing LC₅₀ value against LD₅₀ values for the inventive compounds tested. Most compounds tested were non-cytotoxic yet were significant inhibitors of proliferation.

### Example 34

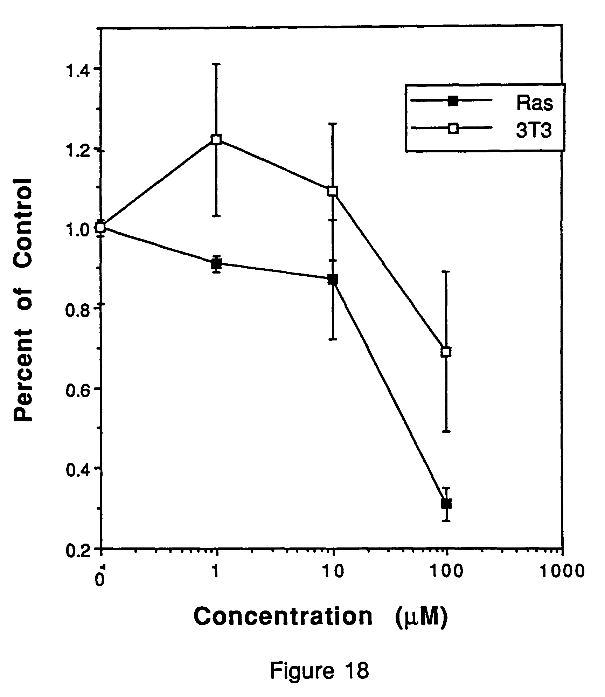

This example compares cytotoxicity results for inventive compound no. 27 for transformed cells and non-transformed cells. In transformed cells (Ras 3T3) and in normal 3T3 cells, cytotoxicity of compound no. 27 at concentrations of 1, 10 and 100 µM was determined. Figure 18 reports results obtained in this assay. At each of the above concentrations, compound no. 27 was more cytotoxic for the cancer cells than normal cells. These results indicate the compound tested has differential toxicity for tumor cells, predicting potential utility in chemotherapeutic cancer treatment.

### Example 35

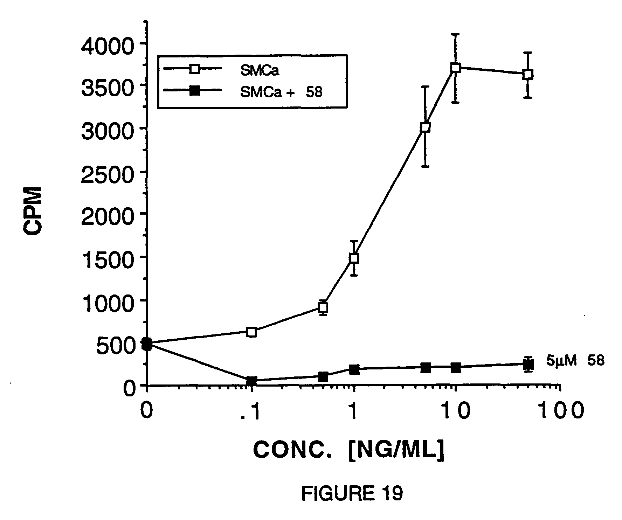

This example demonstrates an inhibitory effect on proliferation of inventive compound no. 58. One assay was used for investigating effects on PDGF-induced proliferation of human aortic smooth muscle cells (aortic SMC). Cells, purchased from a commercial supplier (Cell Systems, Inc., Seattle, WA), were cultured with various concentrations of PDGF-BB with and without addition of compound no. 58 (5 µM). As illustrated in figure 19, compound no. 58 inhibited PDGF-induced proliferation even at high PDGF concentrations providing maximum proliferative stimulation. In addition, some cultures were treated 1 hour prior to PDGF stimulation with inventive compound no. 58. As shown, PDGF stimulated proliferation in this cell line. Addition of 5 µM of compound no. 58 blocked PDGF-stimulated proliferation and no toxic effects of compound no. 58 were observed.

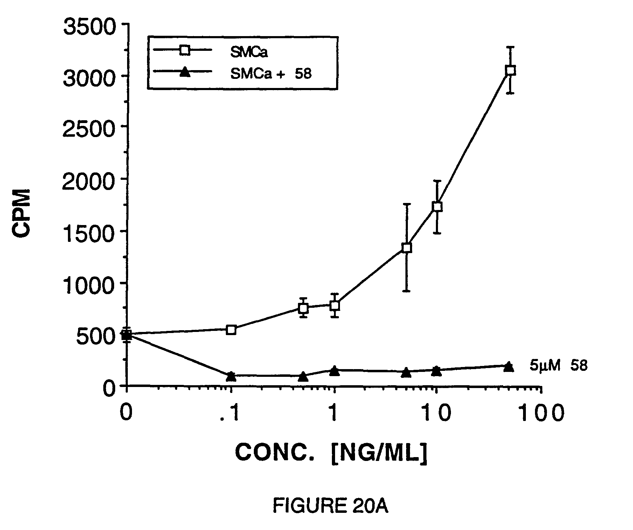

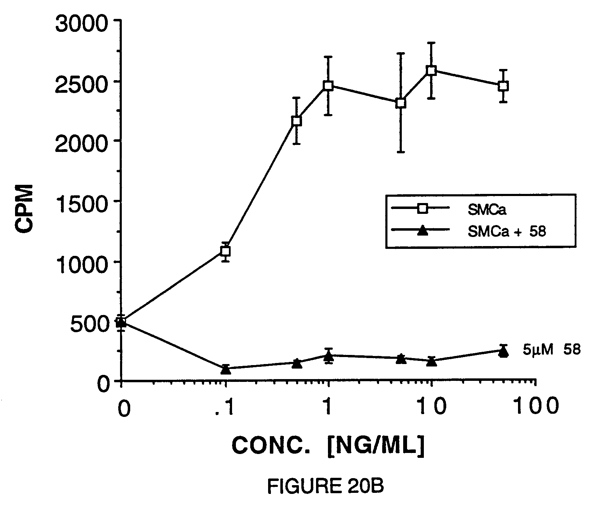

Another assay was used for investigating inhibitory effects of inventive compound no. 58 on either basic or acidic FGF-induced proliferation in human aortic smooth muscle cells (aortic SMC). Disregulated bFGF- or aFGF-induced proliferation is linked to SMC proliferation and neointimal occlusion in atherogenesis and restenosis and plays a role in autocrine and paracrine stimulation of tumor cells and tumor cell-induced angiogenesis. In this assay, cells were grown in reduced serum (0.5% fetal calf serum) for 24 hours prior to stimulating with various concentrations of PDGF. Cells were stimulated with various concentrations overnight of either aFGF or bFGF, adding 5µM of compound no. 58 in selected cultures. Compound no. 58 was a potent inhibitor of both aFGF- and bFGF-induced proliferation in this cell type, representative of other cell types examined. Results shown in figure 20A (aFGF) and figure 20B (bFGF) illustrate the degree of inhibition. No toxic effects of compound no. 58 were observed for this cell type in this assay.

### Example 36

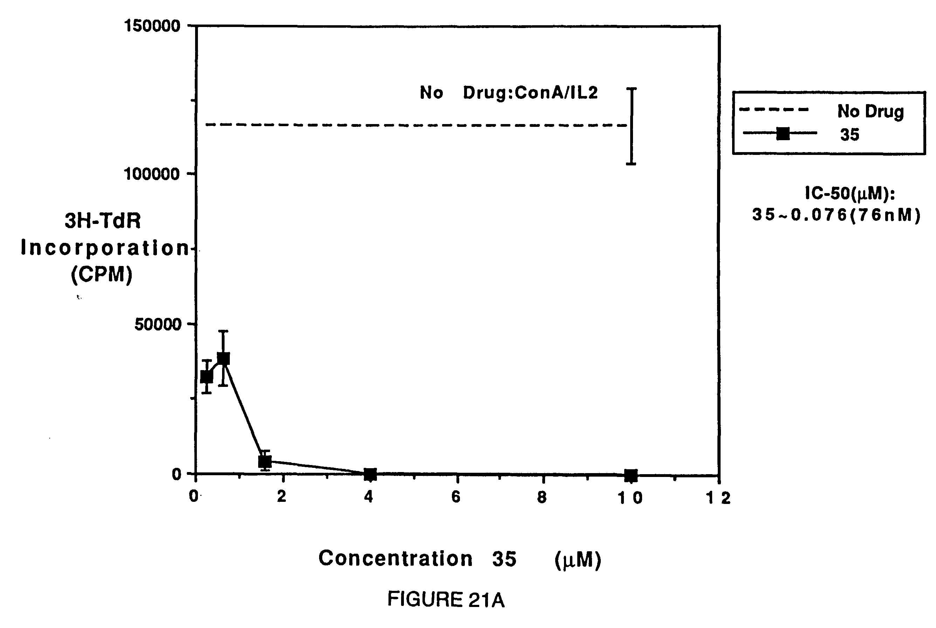

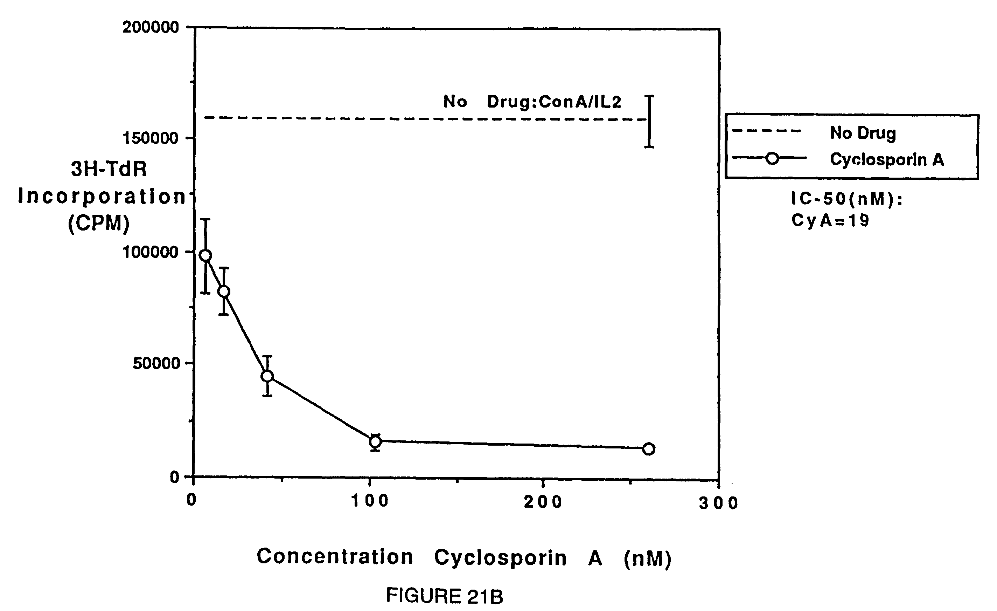

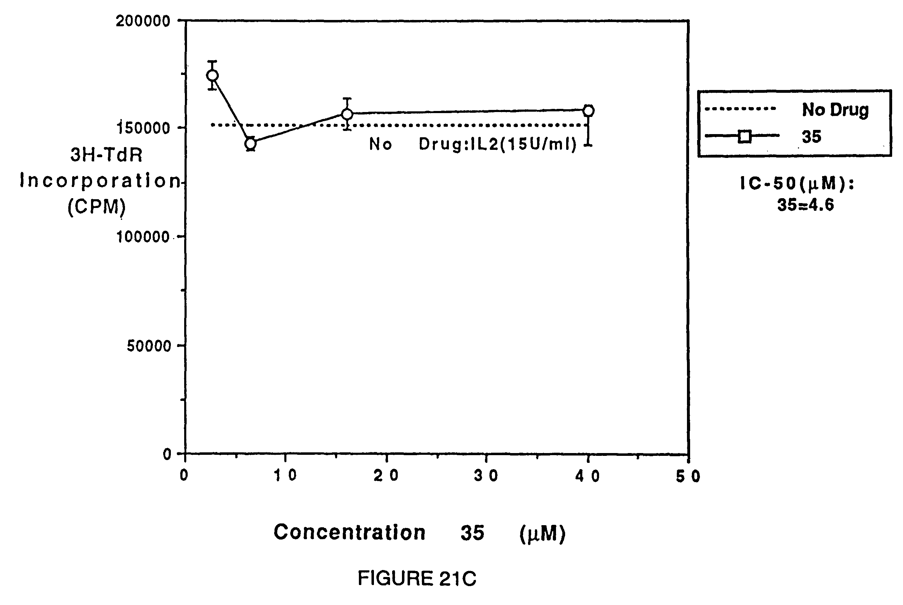

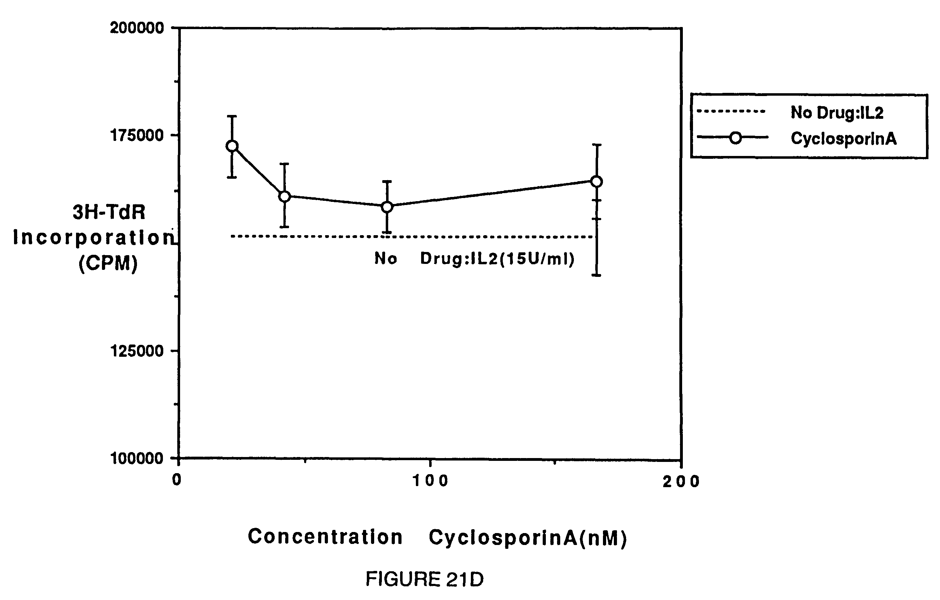

This example illustrates an investigation of proliferation of murine thymocytes co-stimulated with ConA and IL-2 using a procedure akin to the procedure in Example 23. In another related assay, inhibitory effects on CT6 cell proliferation is examined. CT6 cells are a murine IL-2 dependent, cytotoxic T cell line that proliferate in response to murine IL-2 (15 U/ml). Figures 21A, 21B, 21C and 21D illustrate experimental results of both compound no. 35 and CsA in each assay. Figure 21A illustrates the extent of inhibitory activity of compound no. 35 on murine thymocyte co-stimulation and figure 21B shows comparative inhibitory activity of CsA. Both inventive compound no. 35 and CsA exhibit significant inhibition of thymocyte proliferation with IC₅₀ values in the low micromolar and nanomolar ranges, respectively. Figures 21C and 21D illustrate inhibitory effects of compound no. 35 and CsA, respectively. Both compound no. 35 and CsA exhibited no activity in this assay, indicating neither inhibits IL-2-induced proliferation of cytotoxic CT6 cells.

### Example 37

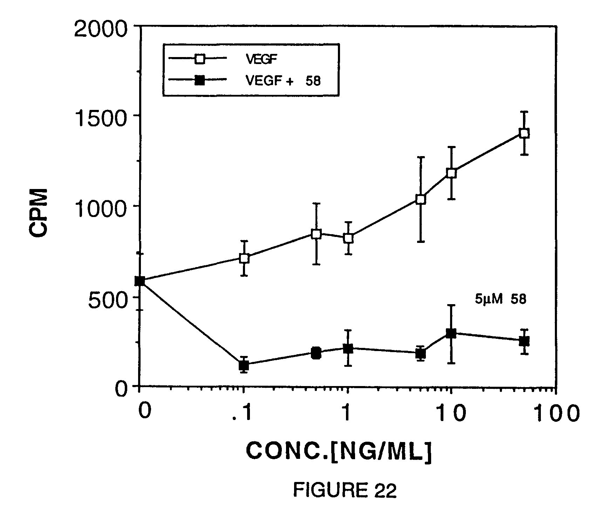

This example illustrates an investigation of inhibitory effects of compound no. 58 on Vascular Endothelial Growth Factor (VEGF)-induced proliferation in a human umbilical vein endothelial cell line (HUVEC). In this assay procedure, cells were grown in reduced serum (0.5% fetal calf serum) for 24 hours prior to stimulating with various concentrations of VEGF. VEGF has been shown to be important in tumor cell- mediated angiogenesis. Compound no. 58, at 5 µM, inhibited VEGF-induced proliferation at all concentrations of VEGF tested, as shown in Figure 22.

### Example 38

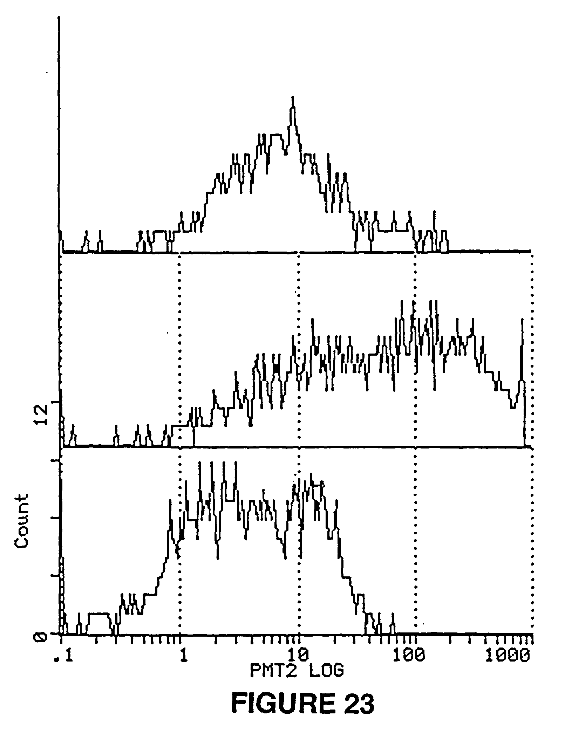

This example illustrates an investigation of inhibition of vascular cell adhesion molecule (VCAM) expression on HUVEC by compound no. 58. VCAM expression by endothelial cells is an early event in the pathogenesis of atherogenesis and multiple sclerosis, among other various autoimmune diseases. Figure 23 is a series of frequency histograms obtained in this exemplary assay. The top panel shows a frequency histogram obtained from flow cytometric analysis of HUVEC cells stained with an antibody directed against VCAM and a second stem goat anti-mouse-FITC antibody. In the absence of TNF, VCAM expression on HUVEC was at a very low level. The middle panel shows a frequency histogram of cells stimulated with TNF for 6 hours prior to analyzing by flow cytometry. The average increase in cell fluorescence was approximately 10-fold. The bottom panel is a frequency histogram of TNF-stimulated cells in the presence of compound no 58. Presence of inventive compound reduced mean fluorescence by a factor of 8, compared with mean fluorescence from TNF-stimulated cells in the absence of compound no. 58.

### Example 39

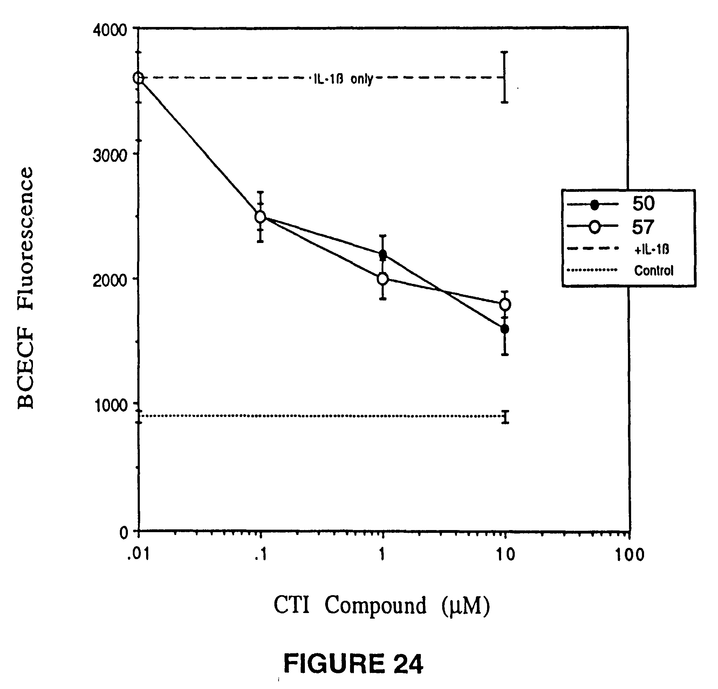

This example illustrates inhibitive activity of compounds nos. 50 and 57 on THP-1 cell adhesion to IL-1-activated HUVEC. In an investigative assay, HUVEC were stimulated with IL-1 (10 ng/ml), both in the absence and presence of varying concentrations of drugs for 8 hours in a 96-well microtiter plate. In the wellplate, human monocytic leukemia cell line THP-1 cells were added at 50,000 cells per well. The THP-1 cells were pre-incubated with BCECF, a fluorescence dye that can be used to measure cell number using a fluorescence plate reader. After 10 minutes at 37 °C, the microtiter plate was inverted and spun at 900 rpm. The remaining adhering THP-1 cells were then analyzed. As shown in figure 24, non-stimulated background adherence was approximately 1500 relative units, increasing to approximately 6500 under TNF stimulation. The inventive compounds tested significantly inhibited THP-1 adhesion, even at low concentratations.

### Example 40

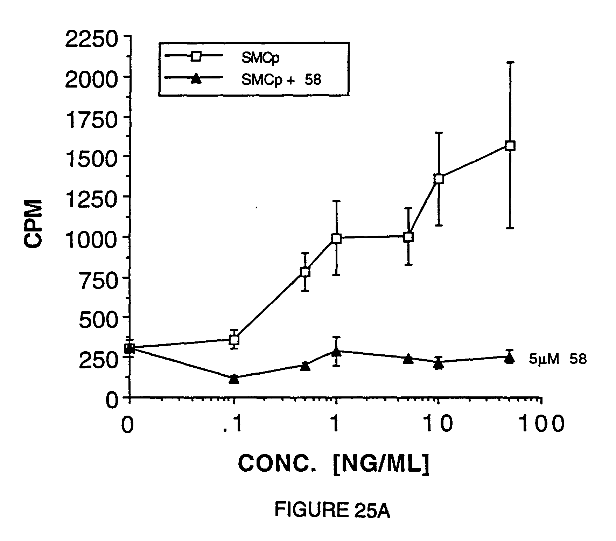

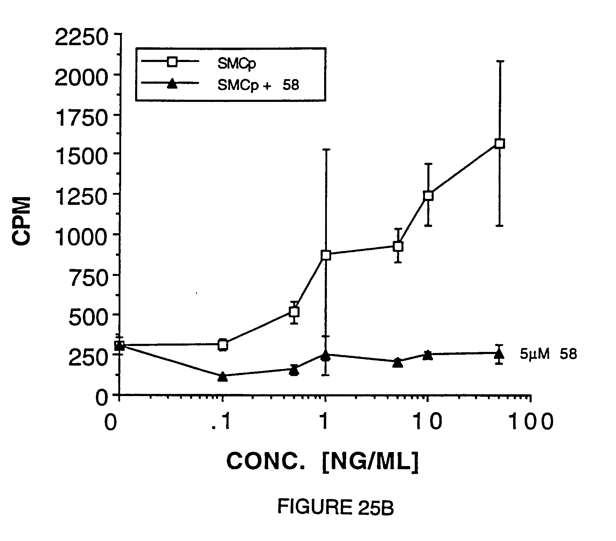

This example shows the inhibitory effect of inventive compound no. 58 on either aFGF or bFGF-induced proliferation in human pulmonary smooth muscle cells. Cells were grown in reduced serum (0.5% fetal calf serum) for 24 hours prior to stimulating with various concentrations of PDGF. Cells, stimulated with either aFGF or bFGF, were analyzed in the presence and absence of compound no. 58 (5 µM). Assay results are reported in figures 25A and 25B (aFGF and bFGF, respectively). As shown in the results, compound no. 58 was a potent inhibitor of both aFGF and bFGF-induced proliferation in this cell type, this pulmonary smooth muscle cell being representative of other cell types examined. No toxic effect of compound no. 58 was observed in this assay.

### Example 41

This example illustrates a synthesis of 1-Octylamino-10-hydroxy-11-(N-methylacetamido)undecane (compound no. 77). Sodium hydride (0.56 g, 23 mmol) was added to a solution of 1-bromo-10-undecene (5.4 g, 23 mmol) and N-methylacetamide (91.7 g, 23 mmol) in DMSO (40 ml). After 8 hours, the reaction was complete and the mixture added to water (100 ml) and extracted with dichloromethane (3 x 100 ml). The combined extracts were washed with water (70 ml) and saturated aqueous salt solution (70 ml), and dried over sodium sulfate. The solvent was evaporated under vacuum to give a yellow residue, which was chromatographed (silica/ethyl acetate), yielding 2.95g of 1-(N-methylacetamido)-10-undecene as a colorless oil (57% yield).

A solution of 1-(N-methylacetamido)-10-undecene (2.0 g, 8.9 mmol), N-methylmorpholine-N-oxide (4 ml of a 60% aqueous solution in water, 18 mmol) and potassium osmate dihydrate (10 mg) in acetone (50 ml), water (30 ml), and *t*-butanol (10 ml) was stirred at ambient temperature for 17 hours. Sodium dithionite (200 mg) was added to reduce the osmium catalyst. After 30 minutes, the mixture was added to dichloromethane (50 ml) and the layers separated. The aqueous layer was extracted with dichloromethane (3 x 50 ml). The combined organic layers were washed with saturated aqueous salt solution (50 ml) and dried over sodium sulfate, the solvent being subsequently evaporated under vacuum to a thick residue. The residue was purified by chromatography using silica and a dichloromethane/15% methanol eluant, resulting in 1.61 g of 1,2-dihydroxy-11-(N-methylacetamido)undecane (70% yield). This resulting diol (1.61 g, 6.2 mmol) was stirred in a 30% solution of hydrogen bromide in acetic acid (3.7 ml) for 1 hour. The solution was added to a mixture of sodium bicarbonate (7 g) in water (50 ml) and dichloromethane (25 ml). After bubbling ceased, the layers were separated and an aqueous layer extracted with dichloromethane (2 x 70 ml). The combined organic layers were washed with water (30 ml) and saturated aqueous salt solution (30 ml), then dried over sodium sulfate. The solvent was removed under vacuum to obtain 2.16 g of 1-(N-methylacetamido)-10-acetoxy- 11-bromoundecane as a colorless oil (96% yield). Sodium methoxide (351 mg, 6.5 mmol) was added to a solution of bromoacetate (2.16 g, 5.9 mmol) in methanol (15 ml). After 1 hour of stirring, the mixture was added to water (25 ml) and extracted with dichloromethane (3 x 25 ml). The combined organic layers were washed with saturated aqueous salt solution (25 ml) and dried over sodium sulfate. The solvent was evaporated under vacuum and the residue purified with chromatography using silica and an ethyl acetate eluant, yielding 831 mg of 1,2-oxido-11-(N-methylacetamido)undecane (58% yield).

A solution of 1,2-oxido-11-(N-methylacetamido)undecane (400 mg, 1.7 mmol), octylamine (0.26 g, 2 mmol), and lithium perchlorate (181 mg, 1.7 mmol) in dry acetonitrile (10 ml) was stirred for 16 hours. The mixture was added to water (20 ml) and extracted with dichloromethane (3 x 30 ml). The combined organic layers were washed with saturated salt solution and dried over sodium sulfate. The solvent was removed under vacuum. Purification of the resulting residue with silica chromatography using a dichloromethane/5% methanol/5% triethylamine eluant yielded 416 mg of compound no. 77 (56% yield).

### Example 42

This example illustrates inhibitive effects of the inventive compounds on Balb/3T3 cell proliferation in response to PDGF stimulation. Research indicates that the inventive compounds possess inhibitory effects on PDGF-induced proliferation of Balb/3T3 cells. Balb/3T3 cells respond vigorously to PDGF stimulation, and are useful *in vitro* models for further study of PDGF-induced proliferation. Disregulated PDGF-proliferative response has been linked to a variety of diseases, including, *e.g.*, restenosis, atherosclerosis, fibrosis, and tumor cell angiogenesis. Cells were plated in low serum-containing medium for 24 hours prior to stimulation with various concentrations of inventive compounds nos. 77, 7 and 78.

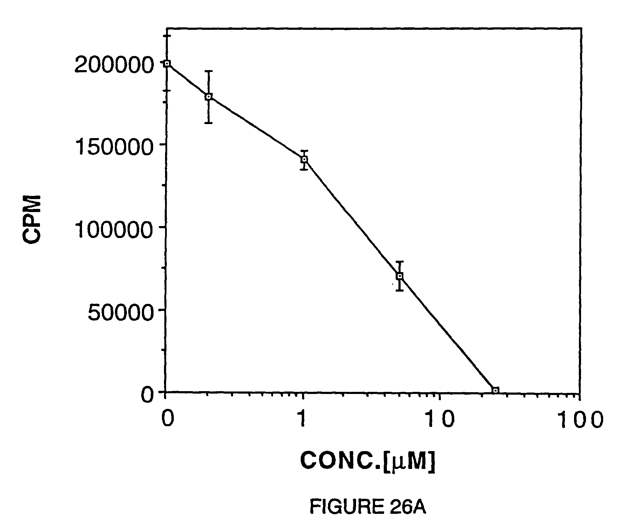

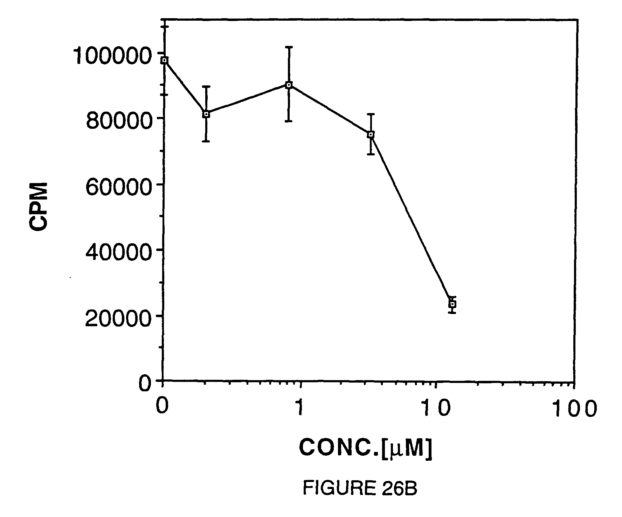

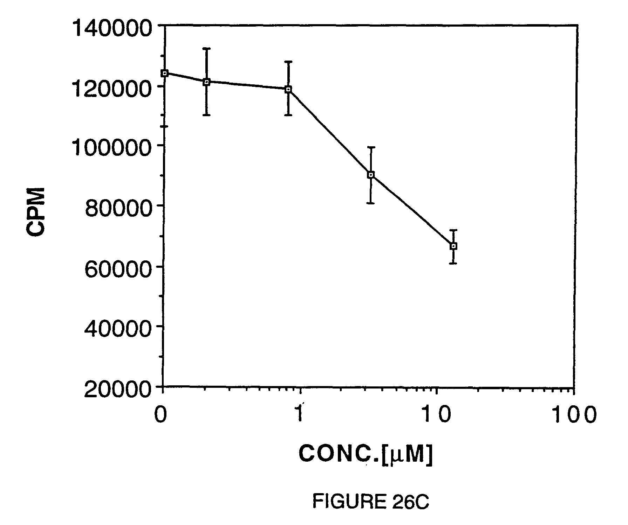

Inventive compounds nos. 77,7 and 78 were added at various concentrations one hour prior to PDGF stimulation. PDGF-BB was added at a constant 10 µM concentrations along with tritiated thymidine. The cells were allowed to incubate for one day, following addition of PDGF and tritiated thymidine. 24 Hours later, the cells were harvested and counted by liquid scintillation counting. Results for compounds nos.77, 7 and 78 are shown in figures 26A, 26B and 26C, respectively. Background counts were approximately 1% of control levels. The results illustrate that the drugs tested inhibit Balb/3T3 proliferation in this predictive, *in vitro* model.

### Example 43

This example illustrates dose response curves used to generate 50% inhibition concentrations (IC₅₀) for inventive compounds nos. 78, 79 and 80 for murine thymocyte proliferation, co-stimulated by ConA and IL-2. Thymuses, obtained from normal, female Balb/C mice, were dissociated and plated into 96-well plates at a density of 2 x 10⁵ cells/well. ConA (0.25 mg/ml) and IL-2 (15 U/ml) were added to the wells. The cells were incubated for 4 days at 37 °C. On day 4, the cells were pulsed with tritiated thymidine and incubated for an additional 4 hours. The amount of tritiated thymidine dye incorporated by the harvested cells was determined in a liquid scintillation counter. Doses (shown in figs. 26A, 26B and 26C) were added two hours prior to ConA and IL-2 activation. Background counts were less than 200 cpm. IC₅₀ values for the inventive compounds tested were 12.4, 10.6 and 3.8 µM for compounds nos. 78, 79 and 80, respectively. All tested compounds inhibited thymocyte proliferation and activation.

### Example 44

This example illustrates conclusive data, compiled from various *in vitro* and *in vivo* assay results that the inventive compounds, as represented by a species of the disclosed genus (compound no. 50) are anti-cancer therapeutics.

Generally, growth and spread of malignant tumors (rapid cell growth, uncontrolled by normal regulatory mechanisms) characterize cancer diseases. Precise causes of cancer remain unknown. Preclinical and clinical trials with the inventive compounds indicate that cancer cell growth may be regulated through the second messenger pathway. Oncogenic mutations result in abnormal continuous stimulation of the pathway, leading to unregulated and undifferentiated growth (*i.e.*, malignant transformation). Cancer cells metastasize (*i.e.*, break through blood vessels and travel to distant body sites) and secrete enzymes called metalloproteases, which "break down" blood vessel walls, allowing the cancer cells to enter the bloodstream and form remote tumors (proteolysis). One such metalloprotease is Type IV collagenase. In addition, tumor cell adhesion receptors (integrins) effect attachment--apparently necessary for tumor residence in organs--of tumor cells to blood vessel walls and normal organs. Cancer cells also secrete certain proteins, such as bFGF, that stimulate new blood vessel development (angiogenesis or neovascularization), these new blood vessels supplying nutrients fostering malignant tumor growth. Resent research results suggest that the second messenger pathway appears integral to Type IV collogenase production, adhesion receptors expression and bFGF secretion.

Unlike conventional anti-cancer therapies, the inventive compounds, by inhibiting the second messenger pathway, decrease: tumor cell growth by blocking oncogene-induced events; metastatic potential by blocking metalloprotease production; tumor adhesion to normal organs by blocking adhesion receptor expression; and a tumor's ability to induce nutrient-carrying blood vessel formation by blocking bFGF or other tumor-dependent growth factor signaling.

The inventive compounds exhibit anti-cancer properties against several malignant conditions, including lung, breast and colon cancer, and unlike conventional cancer chemotherapy, *in vitro,* the inventive compounds are non-toxic to normal cells at concentrations lethal to cancer cells. *In vitro* data obtained for representative compound no. 50 include the following:

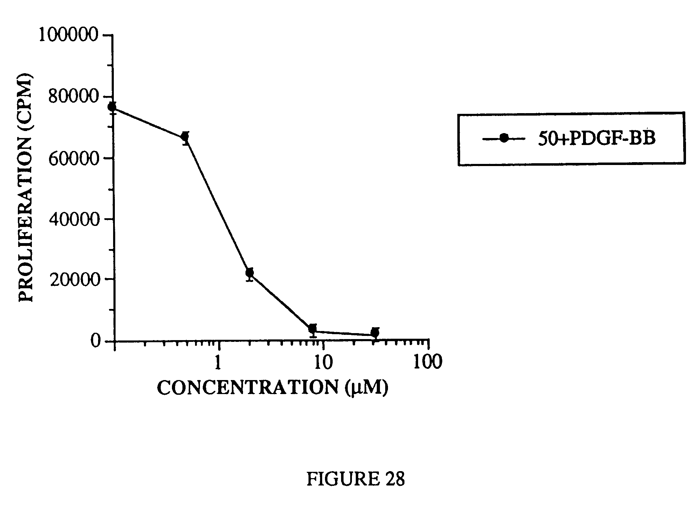

Results obtained for compound no. 50 in the PDGF-induced proliferation of Balb/3T3 cells (Example 32), reported in figure 28, illustrate the inhibitive properties of this compound on proliferation induced by PDGF, representing significant inhibition at concentrations as low as 2 µM. This ability to block proliferation (representative of an oncogene-induced event in tumor-cell growth) suggests that the inventive compounds, specifically compound no. 50, are capable of reducing tumor cell growth by blocking oncogene-induced events.

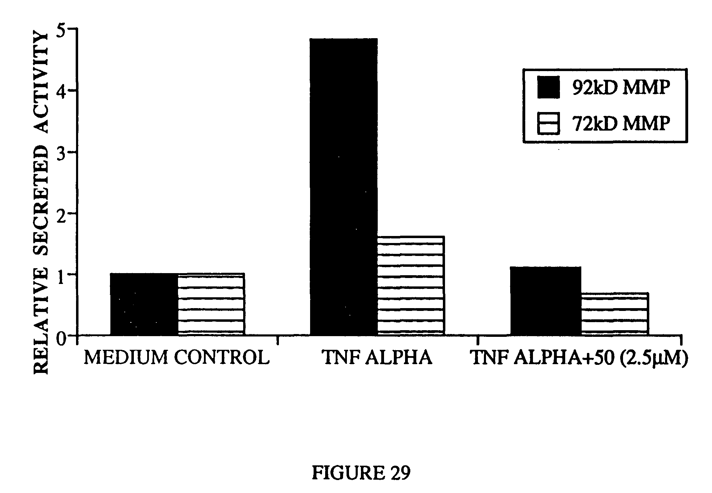

Another assay measures an ability of the inventive compounds, as represented by compound no. 50, to inhibit metalloproteinase production in cancer cells. In the assay protocol, THP-1 human leukemia cells (1-2 x 10⁶/35 mm dish) were plated in RPMI medium with 0.5% serum. Inventive compound no. 50 was added at 2.5 µM. Following incubation for 1 hour, TNF-α was added and incubated for 18 hours. The supernatants from control and treated plates were collected and protease activity was determined in gelatin gels (zymogram) after electrophoretic protein separation. As shown in figure 29, TNF-α substantially increased expression of the 92 kD matrix metalloprotease (MMP) and moderately increased production of the 72 kD MMP. The presence of compound no. 50 blocked the TNF-α-stimulated expression of both the 92 and 72 kD MMPs. Compound no. 50, representative of compounds of the invention, is capable of substantially reducing metastatic potential of cancer cells by blocking metalloprotease production.

Other *in vitro* and *in vivo* evidence of this potent activity of the inventive compounds is represented in the following experimental results.

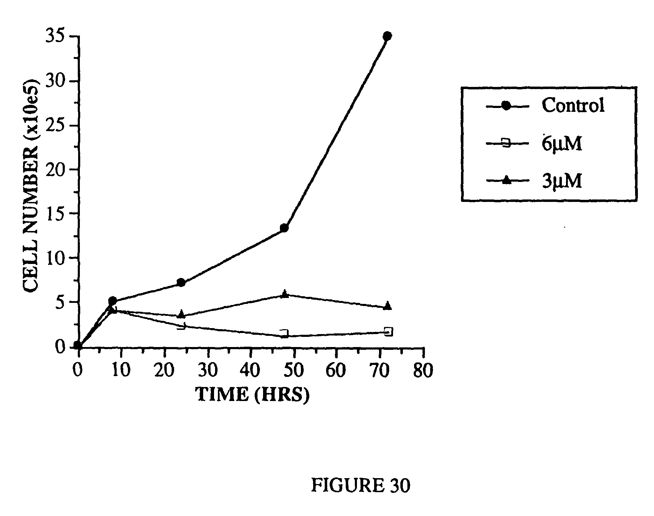

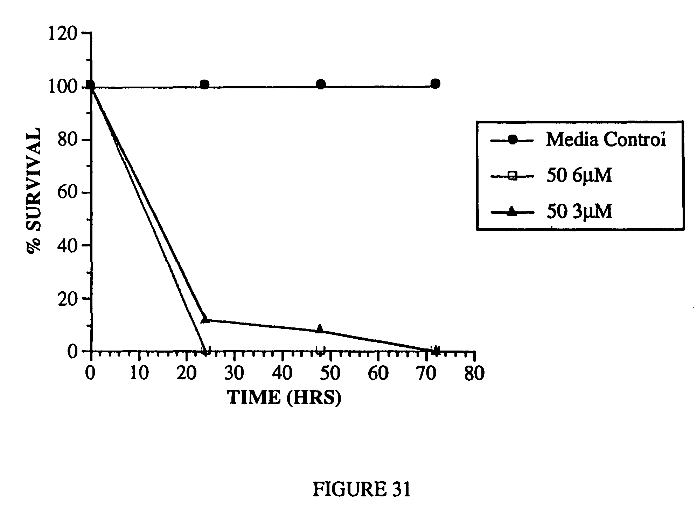

Figures 30 shows anti-proliferative activity and figure 31 reports anti-clonogenicity activity of inventive compound no. 50 with HT-29 cells. HT-29 cells (1 x 10⁵ cells/35 mm dish) were plated in McCoy's medium with 10% serum and incubated overnight. Concentrations of 3 and 6 µM of compound no. 50 were added and viable cell counts made at the times shown. For clonogenic assays, treated and control cells (300/plate) were plated and allowed to grow colonies. After 7 days the colonies were fixed and counted. The values in figure 31 are the means of 3 plates.

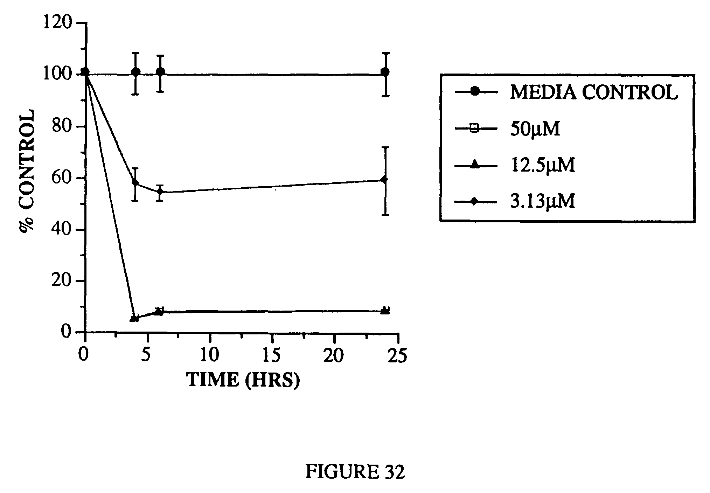

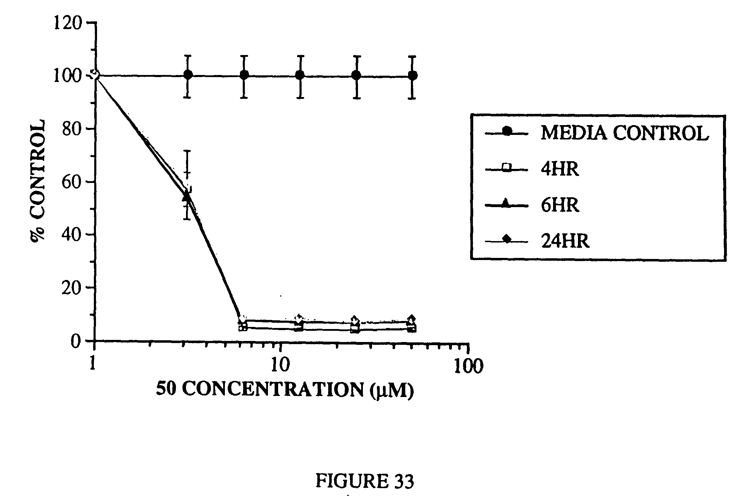

Figures 32 and 33 illustrate cytotoxicity and concentration dependence of inventive compound no. 50 against 3LL cells (Lewis lung carcinoma). 3LL cells (3 x 10³ cells/well) were plated and incubated overnight in RPMI medium containing 10% serum. Compound no. 50 was added at different concentrations and cell number determined at various time points by a vital dye uptake method. The values shown are triplicate of wells.

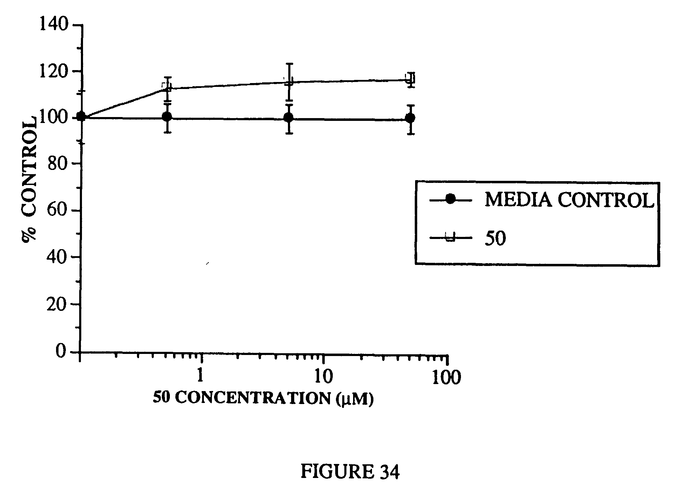

Figure 34 shows that inventive compound no. 50, even at much higher concentrations than shown having cytotoxicity to tumor cells, lacks cytotoxic activity in normal human bone marrow stromal cells. Human bone marrow stromal cells (1 x 10⁴ cells/well) were plated in 96 well plates in McCoy's medium with serum and incubated overnight. Different dilutions of inventive compound no. 50 were added and viable cell counts made by vital dye uptake.

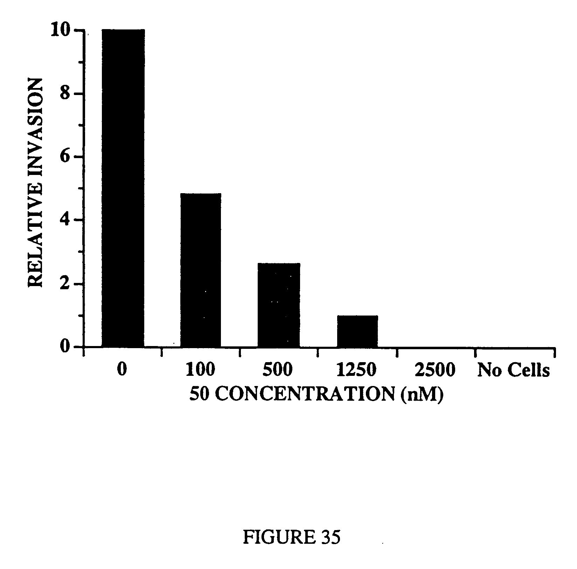

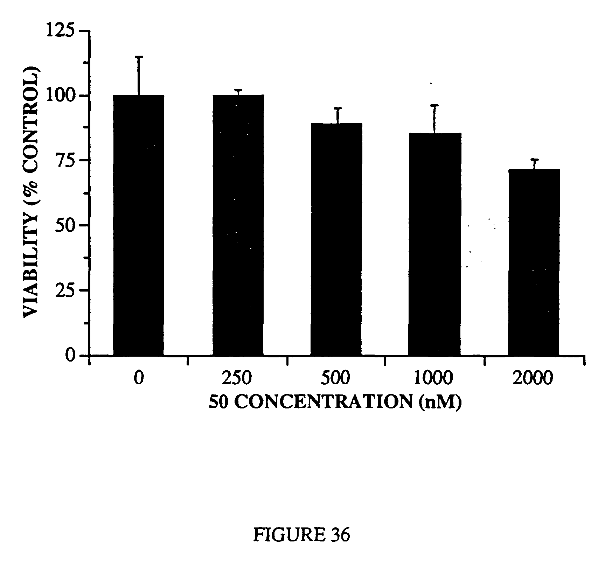

Figures 35 and 36 show the effects of inventive compound no. 50 on matrigel invasion and viability in 3LL cells. 3LL cells (4.5 x 10⁵ cells/well) were plated into the inner membrane of matrigel chambers. Different concentrations of compound no. 50 were added to the chamber and incubated for 48 hours at 37 °C. The cells on top of the membrane were removed and cells that migrated to the bottom were stained with Diff Quick Solutions and scored for relative invasion. The effect of inventive compound no. 50 on viability of 3LL cells at different concentrations was determined separately.

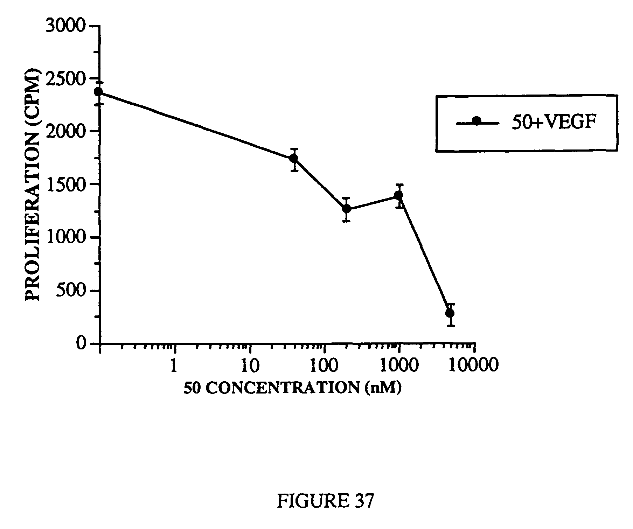

Figure 37 illustrates VEGF induced proliferation of HUVEC as a predictive adhesion assay. HUVEC were plated in EBM medium with serum and allowed to grow for 4 days. Different dilutions of inventive compound no. 50 were added to the plates, followed by VEGF (50 ng/ml) along with tritiated thymidine (1 mCi/ml). Proliferation was measured in quadruplicate and these data show the effect of inventive compound no. 50 to inhibit adhesion.

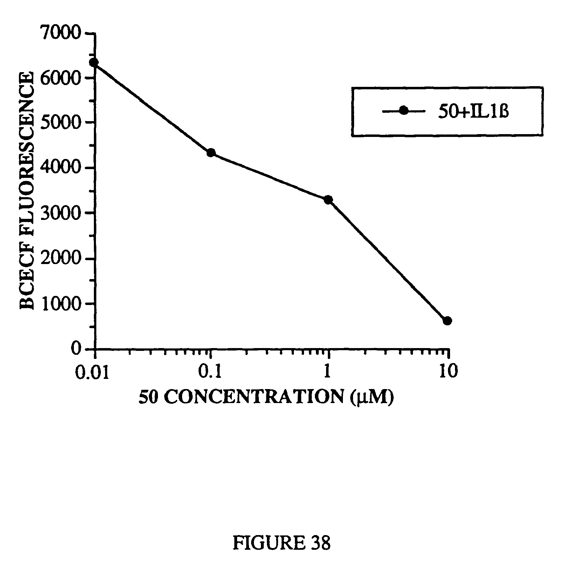

Figure 38 shows the effect of inventive compound no. 50 on THP-1 adherence to IL-1β-stimulated HUVEC. HUVEC (4 x 10³ cells/well) were plated in RPMI medium with 10% serum and incubated for 48 hours. Different concentrations of inventive compound no. 50 were added and incubated for 1 hour. IL-1β (15 ng/ml) was added and incubated for 6 hours. Exponential growth THP-1 tumor cells, prestained with dye BCECF, were added (1.5 x 10⁵ cells/well) and allowed to adhere for 20 minutes. The number of adhering tumor cells was determined after washing to remove non-adherent cells.

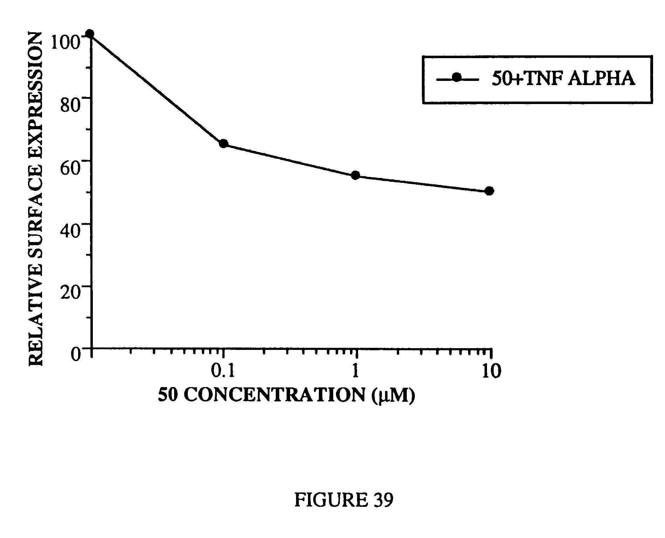

Figure 39 illustrates the effect of inventive compound no. 50 on VCAM-1 surface expression of TNFα-stimulated HUVEC. This assay is a predictive model of adhesion. HUVECs grew to 90% confluence in 6 well plates in RPMI medium with 10% serum. Compound no. 50 was added at different concentrations and incubated for 30 minutes. TNFα (20 ng/ml) was added and the cells were incubated for 5 hours. The cells were collected and the amount of VCAM-1 determined by indirect immunostaining followed by fluorescence activated cell sorter (FACS) analysis. Mean fluorescent intensity of TNFα-stimulated cells was normalized to 100% with drug-treated samples expressed as a percent of control.

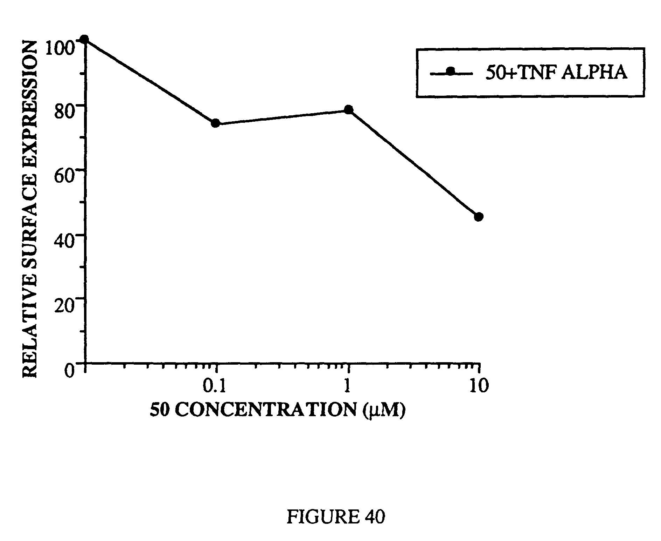

Figure 40 illustrates the effect of inventive compound no. 50 on ICAM-1 surface expression of TNFα-stimulated HUVEC. This assay is a predictive model of adhesion. The procedures followed were the same as used in the immediately preceding assay, except that the cells were stained with an ICAM-1 antibody.

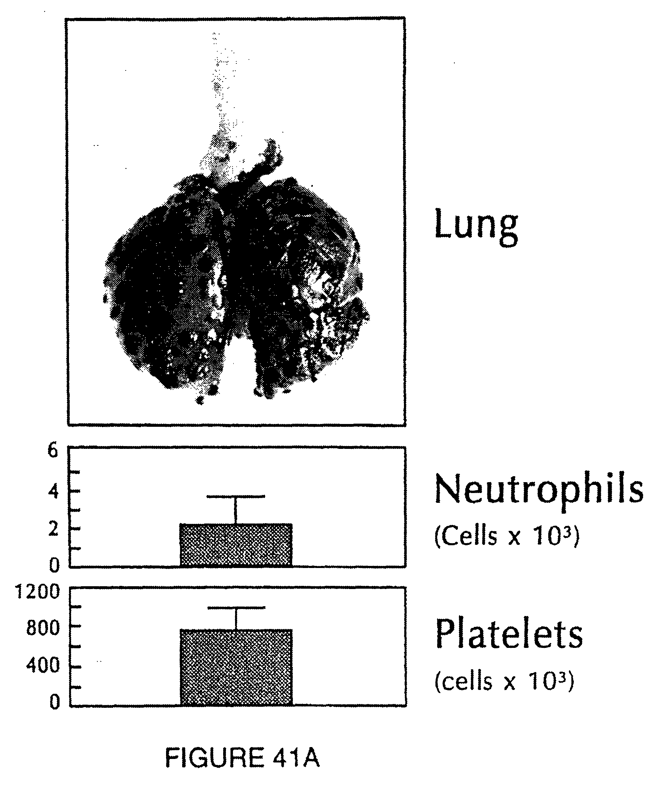

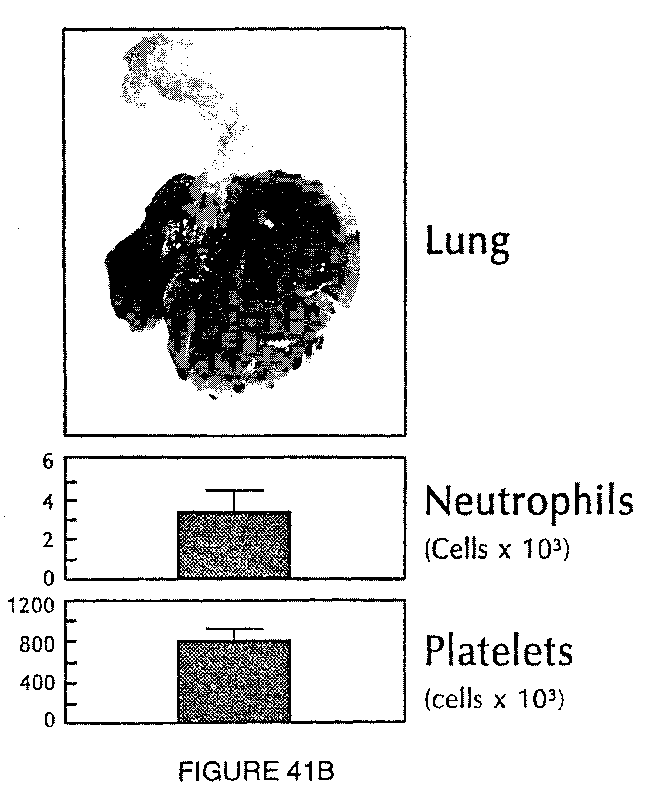

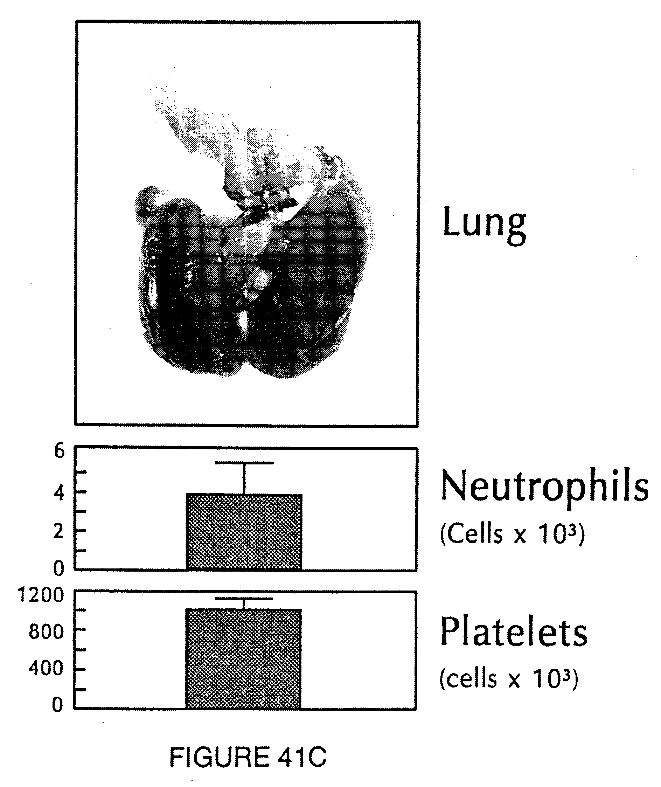

Figures 41A, 41B and 41C illustrates the results from an *in vivo* study in mice where B16 melanoma cells were injected intravenously through a tail vein on day 0 and compound no. 50 was administered inter parenterally at 10 mg/kg QD or 20 mg/kg QOC, starting days 1-14. The mice were sacrificed on day 15 and the lungs were dissected and fixed in formalin. The number of black metastatic foci were scored and are illustrated in the three lungs shown in figures 41A, 41B and 41C. In addition bone marrow toxicity was evaluated by measuring neutrophil and platelet counts in the mice on day 15. These data are shown graphically below the lung photographs in each respective figure. These data show that at either dose administered compound no. 50 was not toxic to the bone marrow (in contrast to every known cancer chemotherapy regimen), and in fact, increased counts over non-treated control animals.

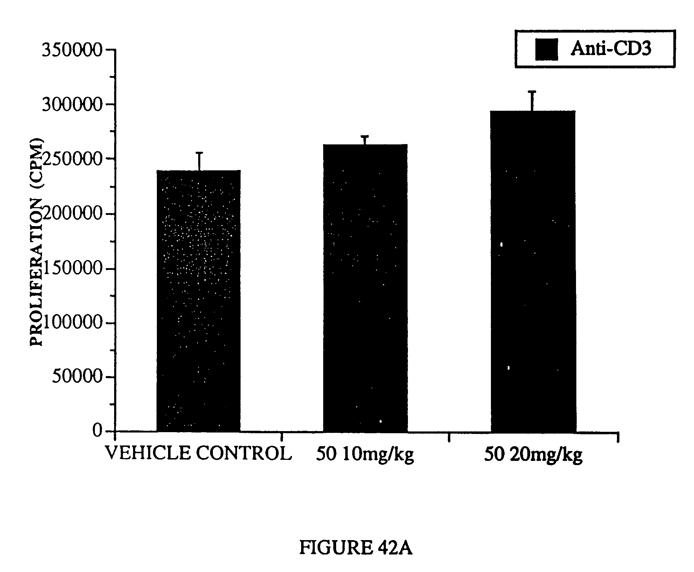

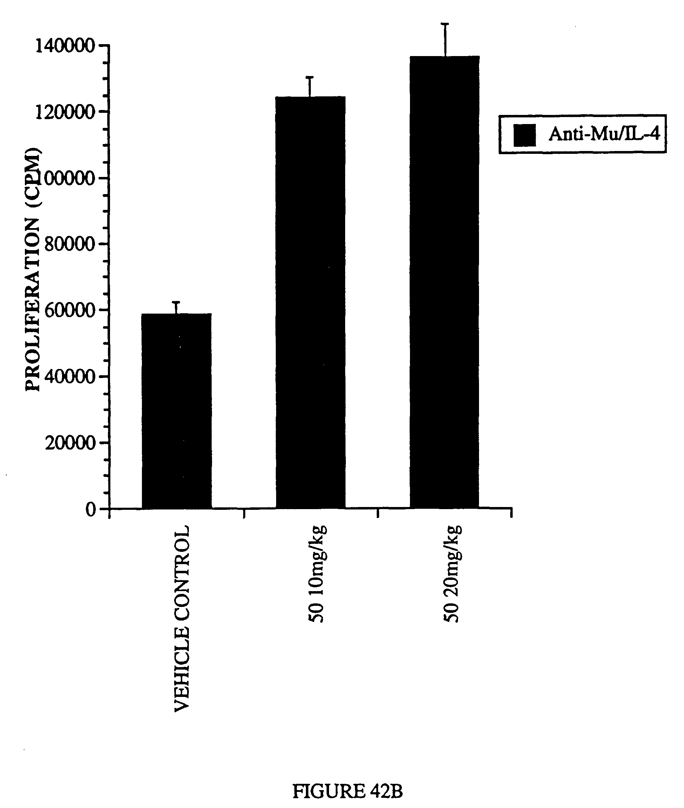

Figures 42A and 42B illustrate T and B cell response assays of mice treated with inventive compound no. 50 in the immediately preceding assay protocol. Spleens from these treated mice were made into single cell suspensions in RPMI medium, supplemented with 10% serum, and placed (200,000 cells/well) in flat-bottomed 96 well plates. Anti-CD3 (figure 42A) or a mixture of an anti-mu/IL-4 (fig. 42B) were added to the wells at final concentrations of 1 mg/ml and 1 mg/ml/12.5 ng/ml, respectively. Appropriate positive and negative controls were set up on each plate and all samples were assayed in quadruplicate. The plates were incubated for 2 days and proliferation was measured by tritiated thymidine incorporation.

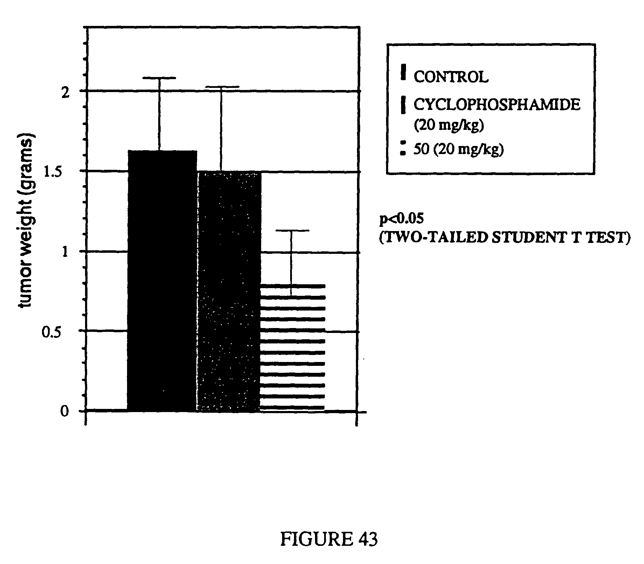

Figure 43 reports results in an *in vivo* experiment showing that inventive compound no. 50 can arrest growth of Lewis Lung Carcinoma in mice. BDFI mice were injected subcutaneously with 1 x 10⁶ 3LL cells on day 0 and then treated with inventive compound no. (20 mg/kg i.p.), cyclophosphamide (20 mg/kg) or vehicle on alternate days beginning on day 7. The animals were sacrificed on day 20 and the lung tumors dissected and weighed. Figure 43 illustrates that compound no. 50 showed superior results over an existing cancer chemotherapeutic agent.

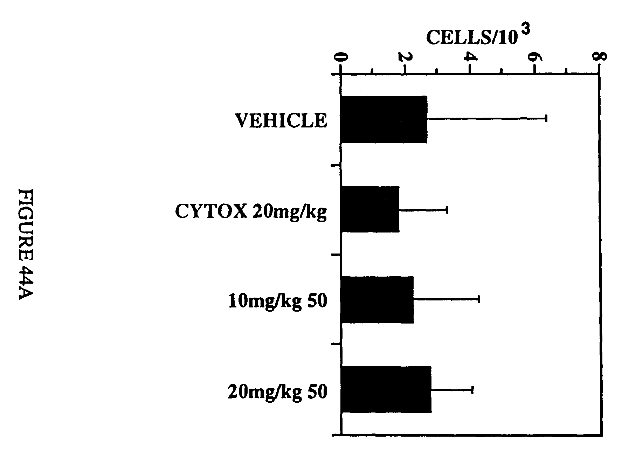

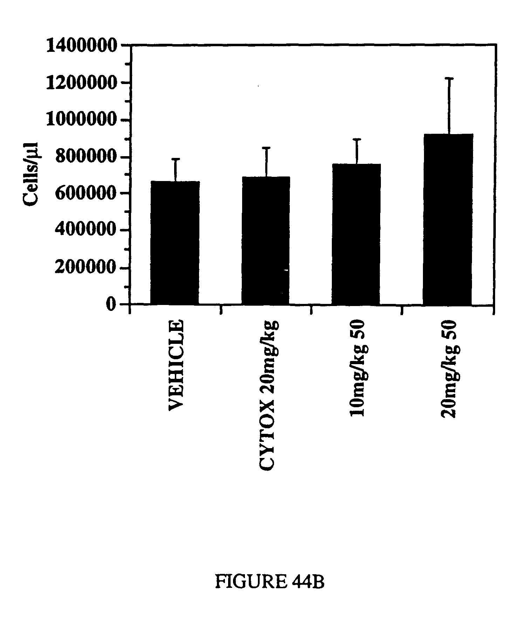

Figure 44A and 44B illustrates platelet and neutrophil counts, respectively, of the sacrificed mice in the immediately preceding assay. In addition, platelet and neutrophil counts in the mice were not altered from vehicle, indicating that bone marrow was not a target of toxicity for inventive compound no. 50.

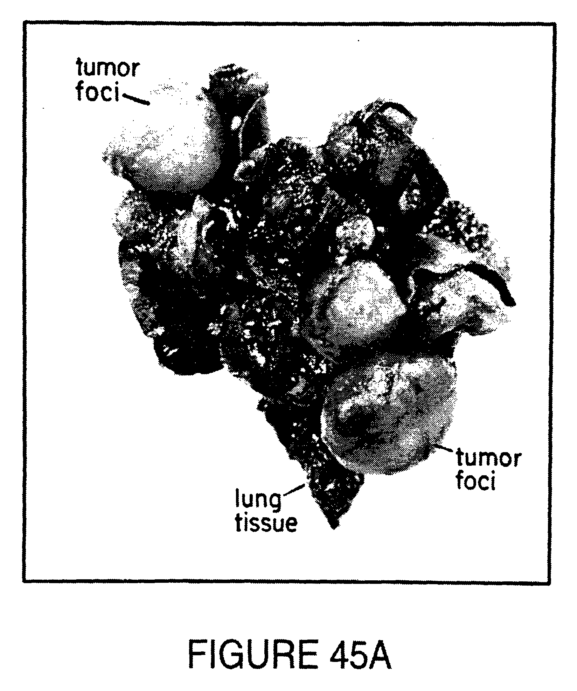

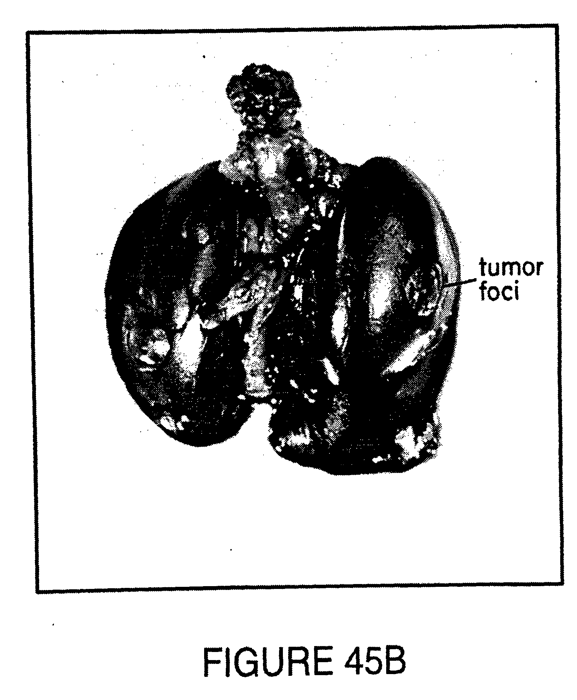

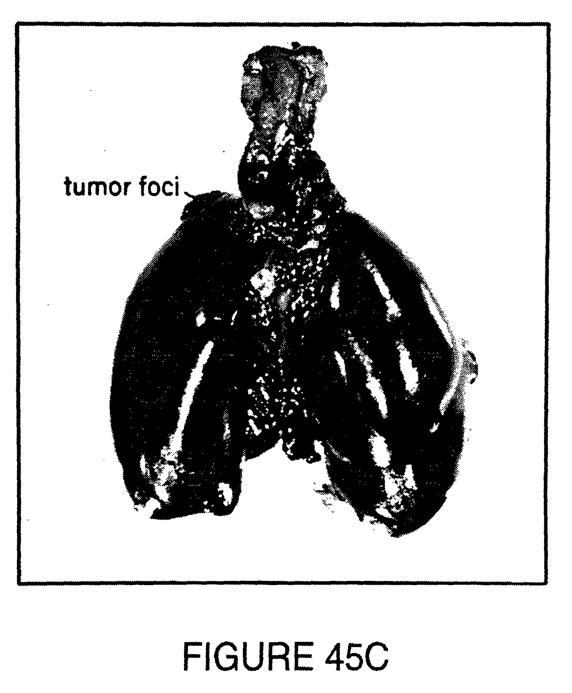

Figures 45A, 45B and 45C illustrate a photographic comparison of lungs from the 3LL exposed mice with or without treatment using inventive compound no. 50. The lungs were injected with india ink such that normal lung tissue stains black and tumors appear white. The difference in lungs treated seven days after tumor cell administration was visually apparent. These *in vivo* data are highly predictive of significant clinical activity and reduced bone marrow toxicity of the inventive compounds.

### Example 45

This example illustrates conclusive, *in vitro* and *in vivo* evidence predicting that the inventive compounds, as represented by a species of the disclosed genus (compound no. 45), are effective therapies for autoimmune diseases and suppressing an immune response.

One such autoimmune disease, rheumatoid arthritis, is triggered by unknown environmental or endogenous events in a genetically susceptible individual. Dysregulation of B lymphocytes leads to the production of immune complexes. Activated T cells in involved joints (predominantly of the CD4 helper type) promote autoantibody production. Macrophages and dendritic cells produce large numbers of inflammatory cytokines, further stimulating lymphocytes. In addition, both lymphocytes and macrophages produce other cytokines which stimulate proliferation of synovial cells leading to "pannus" formation and joint deformity. Degradative enzymes such as type IV collagenase are released into the joint space and destroy connective tissue including the cartilage on articular surfaces. Lymphocyte- and macrophage-released cytokines play various roles in the pathogenesis of joint destruction and systemic symptoms in rheumatoid arthritis. Compounds that affect only a single component of this complex process are unlikely to be effective or disease-modifying unless these compounds target a process that is both proximal in the cascade of and fundamental to the inflammatory process itself. Because biologic systems appear invariably redundant, conventional treatments target a single aspect of a complex reaction and are thus only partially effective.

The inventive compounds interrupt many key components of the cascade of events that lead to both joint destruction and the systemic complications of rheumatoid arthritis. Specifically in the following *in vitro* and *in vivo* assays, inventive compound no. 45 (as a representative compound of the invention) inhibited: T and B cell proliferation, thus inhibiting abnormal autoantibody production; macrophage activation, cytokine production, and most importantly, signaling by multiple cytokines (for example, T and B cell driven proliferation in response to IL-2, IL-4, IL-7, TNFa and T cell receptor activation by antigen); proliferative signals to synovial cells in response to PDGF, FGF, EGF, and insulin-like growth factors (IGF); adhesion molecule expression (including VCAM and ICAM), stimulated by local inflammation, a suppression of these adhesion molecules likely leading to a decrease in lymphocyte and macrophage trafficking to the inflammation site, thus decreasing amplification of the inflammatory process. Therefore, the inventive compounds, represented by compound no. 45, inhibit, from multiple points, the inflammatory and dysregulated immune response resulting in acute and chronic symptomatology of diseases such as rheumatoid arthritis.

Data from *in vitro* and *in vivo* confirms activity of the inventive compounds as an immunosuppressive therapeutic, as represented in the following results.

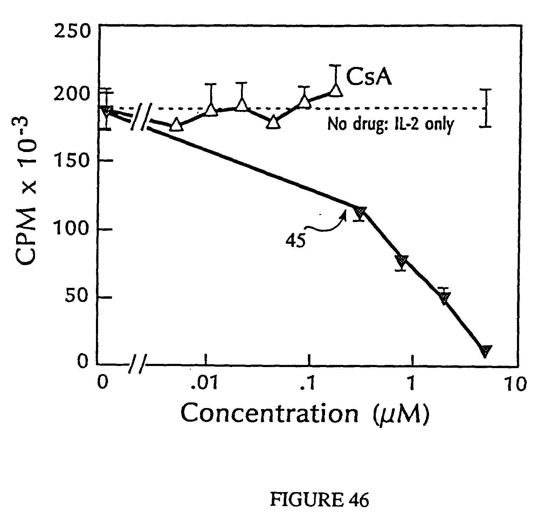

CT-6.1 cells were starved overnight and stimulated with IL-2 (20 ng/ml). Twenty hours later, cells were labeled for 4 hours with ³H-TdR and counted by liquid scintillation. Background counts were approximately 2000 CPM. The IC₅₀ for inhibition in this cell line was approximately 0.8 µM. CsA, used as a negative control, had no effect on IL-2-mediated proliferation. Figure 46 reports results obtained in this assay, illustrating inhibition of proliferation by inventive compound no. 45, as measured by ³H-TdR incorporation using cell counts.

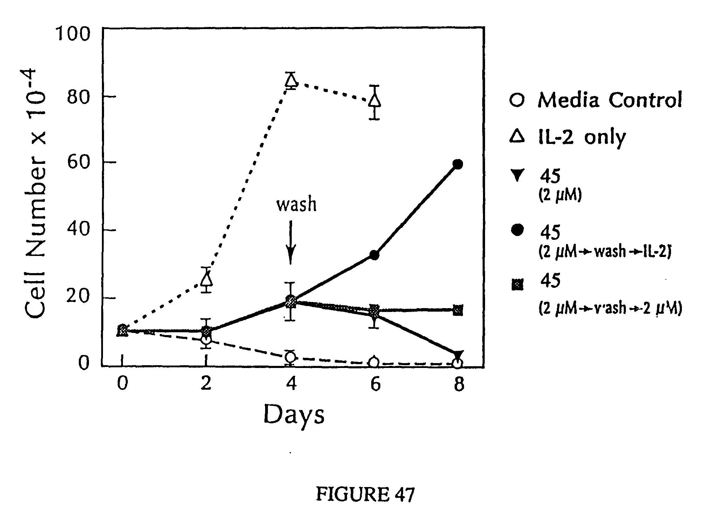

CT6.1 cells were cultured with IL-2 with or without the addition of inventive compound no. 45 (2 µM) and viable cells counted for up to 8 days. On day 4, two cultures previously incubated with compound no. 45, were washed and recultured with fresh IL-2 with or without compound no. 45 (2 µM). Results reported in figure 47 illustrate that inventive compound no. 45 inhibits proliferation as measured by cell number, its effect being reversible even after 4 days in culture.

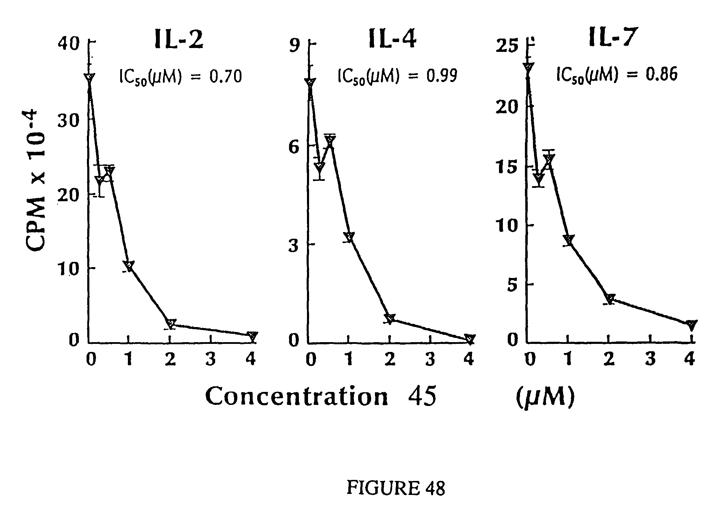

The results shown in figure 48 illustrate that inventive compound no. 45 also inhibited mitogenic responses to IL-2, IL-4 and IL-7. Procedurally, CT-6.1 cells were starved overnight and stimulated with either IL-2 (20 ng/ml), IL-4 (50 ng/ml) or IL-7 (25 ng/ml). ³H-TdR was added at 20 hours, cells were harvested 4 hours later, and the incorporation of ³H-TdR was determined. Inventive compound no. 45 illustrated IC₅₀'s < 1.0 µM for IL-2, IL-4 or IL-7-stimulated proliferation of CT6.1 cells. All of the incorporation studies were confirmed with parallel cell counting experiments (data not shown). These data predict that the inventive compounds induce immunosuppression similar to an X-linked SCID-like condition.

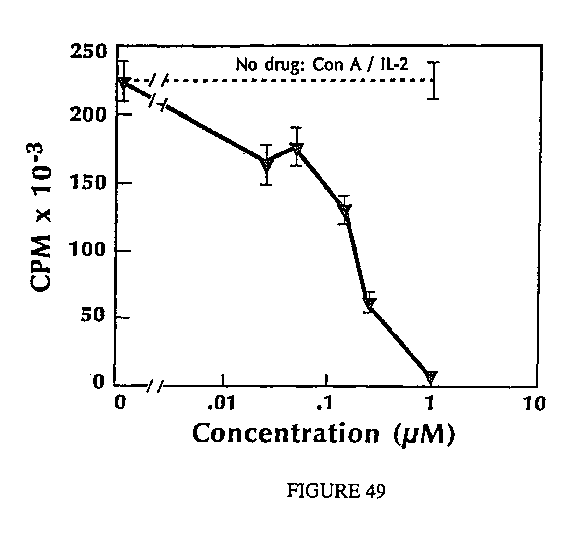

In another *in vitro* protocol, thymocyte proliferation was induced by sub-mitogenic doses of Con A (0.25 µg/ml) and IL-2 (20 ng/ml). Cells were stimulated for 96 hours, prior to addition of ³H-TdR for 4 hours. The cells were harvested and incorporation of ³H-TdR was determined. Background counts were approximately 2000 cpm. In the results reported in figure 49, inventive compound no. 45 also inhibited murine thymocyte proliferation in response to ConA and IL-2, with an IC₅₀ of approximately 0.35 µM.

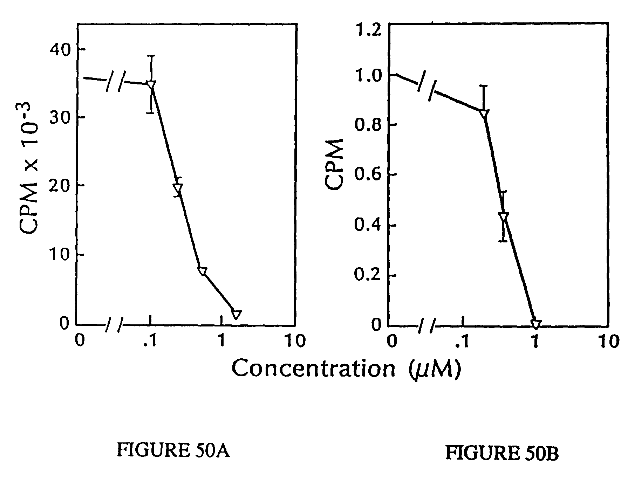

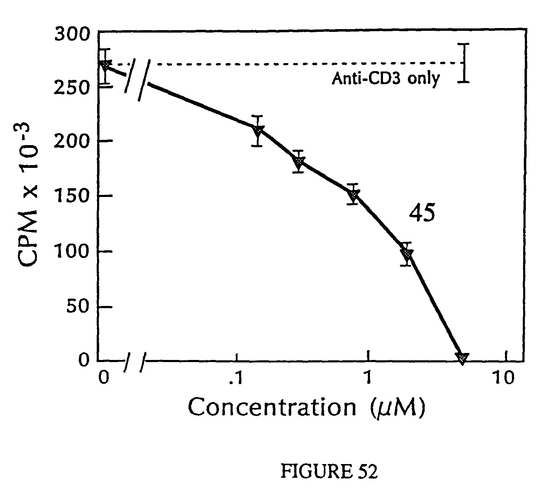

Figures 50A and 50B illustrate that compound no. 45 inhibits a murine mixed tumor lymophocyte culture (MTLC) and a human mixed leukocyte reaction (MLR). In the MTLC, splenocytes were stimulated (co-culture) with an alloantigen, B cell tumor target cell line (2PK3) in the presence or absence of compound no. 45. Cells were stimulated for 3 days, ³H-TdR was added and the cells were harvested 4 hours later. Incorporation of ³H-TdR was determined by liquid scintillation. Background counts were 1000-1200 CPM. For the human MLR, purified peripheral lymphocytes from two HLA disparate individuals were co-cultured for 6 days with or without compound no. 45. On the 7th day the cultures were pulsed with ³HTdR for 24 hours and counted by scintillation. As reported in figures 52A and 52B, an IC50 for the MTLC and MLR were both approximately 0.5 µM.

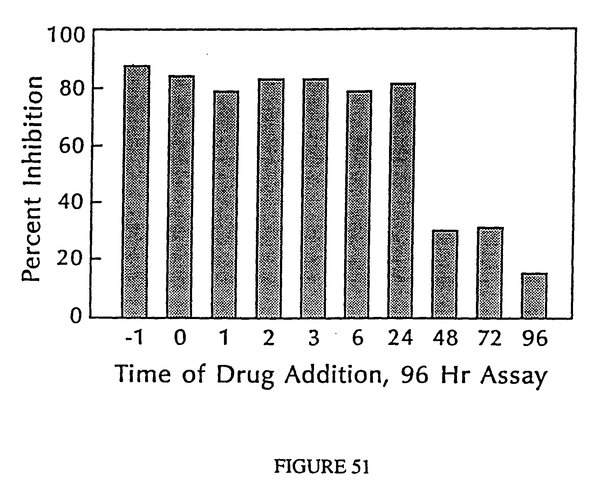

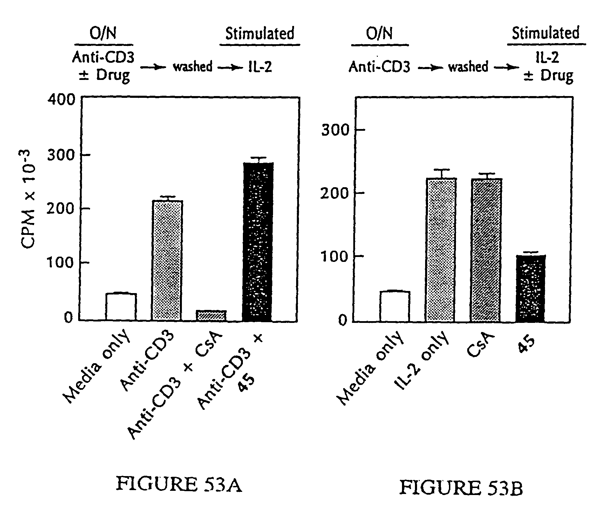

Figure 51 illustrates an effect of the delayed addition of inventive compound no. 45 on co-stimulated thymocyte proliferation. In an assay procedure, murine thymocytes were stimulated with ConA and IL-2 with 1 µM compound no. 45 added either 1 hour prior to IL-2 (-1), simultaneous with IL-2 (0) or at various times following IL-2 addition (1-92 hour). At 92 hours the thymocytes were pulsed with ³H-TdR, harvested and counted by scintillation 4 hours later. The results reported in Figure 53 illustrate that inventive compound no. 45 maximally inhibits thymocyte proliferation even when added 24 hours following the IL-2 stimulation. Furthermore, significant inhibition remained even at 72 and 92 hours following addition of IL-2.

Figure 52 illustrates that inventive compound no. 45 inhibited anti-CD3 stimulated splenocyte proliferation. In the assay, murine splenocytes were activated with a monoclonal antibody directed against CD3 (1 µg/ml), resulting in an IL-2 mediated proliferative response. Background was approximately 2000 cpm. As reported in figure 52, the IC₅₀ for inhibition in splenocytes was approximately 0.85 µM.

Figures 53A and 53B illustrate that compound no. 45 did not inhibit T cell receptor (CD3) mediated signaling. Procedurally, murine splenocytes were incubated overnight with anti-CD3 in the presence or absence of CsA (0.1 µM) or compound no. 45 (1 µM)--fig. 53A. Following overnight incubation, cells were washed and restimulated with IL-2 for 20 hours without addition of CsA or inventive compound. The cells were pulsed with ³H-TdR for 4 hours and harvested and incorporation of ³H-TdR determined. Preincubation of splenocytes with CsA together with anti-CD3 blocks the ability of the cells to respond to IL-2. This is due to the fact that CsA inhibited TCR mediated up-regulation of the IL-2 alpha chain receptor (CD25). In contrast, preincubation of the splenocytes with the inventive compound and anti-CD3 did not block the splenocyte's ability to respond to IL-2.

If the cells were first treated with anti-CD3 overnight, washed and recultured using IL-2 with CsA (0.1 µM), proliferation was not inhibited compared to the IL-2 only control--figure 53B. CsA did not block IL-2 mediated signaling events. However if the anti-CD3 "primed" splenocytes were stimulated with IL-2 in the presence of CT-2576 (1 µM), proliferation was inhibited. These data demonstrate that inventive compound no. 45 specifically inhibited IL-2 induced proliferation of anti-CD3 activated splenocytes, without blocking TCR-mediated activation, therefore predicting a distinctly different mechanism of action than CsA.

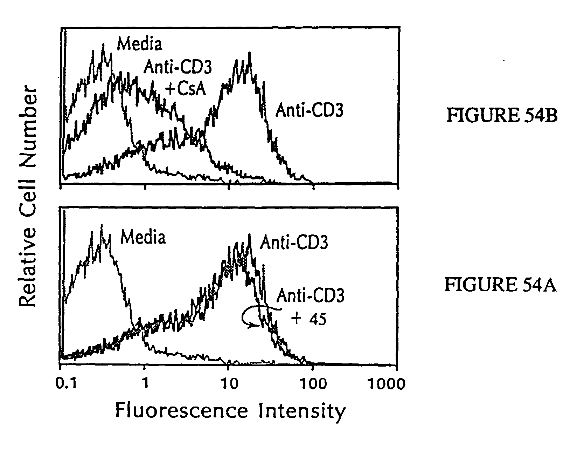

In another assay, inventive compound no. 45 did not inhibit anti-CD3 mediated upregulation of the IL-2 receptor alpha subunit. These data are presented in the histograms in figures 54A and 54B. Murine splenocytes were activated for 24 hours with anti-CD3 (1 µg/ml), stained with a fluorescent antibody to CD25 and analyzed by flow cytometry. Figure 54A is a histogram for cells treated with or without 100 nM CsA or the media control. CsA inhibits CD-25 receptor upregulation by inhibiting TCR signaling. However, in figure 54B, a similar experiment is shown, except that the cells were treated with inventive compound no. 45 (1 µM) rather than CsA. Results reported confirm that inventive compound no. 45 did not inhibit CD-25 (p55) receptor upregulation by anti-CD3.

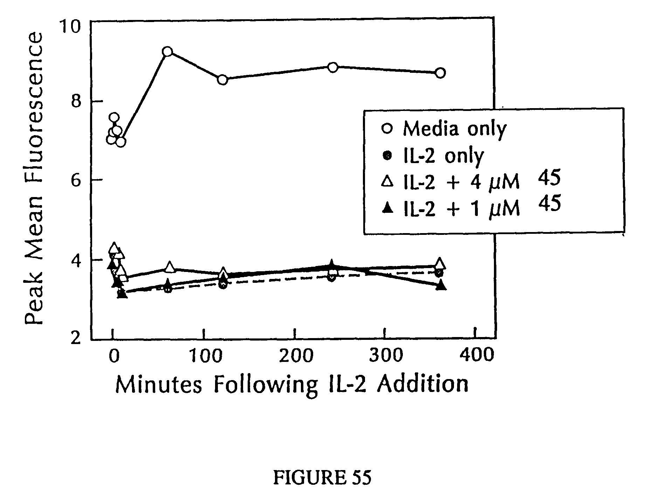

In figure 55, inventive compound no. 45 did not inhibit IL-2 receptor beta (p70) subunit internalization and cell surface down-regulation following IL-2 stimulation. In this assay protocol, CT-6.1 cells were starved overnight and stimulated with 20 ng/ml IL-2. At various times following IL-2 stimulation, cells were rapidly suspended in ice cold PBS and stained using a fluoresceinated monoclonal recognizing the beta subunit of the IL-2 receptor and cell surface expression analyzed by flow cytometry. Figure 55 is a plot of mean fluorescence versus time (in minutes) following IL-2 addition, illustrating that IL-2r beta subunit was rapidly internalized following IL-2 addition with cell surface expression remaining low for 6 hours. Inventive compound no. 45 did not inhibit this receptor internalization activity.

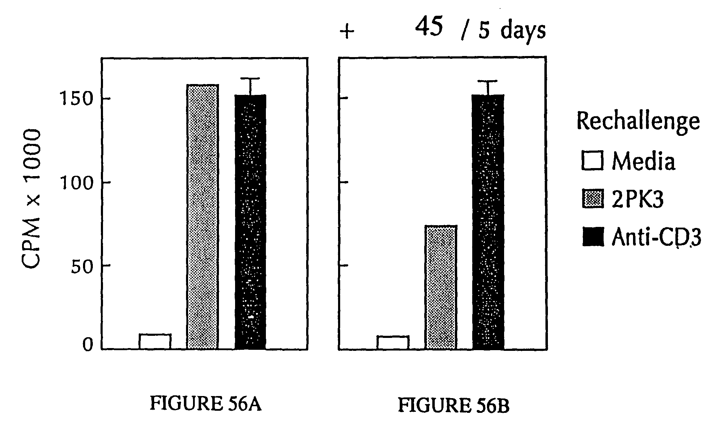

As shown in figures 56A and 56B, inventive compound no. 45 also induces antigen specific T-cell anergy. Procedurally, murine splenocytes were stimulated with an alloantigen target cell line 2PK3 in the presence or absence of compound no. 45 (1 µM; approximate IC₅₀). Following 5 days in culture, the cells were washed, re-cultured and restimulated with the original priming antigen and anti-CD3 monoclonal antibody. The secondary response to the priming antigen was inhibited if the cells were cultured with inventive compound no. 45 during the five day primary culture. The polyclonal T-cell response to anti-CD3 was not affected, indicating that the inventive compound was not cytotoxic during the 5 day priming period. Although cells pretreated with inventive compound no. 45 could respond normally to polyclonal stimulation, those that were challenged with the alloantigen could not. Therefore, compound no. 45 induced a specific state of unresponsiveness or anergy to the alloantigen.

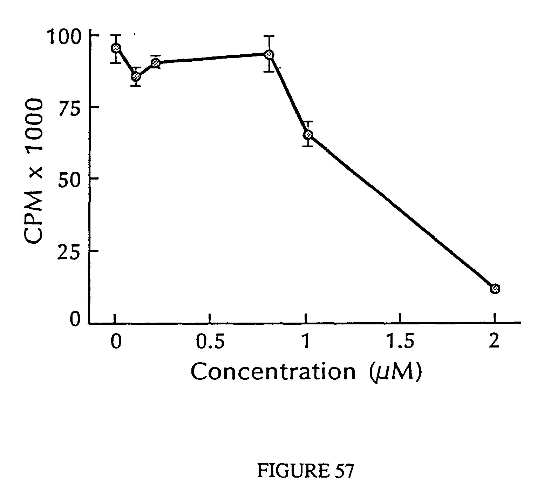

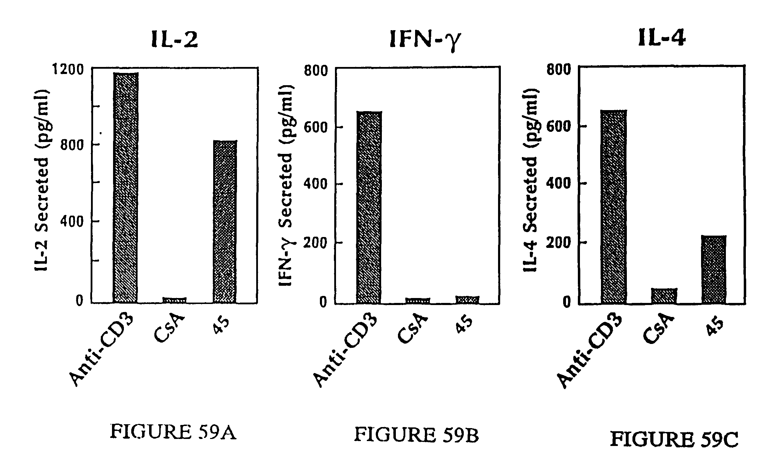

In figure 57, results shown illustrate that inventive compound no. 45 inhibits B cell proliferation. Murine splenocytes were stimulated with anti-IgM antibodies (10 µg/ml) and murine IL-4 (12.5 ng/ml). Cells were pulsed with ³HTdR at 44 hours, harvested 4 hours later, and the incorporation of ³H-TdR was determined by liquid scintillation. As shown in figure 59, the inventive compound inhibited proliferation, with an IC₅₀ of 1.2 µM.

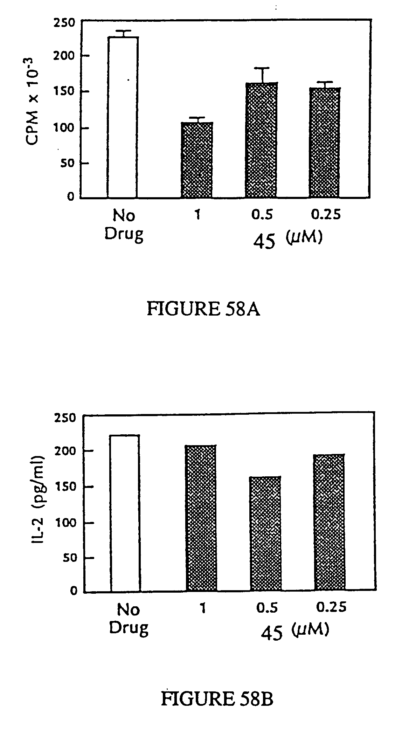

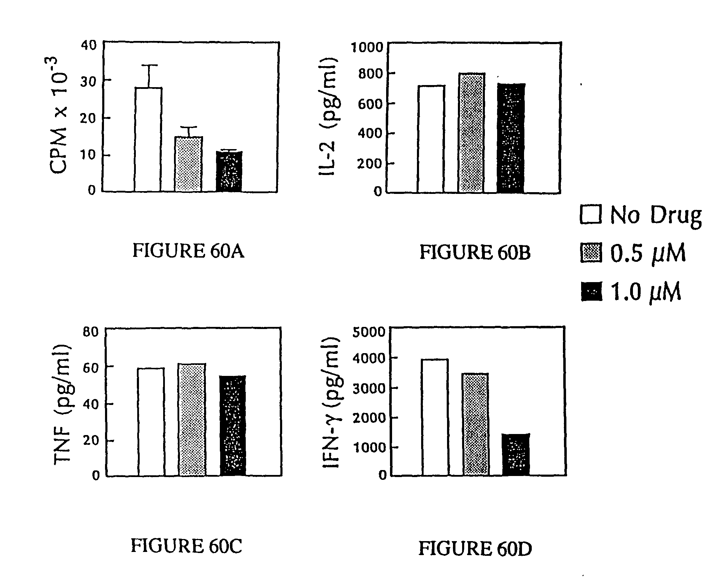

Figures 58A and 58B illustrate that inventive compound no. 45 did not inhibit CD28-mediated IL-2 release. Murine splenocytes were stimulated with a mixture of anti-CD3 and anti-CD28 monoclonal antibodies. Inventive compound no. 45 inhibited T cell proliferation in this system (figure 60A) but did not inhibit the CD28-mediated release of IL-2 (figure 60B).

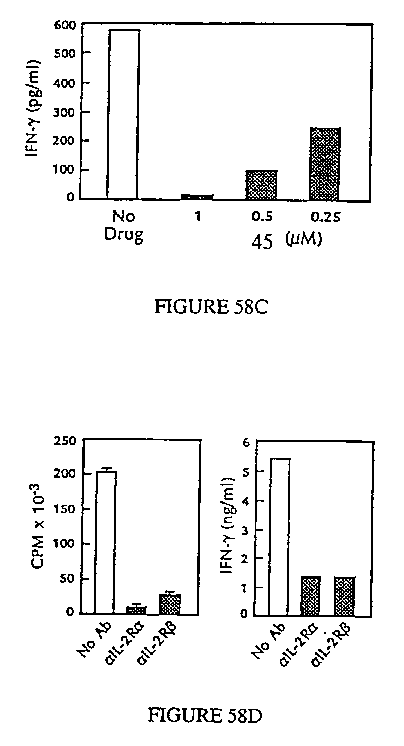

Figures 58C and 58D illustrate that compound no. 45 inhibited IFN-γ release by blocking IL-2 signaling. Procedurally, murine splenocytes were stimulated with a mixture of anti-CD3/CD28 antibodies. Inventive compound no. 45 inhibited release of IFN-γ (figure 60C). Addition of an anti-IL-2 receptor alpha subunit antibody and an anti-IL-2 receptor beta subunit antibody to the cultures inhibited both proliferation and the release of IFN-γ (figure 60D). This demonstrates that the inhibition of IFN-γ release after stimulation with anti-CD28/CD3 was due to blocking an IL-2 signal.

Figures 59A, 59B and 59C report assay results investigating the effect of inventive compound no. 45 on cytokine release from anti-CD3-stimulated mouse splenocytes. Cells were stimulated with anti-CD3 overnight with or without either CsA (50 nM) or compound no. 45 (1 µM). Concentrations chosen were approximately equal to IC₅₀ values. The supernatants were harvested and assayed for cytokine levels using commercial ELISAs for proliferation. Anti-CD3 stimulated release of IL-2, IFN-γ and IL-4 (background levels for media control were negligible). As shown in figure 59A, compound no. 45 did not significantly inhibit IL-2 release, in contrast to CsA. However, compound no. 45 drastically inhibited IFN-γ release similar to CsA (fig. 59B), predicting that the inventive compound had a differential effect on Th-2 cell cytokine release, which secrete both IL-2 and IFN-γ. Compound no. 45 only partially inhibits IL-4 release, which is produced by Th-2 cells (fig. 59C).

In an assay related to the MTLC assay, cytokines levels were measured by ELISA from the supernatants of an MTLC culture. Figure 60A shows inhibition of overall proliferation with compound no. 45. Figures 60B and 60C illustrate that compound no. 45 did not inhibit either IL-2 or TNFα release from the MTLC. In contrast, figure 60D shows that inventive compound no. 45 inhibited IFN-γ release.

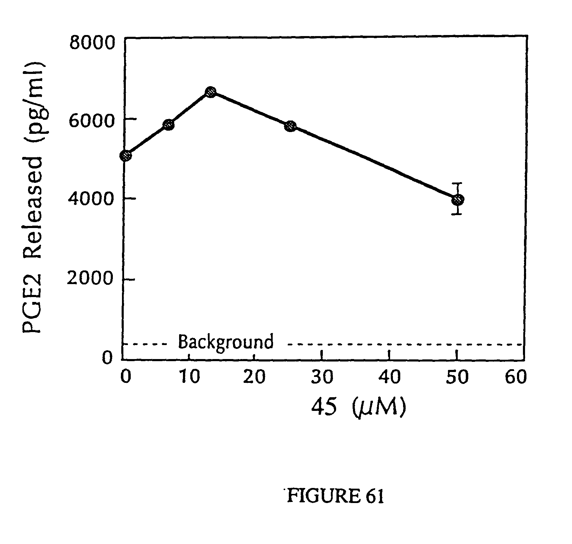

Figure 61 illustrates that inventive compound no. 45 did not have an effect on prostaglandin E2 release from IL-1α-stimulated human foreskin fibroblasts, HS68. Cells were stimulated with 100 pg/ml IL-1α. Inventive compound no. 45 was added 1 hour prior to stimulation. Supernatants were harvested 24 hours later and levels of PGE₂ were analyzed by commercial immunoassay. The results are reported in figure 61 and confirm no effect on PGE₂ release.

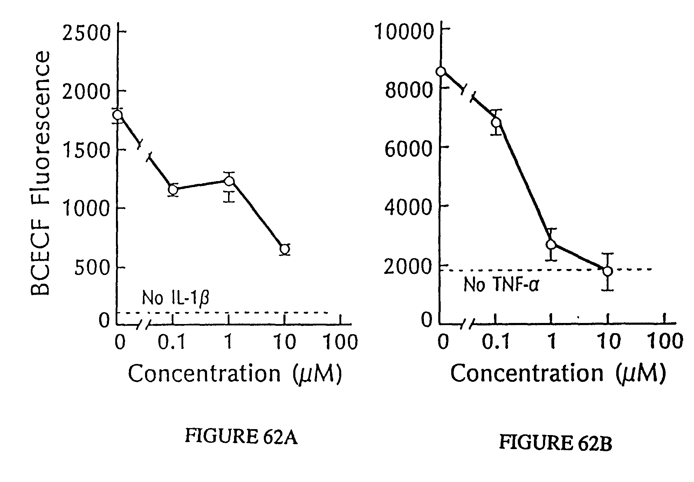

Inventive compound no. 45 inhibited adhesion of monocytic leukemia cells (THP-1) to IL-1β or TNFα-activated HUVEC. HUVEC were stimulated with 20 ng/ml IL-1β or 15 ng/ml TNFα for 6 hours. Fluorescence labeled (BCECF-AM) THP-1 cells were allowed to adhere to the HUVEC for 20 minutes, washed once and the amount of fluorescence remaining analyzed on a fluorescence plate reader. The results, reported in figures 62A and 62B (TNFα or IL-1β, respectively), show inhibition of THP-1 adhesion to HUVEC.

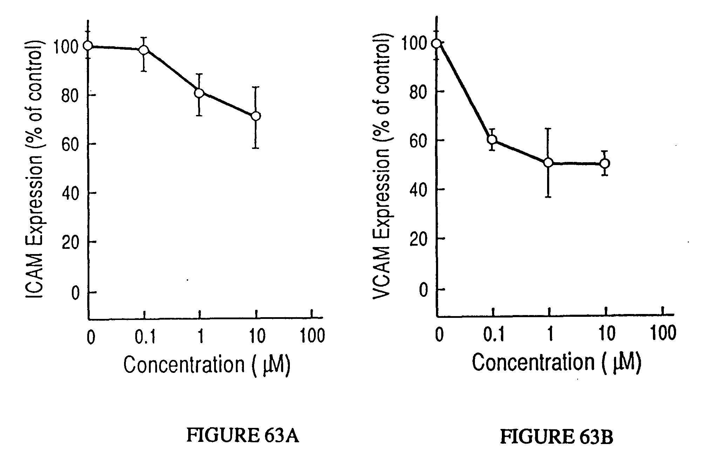

Assay results reported in figures 63A and 63B, illustrate that inventive compound no. 45 inhibited adhesion receptor expression on HUVEC. HUVEC were stimulated with TNFα (20 ng/ml) for 5 hours and stained using fluorescent antibodies to ICAM-1 or VCAM-1. Cell surface expression was analyzed by flow cytometry. All values were normalized to the peak mean fluorescence of the positive control (=100%). Average levels of induction using TNFα- stimulation were 20-fold higher for ICAM-1 and over 50-fold higher for VCAM-1 from that of nonstimulated controls, as shown in figures 63 and 63B.

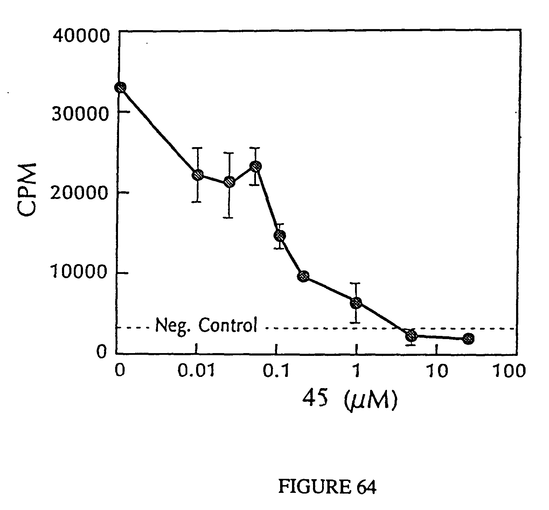

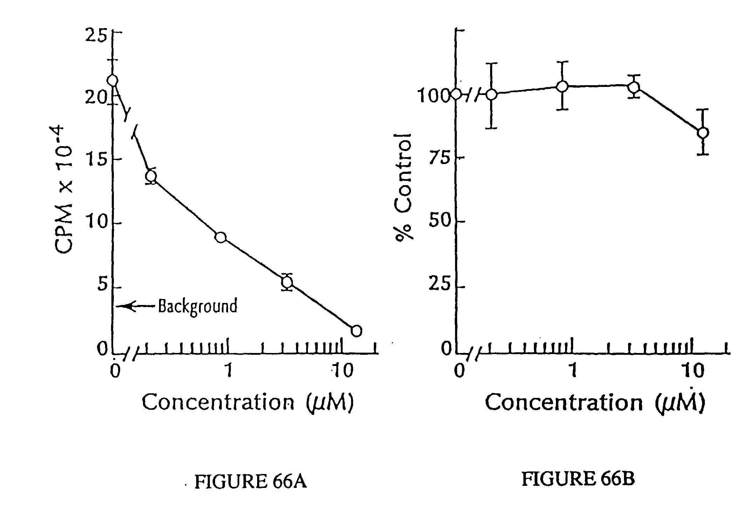

Figure 66 reports that inventive compound no. 45 inhibited PDGF-induced murine BALB/3T3 proliferation. Procedurally, cells were rested overnight in 0.2% serum and stimulated with 25 ng/ml PDGF. Compound no. 45 was added 1 hour prior to stimulation. Cells were pulsed with ³H-TdR 24 hours later and harvested for scintillation counting 4 hours later. As shown in figure 64, compound no 45 inhibited proliferation in this assay at concentrations less than 30 µM.

Collectively, the foregoing data substantially support the conclusion that compound no. 45, representative of the inventive compounds disclosed herein, is a good immunosuppressive and anti-inflammatory therapeutic, effecting many of the cascading events characteristic of diseases such as rheumatoid arthritis.

### Example 46

This example illustrates *in vitro* evidence predicting that the inventive compounds, as represented by a specie of the disclosed genus (compound no. 58), are effective therapies for atherosclerosis and restinosis.

The pathologic mechanisms leading to restenosis mimmic processes observed in atherosclerosis, yet at an accelerated pace. Arterial narrowing resulting from atherosclerosis is the end result of a complex process involving injury to blood vessels, initiated by subintimal accumulation of lipids triggering a cascade of cellular and cytokine mediated events. Such events include accumulation of platelets and inflammatory cells at the site of injury. Cytokines are released which stimulate smooth muscle cell proliferation. The arterial narrowing observed is predominantly due to the localized accumulation of macrophages and proliferation of smooth muscle cells within the arterial wall. Unlike the slow chronic narrowing seen in atherosclerosis, the disease process is greatly accelerated after arterial injury caused by angioplasty or vascular surgery. In the assay data which follows, the cumulative reported results illustrate that inventive compound no. 58, representative of the inventive compounds, inhibits many of the cellular and cytokine-mediated events that lead to atherosclerosis and restenosis.

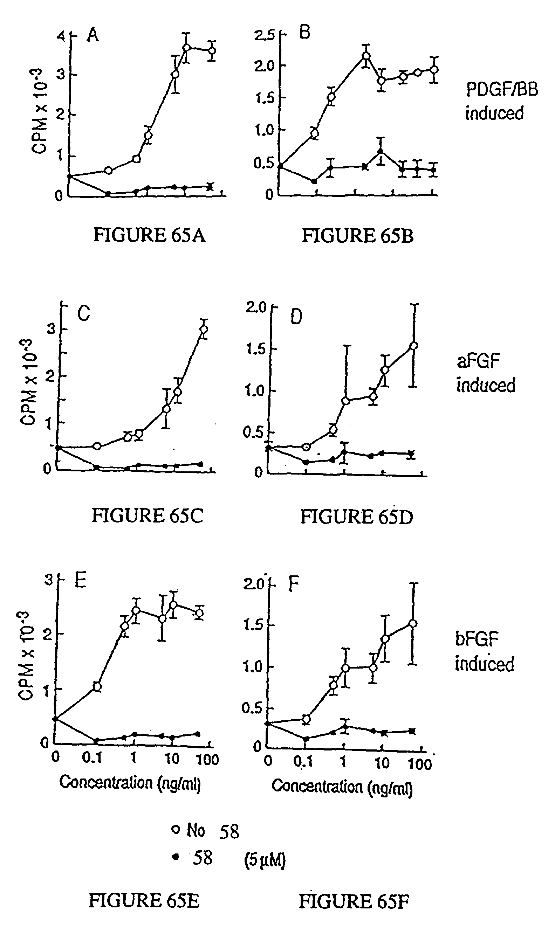

Figures 65A-65F illustrate inhibition of proliferation in either human aortic or pulmonary smooth muscle cells (SMC) by inventive compound no. 58. Procedurally, cells were cultured in 0.5% serum containing medium 24 hours prior to stimulation with various concentrations of either PDGF (figs. 65A and 65B), acidic FGF (figs. 65C and 65D) or basic FGF (figs. 65E and 65F). Cells were stimulated for 24 hours prior to labeling with ³H-TdR for 4 hours and harvested and counted by scintillation. Compound no. 58 was added 1 hour prior to stimulation at a concentration of 5 µM. Even at points of maximum stimulation of the growth factors, compound no. 58 completely inhibited cellular proliferation in both aortic and pulmonary SMC. In addition cell number was assessed and those treated with compound no. 58 showed no increase in cell number following growth factor stimulation.

Figures 66A and 66B are a dose response and cytoxicity curves, respectively, for inhibition of proliferation in Balb/3T3 cells by inventive compound no. 58. In the assay protocol, cells were starved overnight in 0.5% serum containing medium, followed by stimulation with 20 ng/ml PDGF for 24 hours. The cells were labeled with ³H-TdR for 4 hours and harvested and counted by scintillation. The IC₅₀ for inhibition in this cell line was approximately 200-500 nM, the results being reported in figure 66A. Figure 66B illustrates that inventive compound no. 58 was not cytotoxic to Balb/3T3 cells.

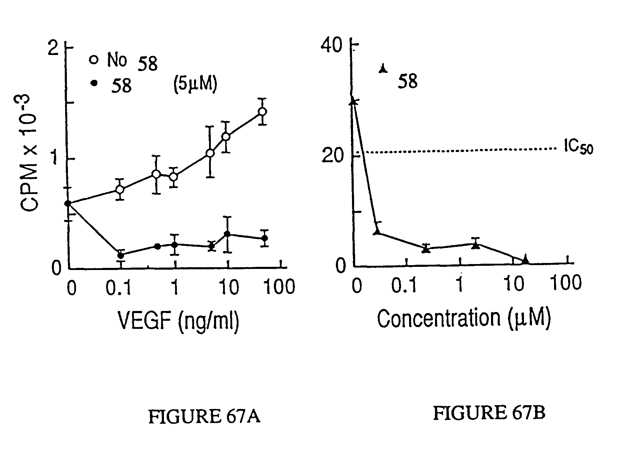

As illustrated in figures 67A and 67B, inventive compound no. 58 inhibited VEGF-induced proliferation in HUVEC and EGF-induced proliferation in Swiss/3T3 cells, respectively. Procedurally, HUVEC were placed in 0.5% serum containing medium prior to stimulation with various concentrations of VEGF, with or without inventive compound no. 58 (5 µM). Twenty four hours later the cells were pulsed with ³H-TdR and 4 hours later harvested and counted by scintillation. The IC₅₀ for inhibition was approximately 50-100 nM. Swiss 3T3 cells were stimulated with 20 ng/ml EGF and 24 hours later harvested and counted by scintillation. The IC₅₀ for inhibition was approximately 20 nM.

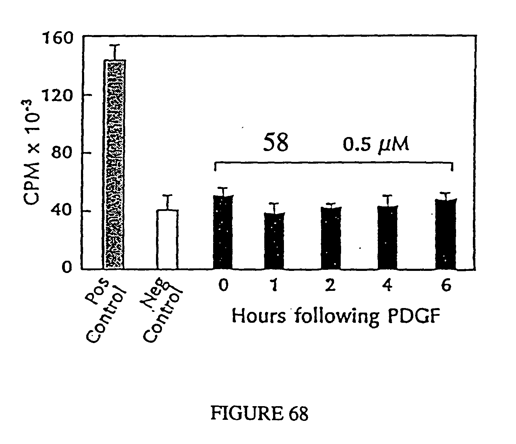

Figure 68 illustrates an effect of the delayed addition of inventive compound no. 58 to Balb/3T3 proliferation in response to PDGF-BB. Cells were stimulated with PDGF (20 ng/ml) and inventive compound no. 58 added either simultaneous with PDGF (0) or at various times after addition of PDGF, up to 6 hours later. At 24 hours, the cells were pulsed with ³H-TdR and counted by scintillation four hours later. Futher experimentation has shown nearly complete inhibition in proliferation, even if compound no. 58 was added as late as 20 hours after PDGF.

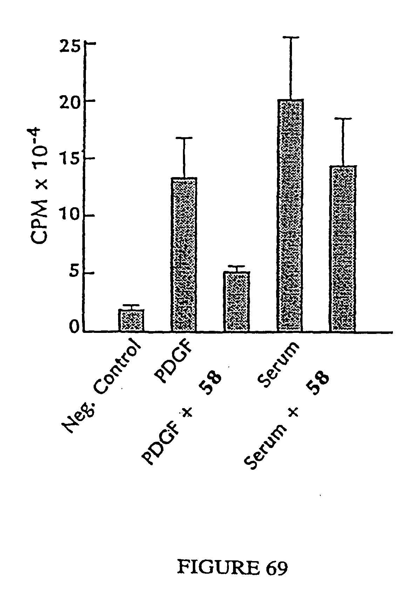

Figure 69 illustrates that inventive compound no. 58 inhibited PDGF- induced proliferation in Balb/3T3 cells to a greater extent than serum-induced proliferation. In the assay protocol, cells were serum starved for 24 hours before adding either 20 ng/ml PDGF or 10% serum. The cells were pulsed with ³HTdR 24 hours later. Inventive compound no. 58 did not have a significant effect on serum-induced Balb/3T3 proliferation.

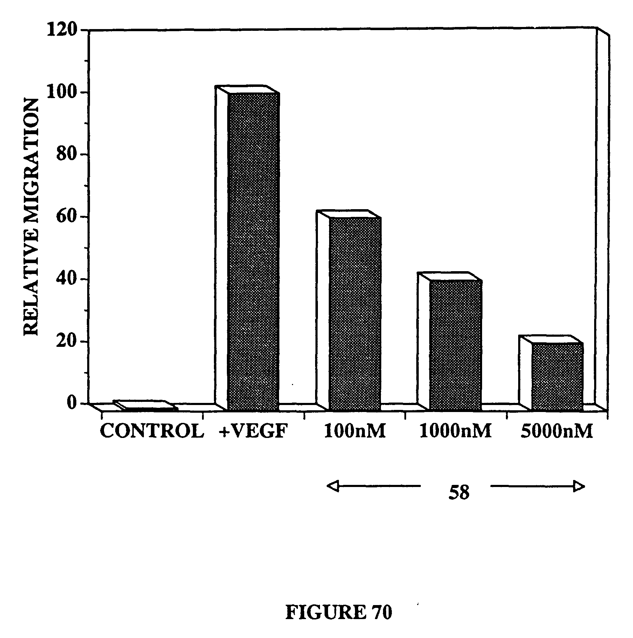

As shown in figure 70, endothelial cell migration is inhibited by inventive compound no. 58. HUVEC were placed in a Matrigel invasion assay system (Becton Dickinson) and VEGF-induced migration assessed with or without varying concentrations of inventive compound no. 58. Matrigel migration of HUVEC to 50 ng/ml VEGF was inhibited in a dose dependent manner, with an IC₅₀ of approximately 100-200 nM.

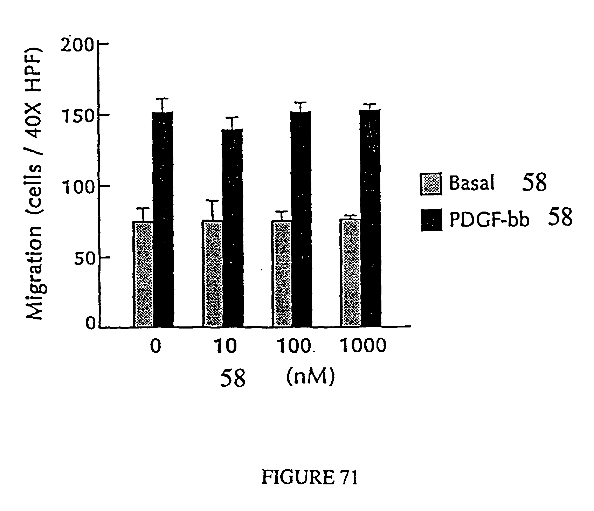

Figure 71 illustrates that inventive compound no. 58 did not inhibit chemotaxis of human smooth muscle cells (SMC) to PDGF. In the assay, cells were seeded in a Boyden chamber with or without various concentrations of inventive compound no. 58. Eight hours later, the number of cells that had migrated were scored visually. Inventive compound no. 58 had no effect on PDGF-directed SMC chemotaxis.

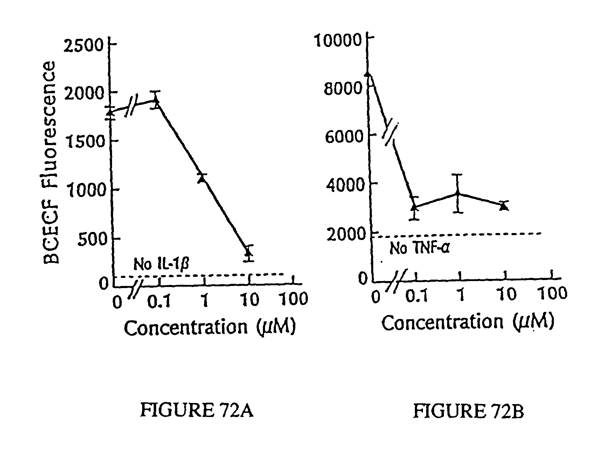

Figures 72A and 72B illustrate that inventive compound no. 58 inhibited THP-1 cell adhesion to either TNF or IL-1-stimulated HUVEC, respectively. Procedurally, HUVEC were stimulated with TNF or IL-1 for 8 hours and THP-1 cells added for 20 minutes. The HUVEC were then washed and analyzed for adherence. Inventive compound no. 58 inhibited adhesion of THP-1 cells to HUVEC with an IC₅₀ of approximately 1 µM for both TNF and IL-1.

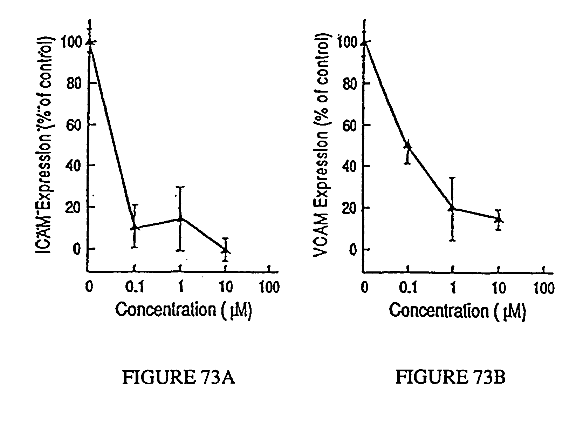

Figures 73A and 73B illustrate inhibition by inventive compound no. 58 of VCAM or ICAM expression, respectively, in HUVEC activated by TNF. Cells were stimulated for 8 hours in the presence or absence of inventive compounds and stained with fluorescent antibodies to VCAM or ICAM and analyzed by flow cytometry.

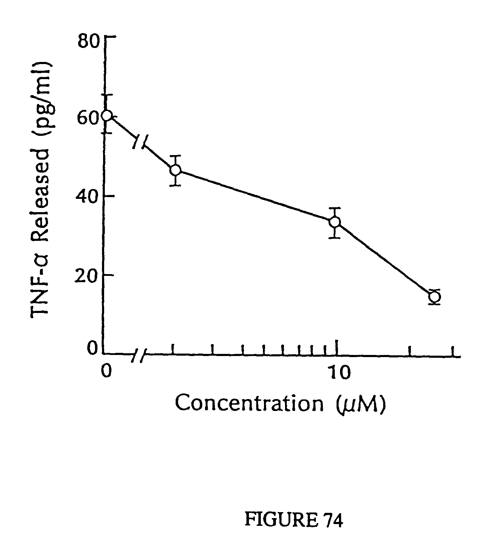

Figure 74 illustrates that inventive compound no. 58 inhibited TNF release. 58 using a human whole blood *ex vivo* assay. In this assay, human whole blood was stimulated with mouse TNFα and human IL-1α with or without various concentrations of inventive compound no. 58. After 6 hours, the plasma was analyzed for human TNFα levels using a commercial ELISA. Results are reported in figure 74.

## Claims

1. A compound having the formula:
(X)j - (core moiety),
wherein j is an integer from one to three, the core moiety is a cyclic or heterocyclic moiety having from one to three, five- to six-membered ring structures, and X is a racemic mixture, R or S enantiomer, solvate, hydrate, or salt of: 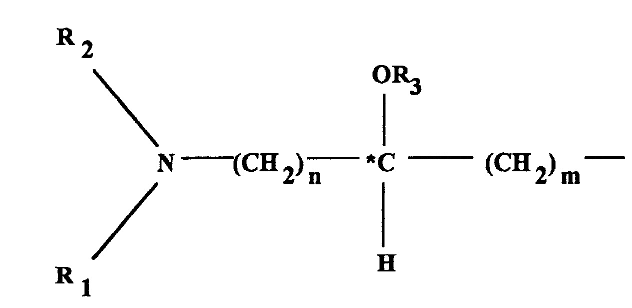 wherein *C is a chiral carbon atom; n is equal to one; the carbon atom of (CH₂)ₙ may be substituted by a keto or hydroxy group; m is an integer from eight to fourteen; independently, R₁ and R₂ are hydrogen, a straight or branched chain alkane or alkene of up to twelve carbon atoms in length, or -(CH₂)_{w}R₅, w being an integer from two to fourteen and R₅ being a mono-, di- or tri-substituted or unsubstituted aryl group, substituents on R₅ being selected from the group consisting of hydroxy, chloro, fluoro, bromo, or C₁₋₆ alkoxy; or jointly, R₁ and R₂ form a heterocyclic group having from four to eight carbon atoms; and R₃ is hydrogen or C₁₋₃.

2. The compound of claim 1, wherein the core moiety has a planar structure.

3. The compound of claim 1, wherein the core moiety is a member selected from the group consisting: barbituric acid; benzamide; benzene; biphenyl; cyclohexanedione; cyclopentanedione; delta-lactam; flutarimide; glutarimide; homophthalimide; imidazole amide; isocarbostyrile; lumazine; napthlalene; pteridine; pthalimide; piperidine; pyridine; pyrimidine; pyrrole amide; quinazolinedione; quinazolinone; quinolone; resorcinol; stilbene; succinimide; theobromine; thymine; triazine and tricyclododecane.

4. The compound of claim 1, wherein the core moiety is selected from the group consisting of glutarimide, methylthymine, methyluracil, thymine, theobromine, uracil and xanthine.

5. The compound of claim 1, wherein the core moiety is xanthine, X is bonded to an N₁ xanthine nitrogen and N₃ and N₇ xanthine nitrogens are independently substituted by a member selected from the group consisting of hydrogen, methyl, fluoro, chloro and amino.

6. The compound of claim 1, wherein the core moiety is selected from the group consisting of 1,3-cyclohexanedione, 1,3-cyclopentanedione; 1,3-dihydroxynaphthalene; 1-methyllumazine; methylbarbituric acid; 3,3-dimethylflutarimide; 2-hydroxypyridine; methyldihydroxypyrazolopyrimidine; methylpyrrolopyrimidine; 2-pyrrole amides; 3-pyrrole amides; 1,2,3,4-tetrahydroisoquinolone; 1-methyl-2,4(1H,3H)-quinazolinedione (1-methylbenzoyleneurea); quinazolin-4(3H)-one; alkyl-substituted (C₁₋₆) thymine; methylthymine; alkyl-substituted (C₁₋₆) uracil; 6-aminouracil; 1-methyl-5,6-dihydrouracil; 1-methyluracil; 1,7-dimethylxanthine, 3,7-dimethylxanthine; 3-methylxanthine; 3-methyl-7-methylpivaloylxanthine; 8-amino-3-methylxanthine; and 7-methylhypoxanthine.

7. The compound of claim 1, the compound being selected from the following compounds:

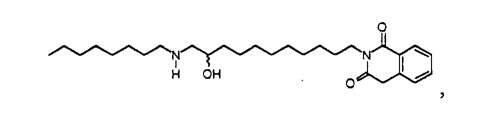

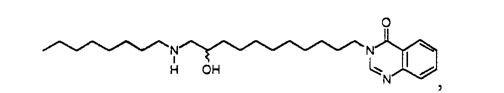

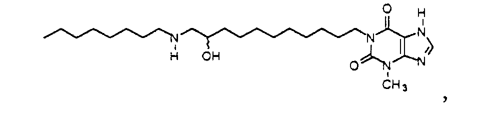

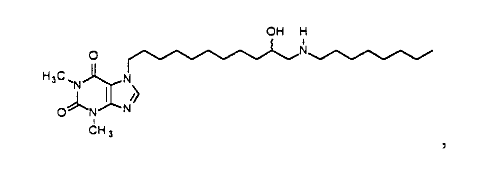

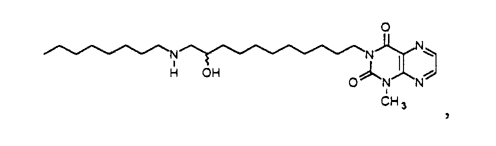

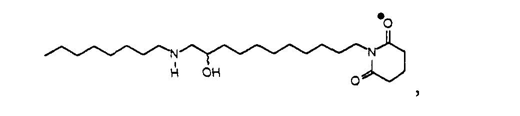

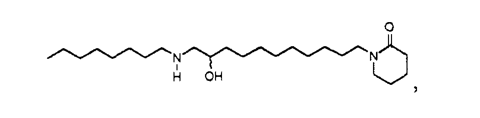

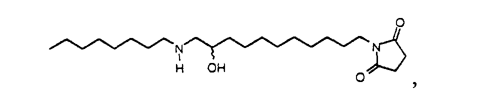

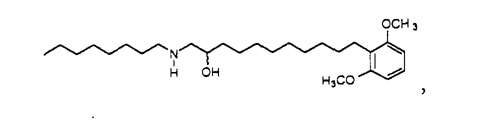

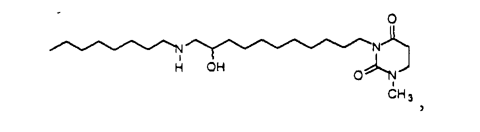

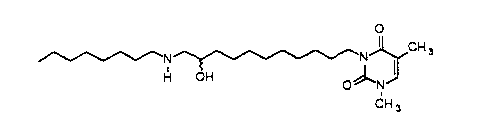

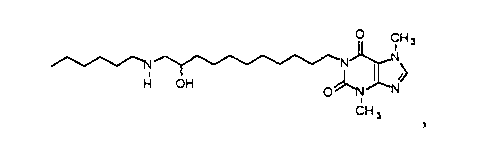

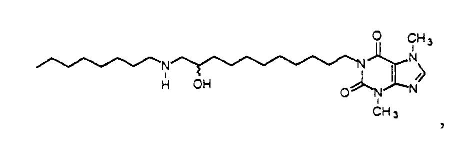

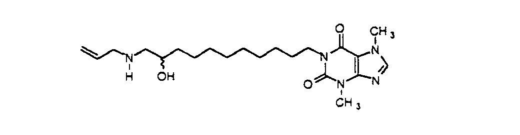

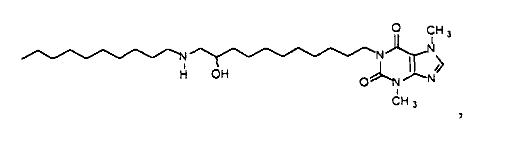

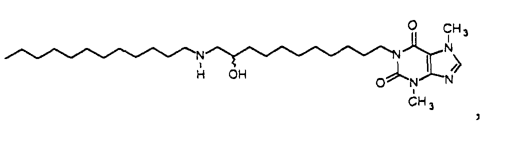

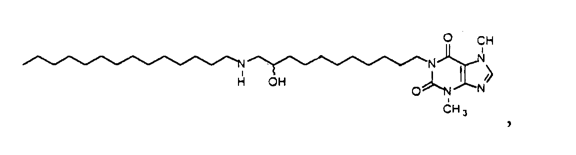

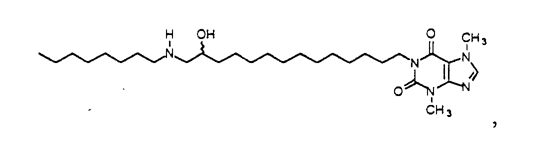

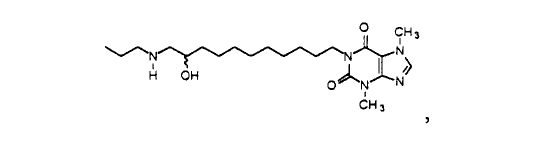

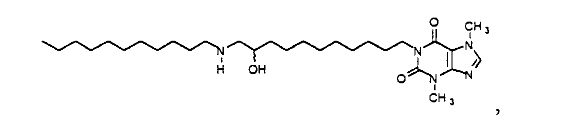

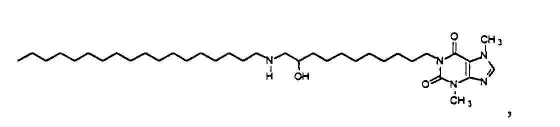

8. A compound being selected from the following compounds:

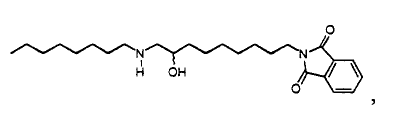

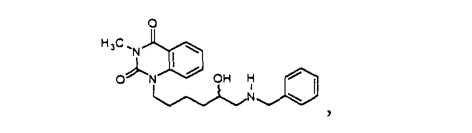

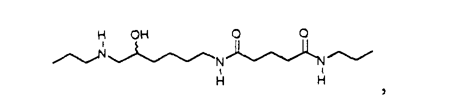

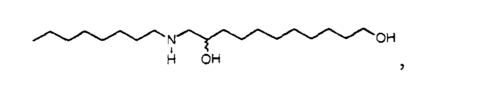

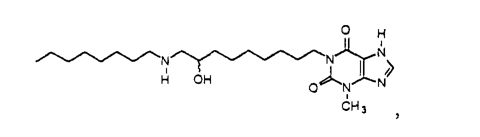

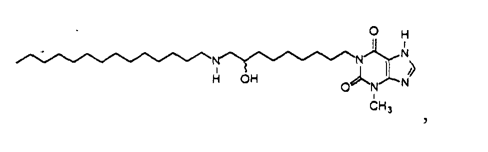

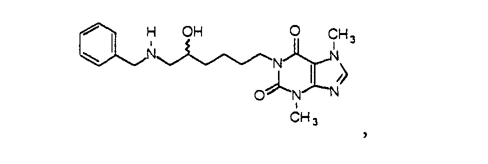

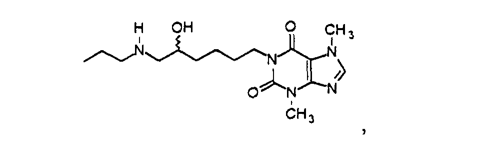

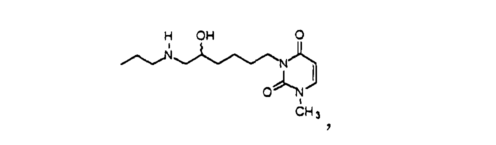

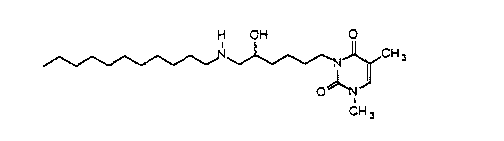

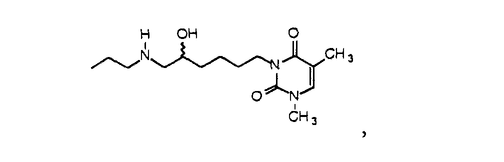

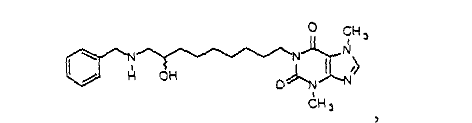

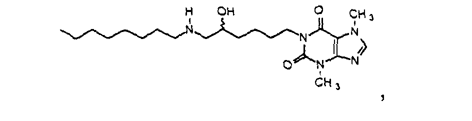

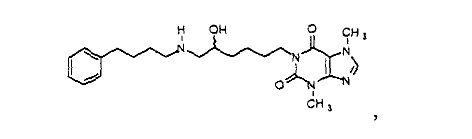

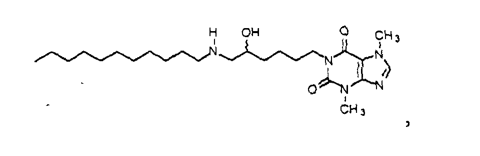

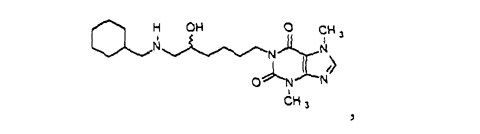

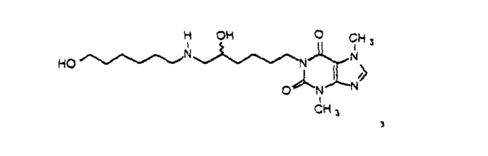

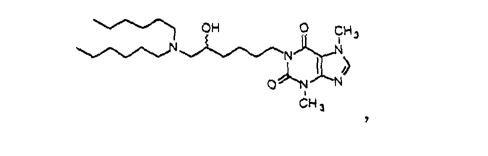

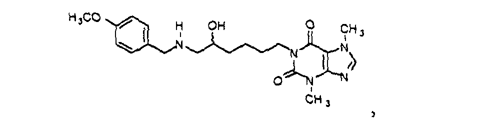

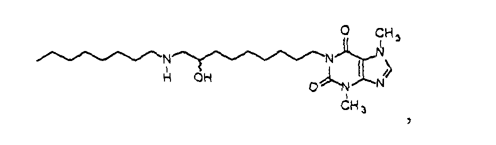

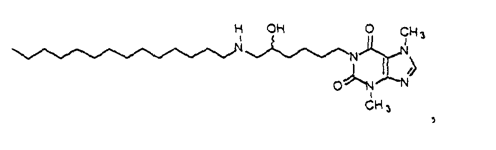

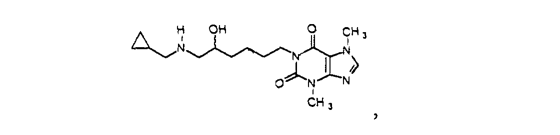

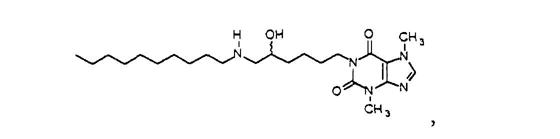

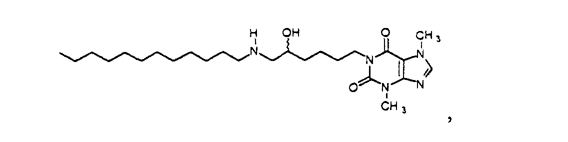

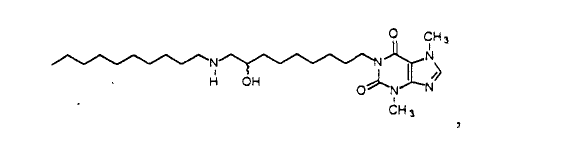

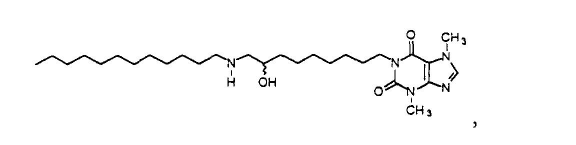

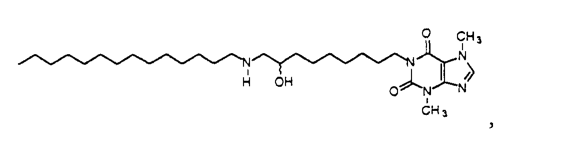

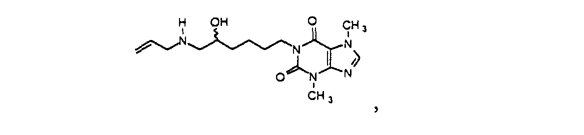

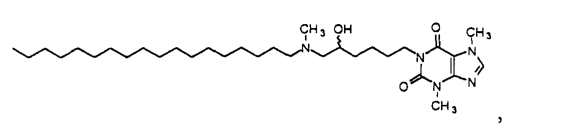

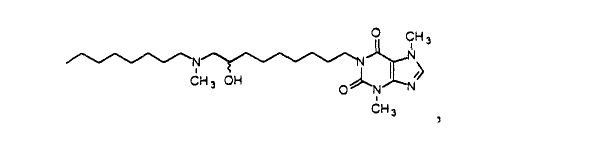

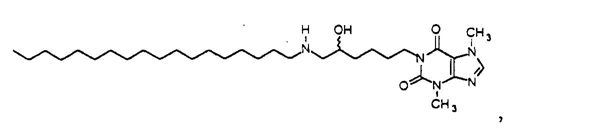

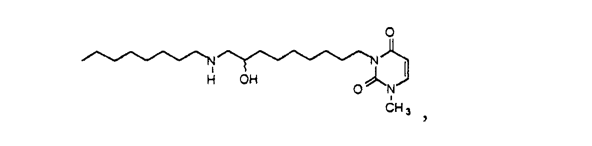

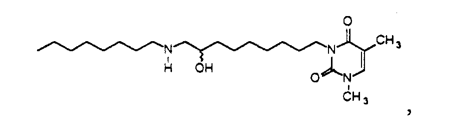

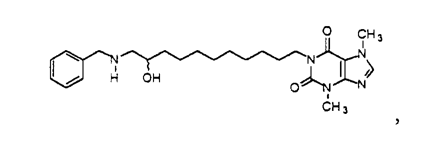

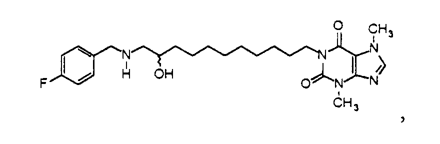

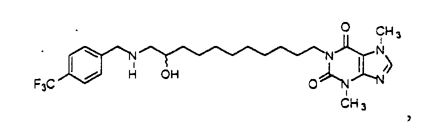

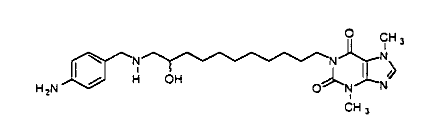

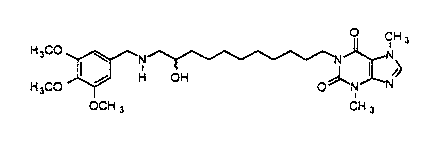

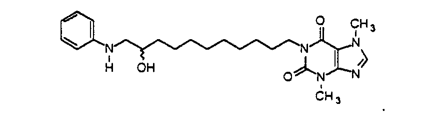

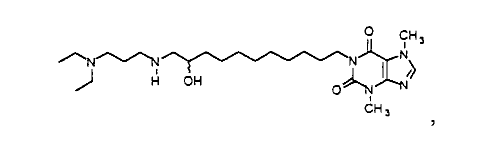

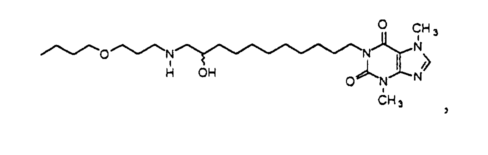

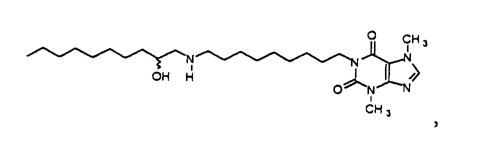

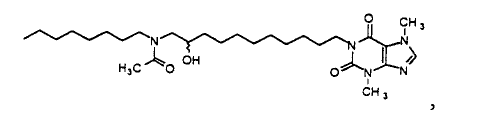

9. A pharmaceutical composition comprising a compound of claim 1 or 8 or its pharmaceutically acceptable salt and a pharmaceutically acceptable excipient, the pharmaceutical composition being formulated for oral, parenteral, *ex vivo* or topical administration to a patient.

10. The composition of claim 9, wherein an oral dose of compound is from about 50 mg to about 1500 mg, twice or three times daily, a parenteral dose is from about 1.0 g to about 5.0 g administered (i.v., i.p., i.m., or s.c.) over a course of 24 hours, a topical formulation is from about 1% to about 4% concentration by weight, and the *ex vivo* culture concentration is from about 10 mM to about 500 mM.

11. Use of a compound according to claim 1 or 8 or its pharmaceutically acceptable salt for preparing a medicament for treating or preventing an autoimmune disease, wherein the autoimmune disease is selected from insulin-dependent diabetes melitus (IDDM), adult-onset diabetes, atherosclerosis, multiple sclerosis, psoriasis, asthma, periodontal disease, osteoporosis, alcoholic hepatitis, premature labor secondary to uterine infection, autoimmune thryroiditis, Alzheimer's disease, rheumatoid arthritis, glomerular nephritis, inflammatory bowel disease and lupus.

12. Use of a compound according to claim 1 or 8 or its pharmaceutically acceptable salt for preparing a medicament for treating or preventing acute or chronic inflammatory diseases, AIDS and AIDS related complex, alcoholic hepatitis, allergies due to degranulation of mast cells and basophils, angiogenesis, coronary artery disease, hairloss or baldness, HIV-associated dementia, lung hypoxia, lupus, malignancies, myelogenous leukemia, organ or hematopoietic injury in response to cytotoxic therapy, osteoarthritis, restenosis, sleep disorders, septic shock, sepsis syndrome, sceloderma, stroke and transplant rejection.

13. A method for preparing a compound according to claim 1 or 8, the method comprising:
reacting a core-containing compound with a suitable base, solvent and substituted olefin to obtain an intermediate product, the intermediate product having a composite structure of the core moiety and substituted olefin;
converting the intermediate product to a core-containing epoxide in a reaction with organic peracid; and
reacting the core-containing epoxide with a substituted or unsubstituted amine to obtain a compound of claim 1 or 8.

## Patentansprüche

1. Verbindung der Formel
(X)j - (Kemeinheit),
wobei j eine ganze Zahl von eins bis drei ist, die Kerneinheit einen zyklischen oder heterozyklischen Rest mit einer bis drei fünf- bis sechsgliedrigen Ringstrukturen darstellt und X ein racemisches Gemisch, R- oder S-Enantiomer, Solvat, Hydrat oder Salz von 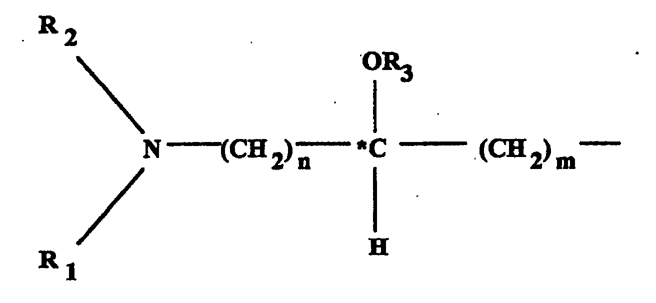 bedeutet, wobei *C ein chirales Kohlenstoffatom bedeutet; n gleich eins ist; das Kohlenstoffatom von (CH₂)ₙ durch eine Keto- oder Hydroxygruppe substituiert sein kann; m eine ganze Zahl von 8 bis 14 ist; R₁ und R₂ unabhängig voneinander ein Wasserstoffatom, ein gerades oder verzweigtes Alkan oder Alken mit einer Länge von bis zu 12 Kohlenstoffatomen oder -(CH₂)_{w}R₅ bedeuten, wobei w eine ganze Zahl von 2 bis 14 ist und R₅ einen mono-, di- oder trisubstituierten oder unsubstituierten Arylrest darstellt, wobei die Substituenten auf R₅ aus der Gruppe ausgewählt sind, die aus einer Hydroxygruppe, einem Chlor-, Fluor-, Bromatom oder einem C₁₋₆-Alkoxyrest besteht; oder wobei R₁ und R₂ zusammen einen heterocyclischen Rest mit 4 bis 8 Kohlenstoffatomen bilden; und R₃ ein Wasserstoffatom oder einen C₁₋₃-Rest bedeutet.

2. Verbindung nach Anspruch 1, wobei die Kerneinheit eine planare Struktur aufweist.

3. Verbindung nach Anspruch 1, wobei die Kerneinheit aus der Gruppe ausgewählt ist, die aus Barbitursäure; Benzamid; Benzol; Biphenyl; Cyclohexandion; Cyclopentandion; Delta-Lactam; Flutarimid; Glutarimid; Homophthalimid; Imidazolamid; Isocarbostyril; Lumazin; Naphthalin; Pteridin; Phthalimid; Piperidin; Pyridin; Pyrimidin; Pyrrolamid; Chinazolindion; Chinazolinon; Chinolon; Resorcin; Stilben; Succinimid; Theobromin; Thymin; Triazin und Tricyclododecan besteht.

4. Verbindung nach Anspruch 1, wobei die Kerneinheit aus der Gruppe ausgewählt ist, die aus Glutarimid, Methylthymin, Methyluracil, Thymin, Theobromin, Uracil und Xanthin besteht.

5. Verbindung nach Anspruch 1, wobei die Kerneinheit Xanthin ist, X an ein N₁-Xanthin-Stickstoffatom gebunden ist und die N₃- und N₇-Xanthin-Stickstoffatome unabhängig voneinander durch ein Mitglied der Gruppe substituiert sind, die aus Wasserstoffatom, Methylgruppe, Fluor-, Chloratom und Aminogruppe besteht.

6. Verbindung nach Anspruch 1, wobei die Kerneinheit aus der Gruppe ausgewählt ist, die aus 1,3-Cyclohexandion; 1,3-Cyclopentandion; 1,3-Dihydroxynaphthalin; 1-Methyllumazin; Methylbarbitursäure; 3,3-Dimethylflutarimid; 2-Hydroxypyridin; Methyldihydroxypyrazolopyrimidin; Methylpyrrolopyrimidin; 2-Pyrrolamiden; 3-Pyrrolamiden; 1,2,3,4-Tetrahydroisochinolon; 1-Methyl-2,4(1H,3H)-chinazolindion-(1-methylbenzoylenharnstoff); Chinazolin-4(3H)-on; Alkyl-substituiertem (C₁₋₆)-Thymin; Methylthymin; Alkyl-substituiertem (C₁₋₆)-Uracil; 6-Aminouracil; 1-Methyl-5,6-dihydrouracil; 1-Methyluracil; 1,7-Dimethylxanthin, 3,7-Dimethylxanthin; 3-Methylxanthin; 3-Methyl-7-methylpivaloylxanthin; 8-Amino-3-methylxanthin; und 7-Methylhypoxanthin besteht.

7. Verbindung nach Anspruch 1, wobei die Verbindung aus den folgenden Verbindungen ausgewählt ist:

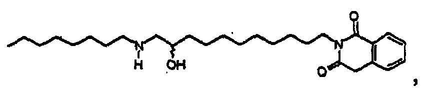

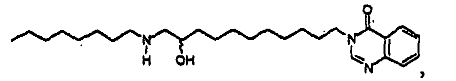

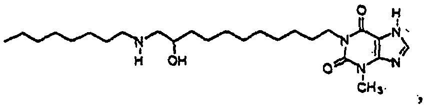

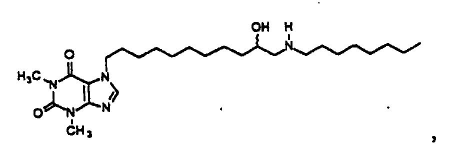

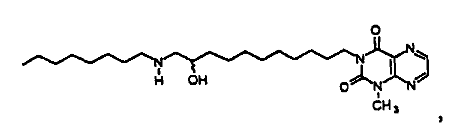

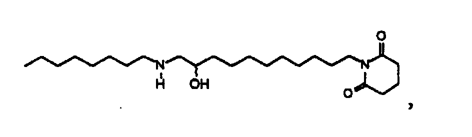

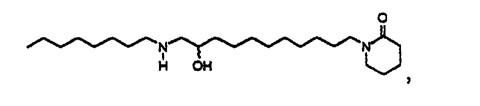

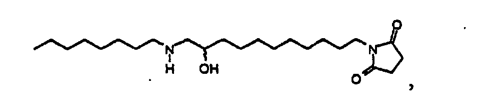

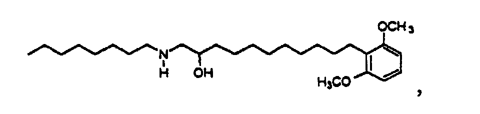

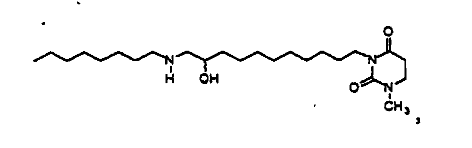

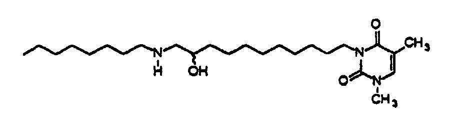

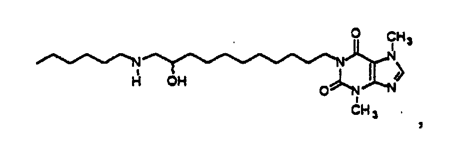

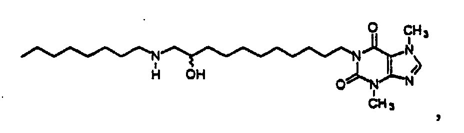

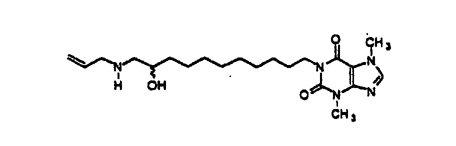

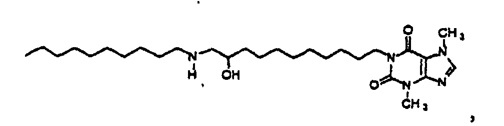

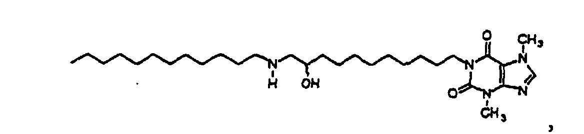

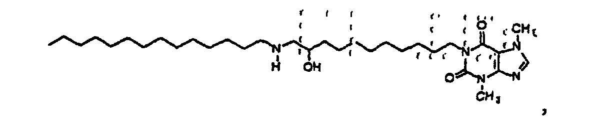

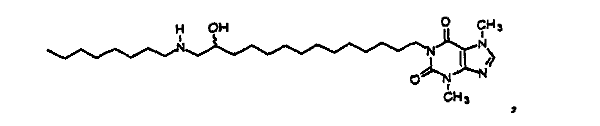

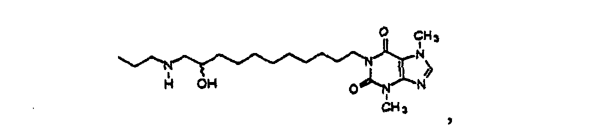

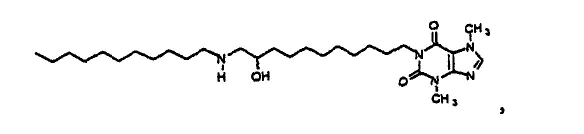

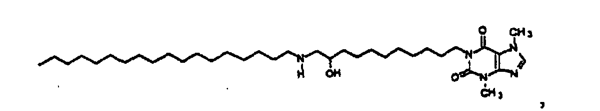

8. Verbindung, die aus den folgenden Verbindungen ausgewählt ist:

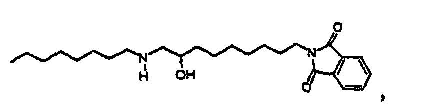

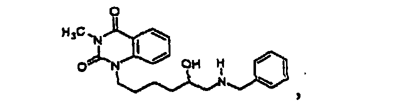

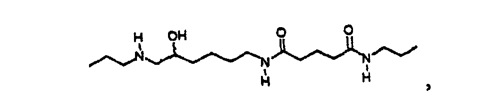

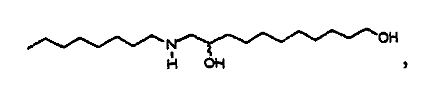

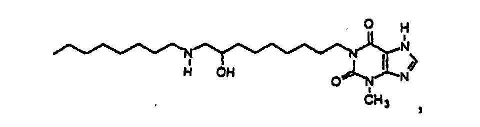

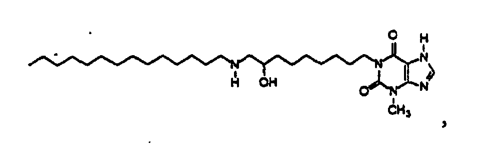

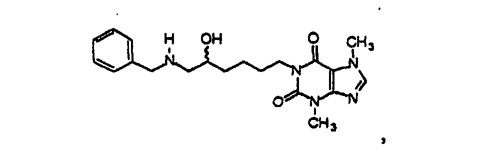

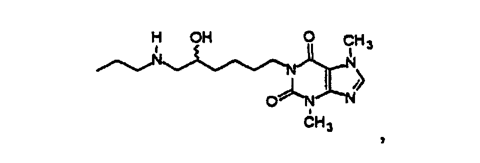

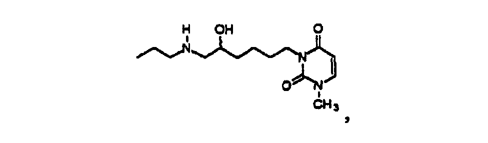

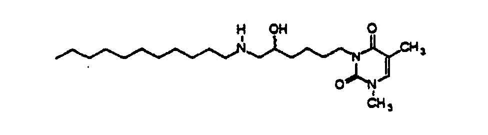

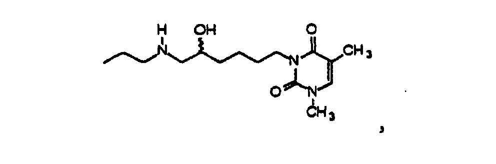

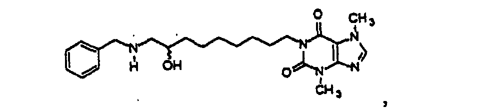

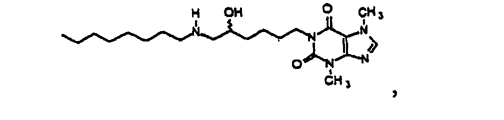

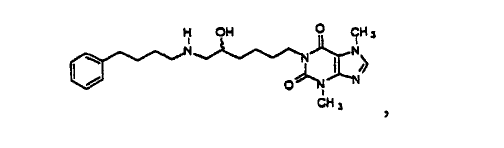

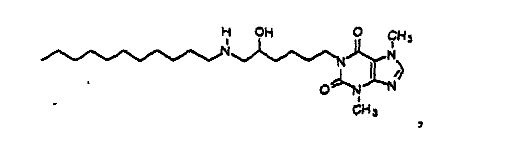

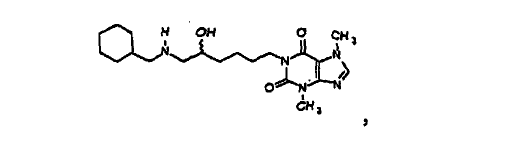

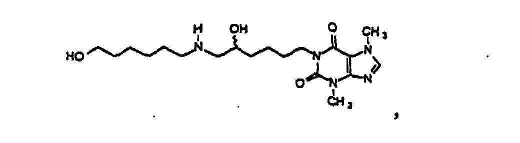

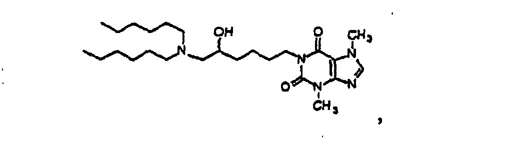

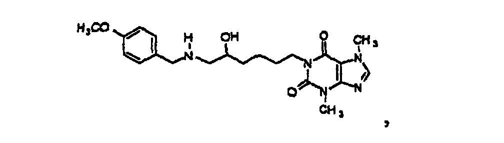

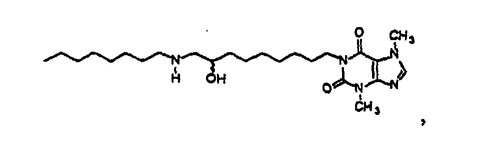

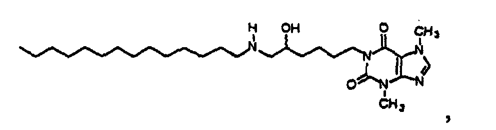

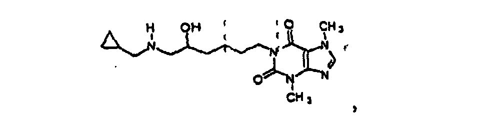

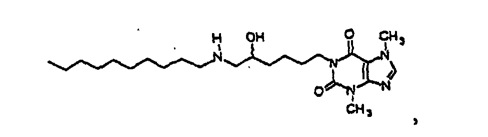

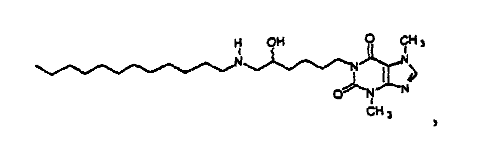

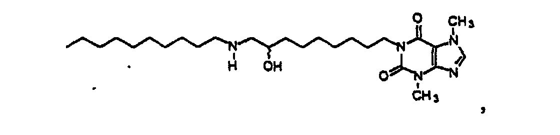

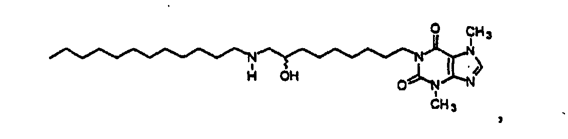

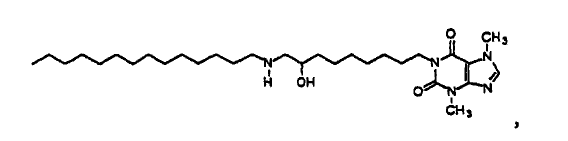

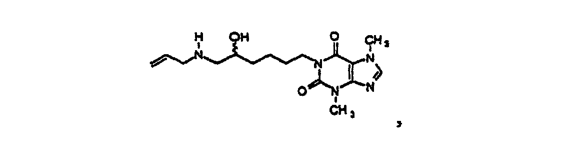

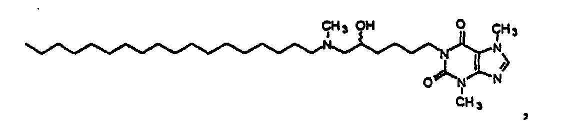

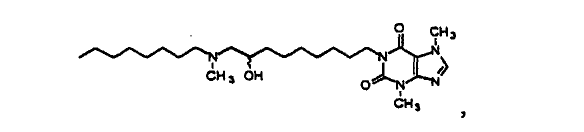

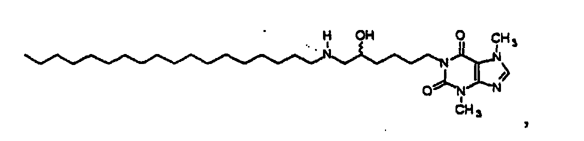

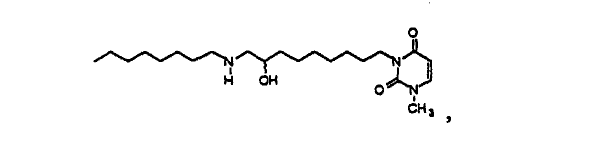

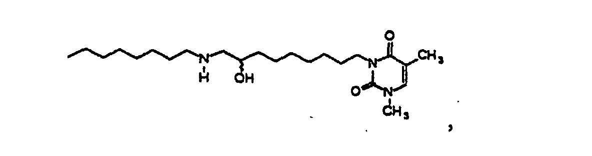

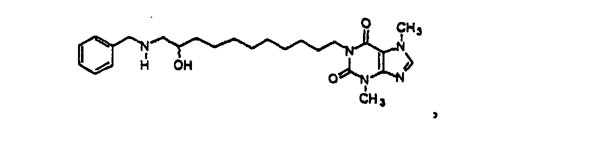

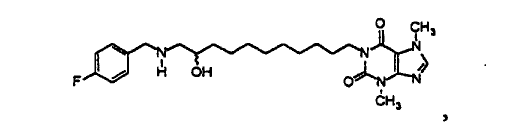

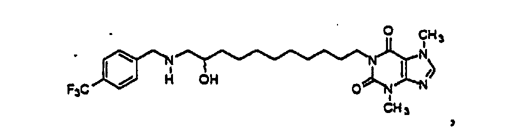

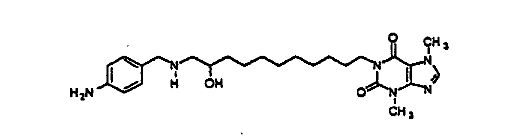

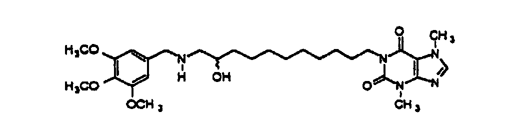

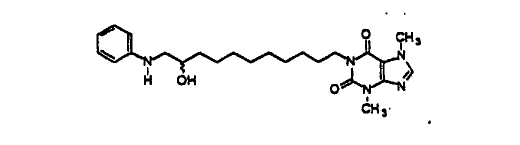

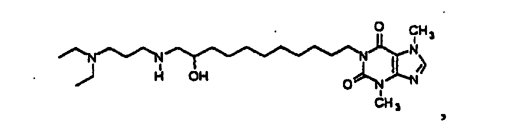

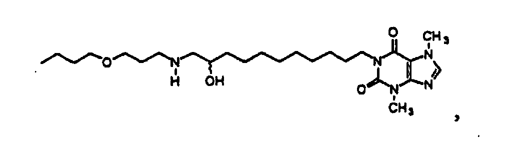

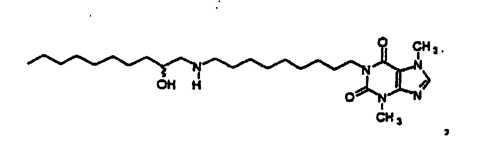

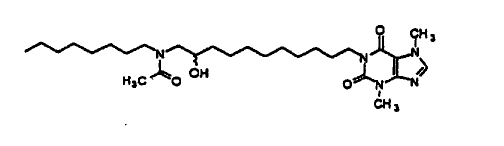

9. Pharmazeutische Zusammensetzung, die eine Verbindung nach Anspruch 1 oder 8 oder ein pharmazeutisch verträgliches Salz davon und ein pharmazeutisch verträgliches Excipiens enthält, wobei die pharmazeutische Zusammensetzung für eine orale, parenterale, *ex vivo-* oder topische Verabreichung an einen Patienten formuliert ist.

10. Zusammensetzung nach Anspruch 9, wobei eine orale Dosis der Verbindung bei etwa 50 mg bis etwa 1500 mg liegt, zwei oder dreimal täglich, eine parenterale Dosis bei etwa 1,0 g bis etwa 5,0 g liegt, die im Laufe von 24 Stunden (i.v., i.p., i.m. oder s.c.) verabreicht wird, eine topische Formulierung mit einer Konzentration von etwa 1 Gew.-% bis etwa 4 Gew.-% vorliegt und die Konzentration einer *ex vivo*-Kultur etwa 10 mM bis etwa 500 mM beträgt.

11. Verwendung einer Verbindung nach Anspruch 1 oder 8 oder eines pharmazeutisch verträglichen Salzes davon zum Herstellen eines Medikaments zum Behandeln oder Verhüten einer Autoimmunkrankheit, wobei die Autoimmunkrankheit ausgewählt ist aus Insulin-abhängigem Diabetes mellitus (IDDM), Erwachsenendiabetes, Arteriosklerose, Multipler Sklerose, Psoriasis, Asthma, Paradontose, Osteoporose, alkoholischer Hepatitis, vorzeitigen Wehen nach einer Uterusinfektion, Autoimmunthyroiditis, Alzheimer-Krankheit, rheumatoider Arthritis, Glomerulonephritis, entzündlicher Darmerkrankung und Lupus.

12. Verwendung einer Verbindung nach Anspruch 1 oder 8 oder eines pharmazeutisch verträglichen Salzes davon zum Herstellen eines Medikaments zum Behandeln oder Verhüten von akuten oder chronischen entzündlichen Krankheiten, AIDS und AIDS-Komplex, alkoholischer Hepatitis, Allergien aufgrund von Degranulation von Mastzellen und Basophilen, Angiogenese, koronaren Herzerkrankung, Haarausfall oder Glatze, HIV-assoziierter Demenz, Lungen-Hypoxie, Lupus, malignen Erkrankungen, myelogener Leukämie, Organschädigung oder Schädigung der Hämatopoese infolge einer zytotoxischen Therapie, Osteoarthritis, Stenoserezidiv, Schlafstörungen, septischem Schock, Sepsis-Syndrom, Sklerodermie, Schlaganfall und Transplantatabstoßung.

13. Verfahren zum Herstellen einer Verbindung nach Anspruch 1 oder 8, wobei das Verfahren folgendes umfasst:
Umsetzen einer Kern-enthaltenden Verbindung mit einer geeigneten Base, einem geeigneten Lösungsmittel und einem geeigneten substituierten Olefin, wodurch ein Zwischenprodukt erhalten wird, wobei das Zwischenprodukt eine zusammengesetzte Struktur aus der Kerneinheit und dem substituierten Olefin aufweist;
Umwandeln des Zwischenprodukts zu einem Kem-enthaltenden Epoxid in einer Reaktion mit einer organischen Persäure; und
Umsetzen des Kern-enthaltenden Epoxids mit einem substituierten oder unsubstituierten Amin, wodurch eine Verbindung nach Anspruch 1 oder 8 erhalten wird.

## Revendications

1. Composé ayant la formule :
(X)j - (groupe fonctionnel central),
dans lequel j est un entier de un à trois, le groupe fonctionnel central est un groupe fonctionnel cyclique ou hétérocyclique ayant de une à trois structures annulaires de cinq à six éléments, et X est un mélange racémique, un énantiomère R ou S, un solvate, un hydrate ou un sel de : 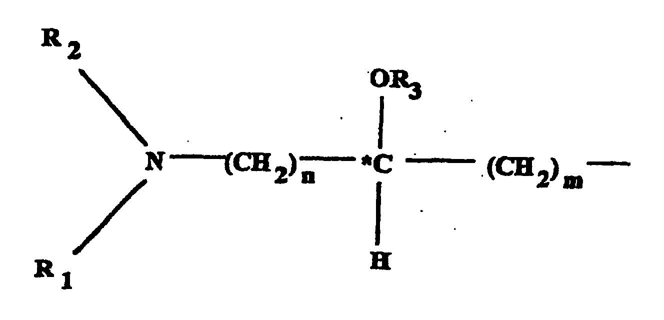 dans lequel *C est un atome de carbone chiral ; n est égal à un ; l'atome de carbone de (CH₂)ₙ peut être substitué par un groupe céto ou hydroxy ; m est un entier de huit à quatorze ; indépendamment, R₁ et R₂ sont de l'hydrogène, une chaîne droite ou ramifiée d'alcane ou d'alcène de longueur allant jusqu'à douze atomes de carbone, ou -(CH₂)_{W}R₅, w étant un entier de deux à quatorze et R₅ étant un groupe aryle mono-, di- ou tri-substitué ou non substitué, les substituants de R₅ étant choisis dans le groupe composé d'hydroxy, de chloro, de fluoro, de bromo ou de C₁₋₆ alkoxy ; ou conjointement, R₁ et R₂ constituent un groupe hétérocyclique ayant de quatre à huit atomes de carbone ; et R₃ est de l'hydrogène ou C₁₋₃.

2. Composé selon la revendication 1, dans lequel le groupe fonctionnel central a une structure planaire.

3. Composé selon la revendication 1, dans lequel le groupe fonctionnel central est un élément choisi dans le groupe constitué : d'acide barbiturique ; de benzamide ; de benzène ; de diphényle ; de cyclohexanedione ; de cyclopentanedione ; de delta-lactame ; de flutarimide ; de glutarimide ; d'homophthalimide ; d'imidazole amide ; d'isocarbostyrile ; de lumazine ; de naphthaline ; de ptéridine ; de phthalimide ; de pipéridine ; de pyridine ; de pyrimidine ; de pyrrole amide ; de quinazolinedione ; de quinazolinone ; de quinolone ; de résorcinol ; de stilbène ; de succinimide ; de théobromine ; de thymine : de triazine et de tricyclododécane.

4. Composé selon la revendication 1, dans lequel le groupe fonctionnel central est choisi dans le groupe constitué de glutarimide, de méthylthymine, de méthyluracil, de thymine, de théobromine, d'uracile et de xanthine.

5. Composé selon la revendication 1, dans lequel le groupe fonctionnel central est une xanthine, X est lié à un azote N₁ de xanthine et les azotes N₃ et N₇ de xanthine sont indépendamment substitués par un élément choisi dans le groupe constitué d'hydrogène, de méthyle, de fluoro, de chloro ou d'amino.

6. Composé selon la revendication 1, dans lequel le groupe fonctionnel central est choisi dans le groupe constitué de 1,3-cyclohexanedione, de 1,3-cyclopentanedione ; de 1,3-dihydroxynaphthalène ; de 1-méthyllumazine ; d'acide méthylbarbiturique ; de 3,3-diméthylflutarimide ; de 2-hydroxypyridine ; de méthyldihydroxypyrazolopyrimidine ; de méthylpyrrolopyrimidine ; de 2-pyrrole amides ; de 3-pyrrole amides ; de 1,2,3,4-tétrahydroisoquinolone ; de 1-méthyl-2,4(1H,3H)-quinazolinedione (1-méthylbenzoyleneurea) ; de quinazoline-4(3H)-one ; de thymine substituée par un alkyle (C₁₋₆) ; de méthylthymine ; d'uracile substitué par un alkyle (C₁₋₆) ; de 6-aminouracile ; de 1-méthyl-5,6-dihydrouracile ; de 1-méthyluracile ; de 1,7-diméthylxanthine ; de 3,7-diméthylxanthine ; de 3-méthylxanthine ; de 3-méthyl-7-méthylpivaloylxanthine ; de 8-amino-3-méthylxanthine ; et de 7-méthylhypoxanthine.

7. Composé selon la revendication 1, le composé étant choisi parmi les composés suivants :

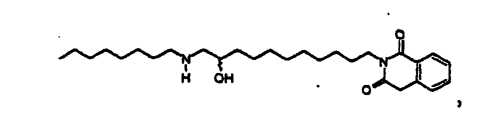

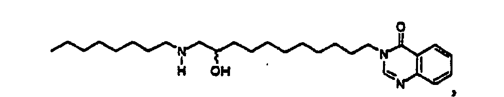

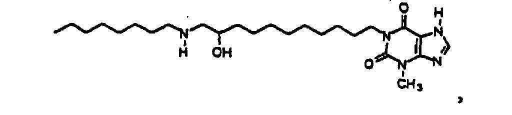

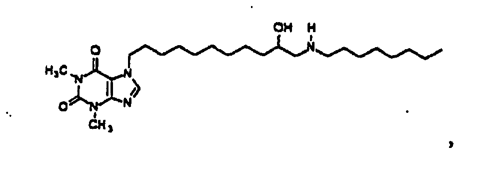

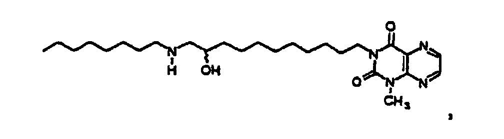

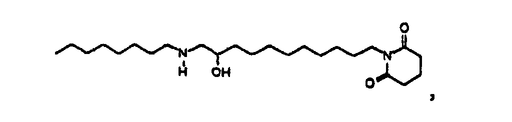

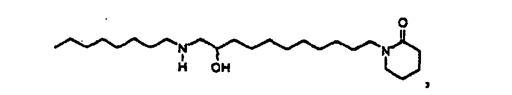

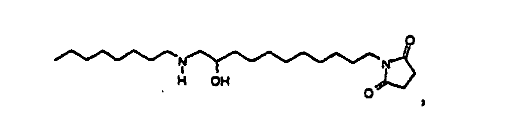

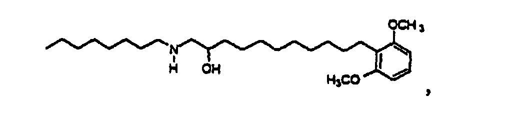

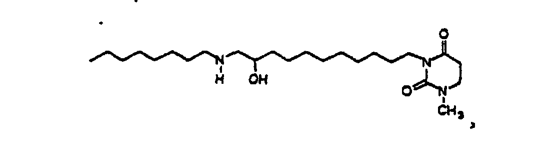

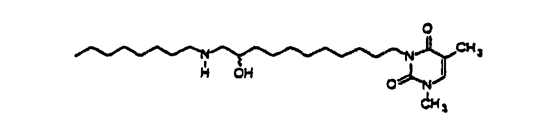

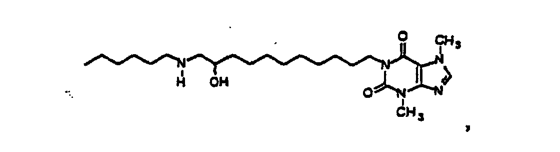

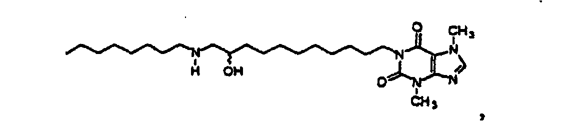

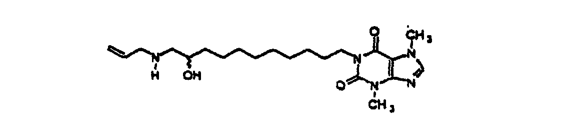

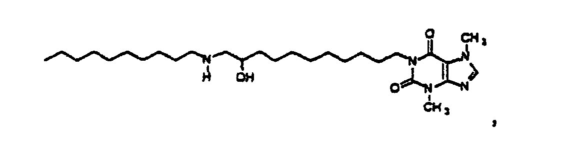

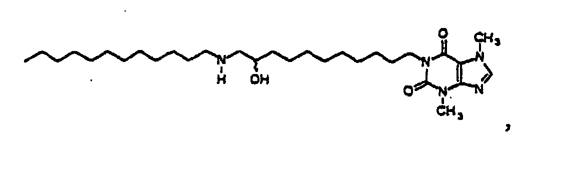

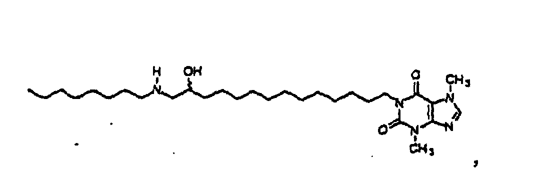

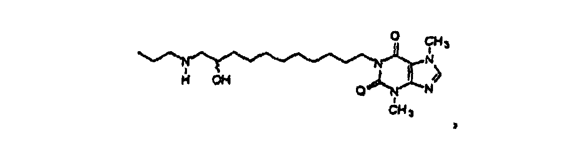

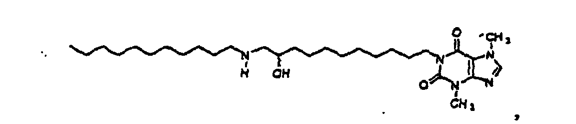

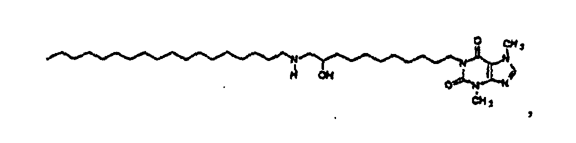

8. Composé étant choisi parmi les composés suivants :

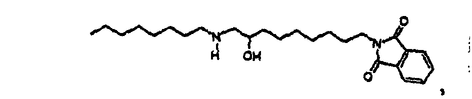

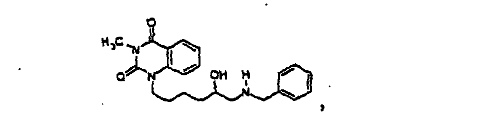

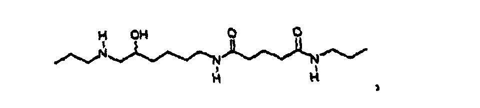

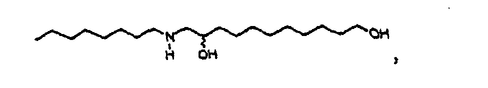

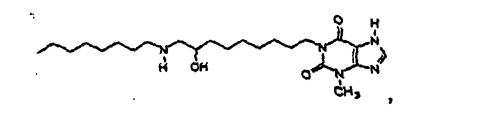

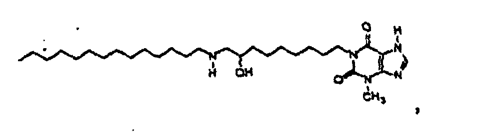

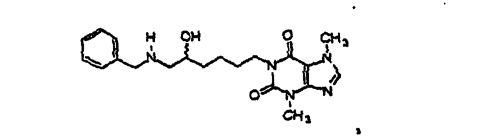

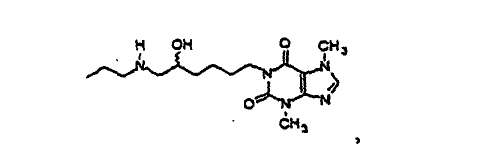

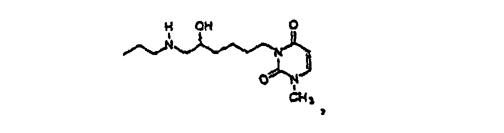

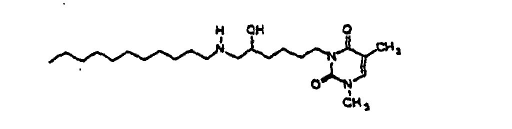

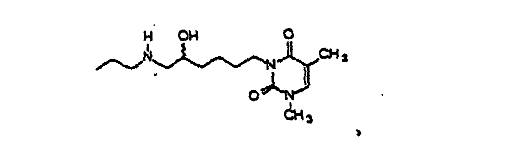

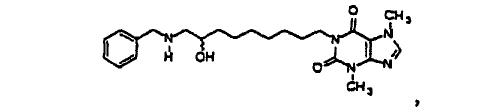

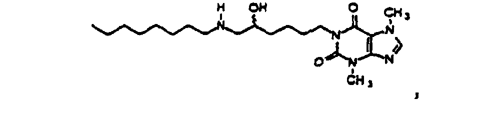

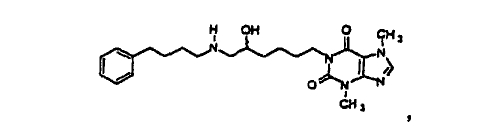

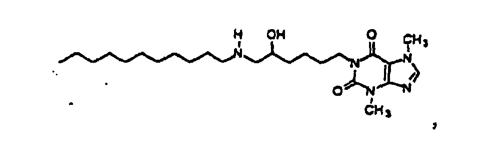

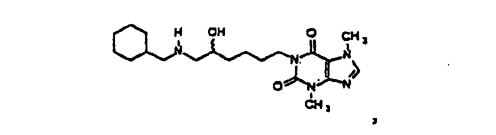

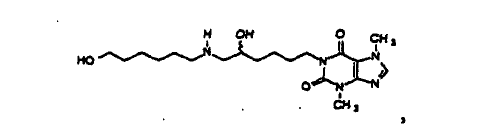

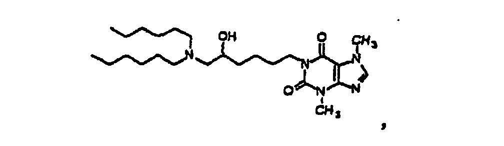

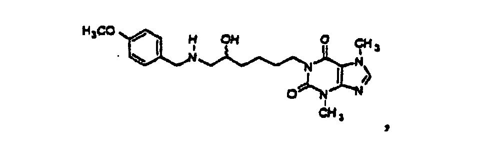

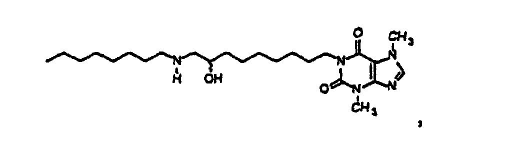

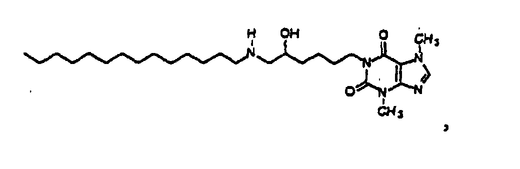

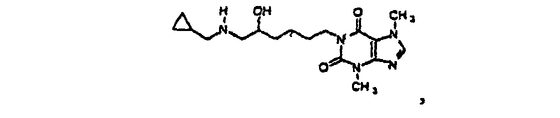

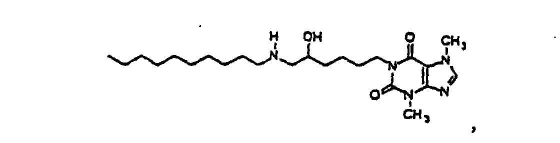

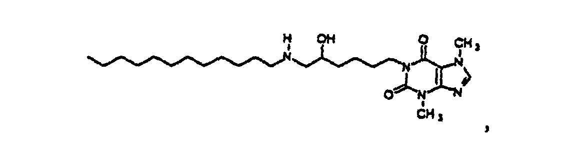

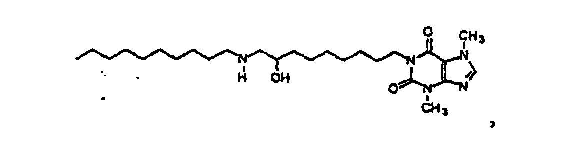

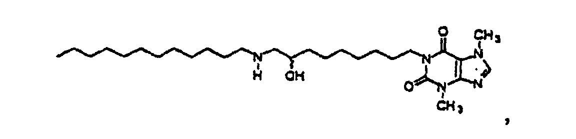

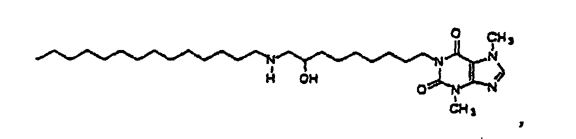

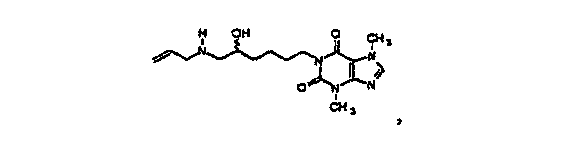

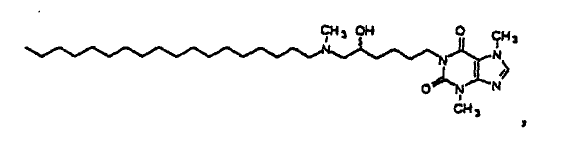

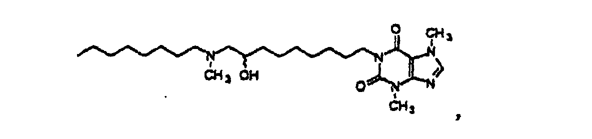

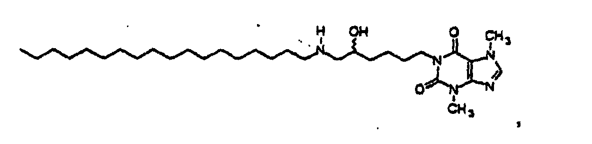

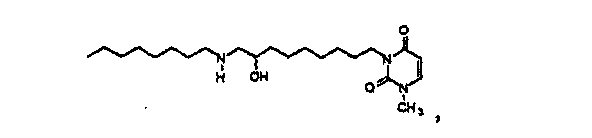

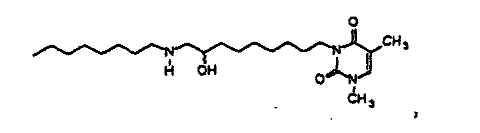

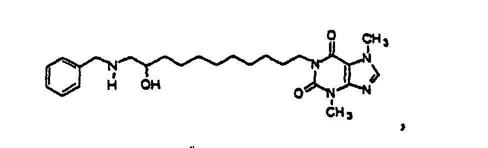

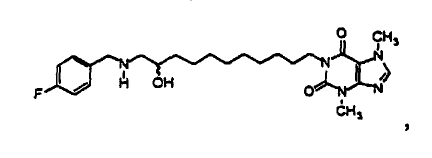

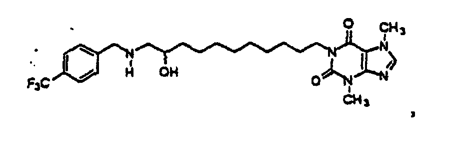

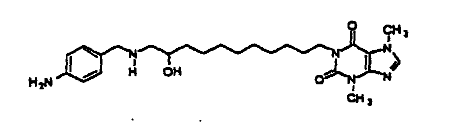

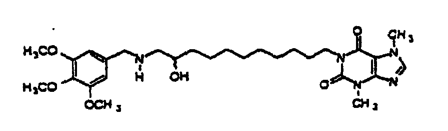

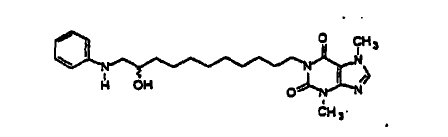

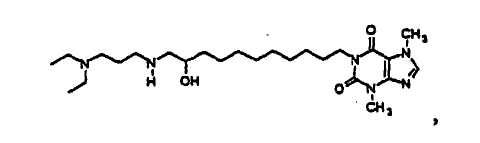

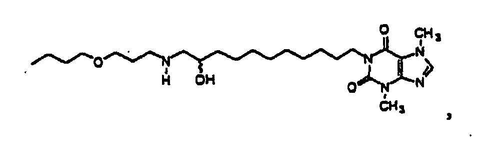

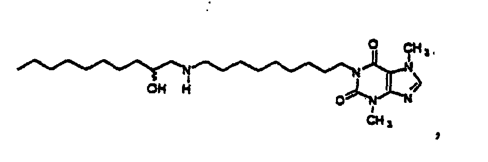

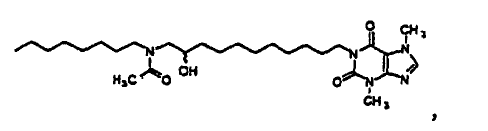

9. Composition pharmaceutique comprenant un composé selon la revendication 1 ou 8 ou son sel acceptable d'un point de vue pharmaceutique et un excipient acceptable d'un point de vue pharmaceutique, la composition pharmaceutique étant préparée pour une administration orale, parentérale, *ex vivo* ou topique pour un patient.

10. Composition selon la revendication 9, dans laquelle une dose orale de composé est comprise dans l'intervalle allant de 50 mg environ à 1500 mg environ, deux ou trois fois par jour, une dose parentérale est comprise dans l'intervalle allant de 1,0 g environ à 5,0 g environ administrés (i.v., i.p., i.m. ou s.c.) sur une durée de 24 heures, une préparation topique a une concentration en poids comprise entre 1 % environ et 4 % environ, et la concentration de la culture *ex vivo* est comprise entre 10 mM environ et 500 mM environ.

11. Utilisation d'un composé selon la revendication 1 ou 8 ou de son sel acceptable d'un point de vue pharmaceutique, pour préparer un médicament pour traiter ou prévenir une maladie auto-immune, dans laquelle la maladie auto-immune est choisie parmi le diabète sucré insulo-dépendant (IDDM, "insulin-dependent diabetes melitus"), le diabète affectant les adultes, l'athérosclérose, la sclérose en plaques, le psoriasis, l'asthme, la parodontopathie, l'ostéoporose, l'hépatite alcoolique, l'accouchement prématuré provoqué par une infection utérine, la thyroïdite auto-immune, la maladie d'Alzheimer, la polyarthrite rhumatoïde, la glomérulonéphrite, l'affection abdominale inflammatoire et le lupus.

12. Utilisation d'un composé selon la revendication 1 ou 8 ou de son sel acceptable d'un point de vue pharmaceutique, pour préparer un médicament pour traiter ou prévenir des maladies inflammatoires aiguës ou chroniques, le SIDA et le syndrome associé au SIDA, l'hépatite alcoolique, les allergies dues à la dégranulation des mastocytes et des cellules basophiles, l'angiogenèse, la maladie coronarienne, la chute des cheveux ou la calvitie, la démence associée au VIH, l'hypoxie des poumons, le lupus, les tumeurs malignes, la leucémie myéloïde, une blessure des organes ou hématopoïétique en réponse à une thérapie cytotoxique, l'arthrose, la resténose, les troubles du sommeil, un choc infectieux, le syndrome de sepsie, la sclerodermie, une attaque ou un rejet de greffe.

13. Procédé pour préparer un composé selon la revendication 1 ou 8, le procédé comprenant les étapes consistant à :
faire réagir le composé contenant le groupe central avec une base, un solvant et une oléfine substituée adéquats pour obtenir un produit intermédiaire, le produit intermédiaire ayant une structure composite de groupe fonctionnel central et d'oléfine substituée ;
transformer le produit intermédiaire en époxyde contenant le groupe central dans une réaction avec un peracide organique ; et
faire réagir l'époxyde contenant le groupe central avec une amine substituée ou non substituée pour obtenir un composé de la revendication 1 ou 8.
